(19) Europäisches Patentamt
European Patent Office
Office européen des brevets

(11) Publication number: **0 076 017 B1**

(12) **EUROPEAN PATENT SPECIFICATION**

(45) Date of publication of patent specification: **16.08.90**

(51) Int. Cl.$^5$: **A 61 K 31/33**, C 07 D 213/74, C 07 D 471/04 // (C07D471/04, 243:00, 221:00)

(21) Application number: **82300080.7**

(22) Date of filing: **07.01.82**

(54) **Phenyl substituted pyrido(1,4)benzodiazepines and intermediates therefor.**

(30) Priority: **24.09.81 US 305080**
**12.11.81 GB 8134121**

(43) Date of publication of application:
**06.04.83 Bulletin 83/14**

(45) Publication of the grant of the patent:
**16.08.90 Bulletin 90/33**

(84) Designated Contracting States:
**AT BE CH DE FR GB IT LI LU NL SE**

(56) References cited:
**GB-A- 696 473**
**GB-A- 738 013**
**GB-A-1 456 627**
**GB-A-1 542 940**

**Chemical Abstracts, vol. 91, 1979, page 475, no. 18331x, Columbus, Ohio, USA A.V. Bogatskii et al.: "Enzymic reduction of 2,4-dinitro-5H-11-(p-R-phenyl)dibenzo(b,f)(1,4)diazepines"**
**Helv. Chim. Acta., 46, 2337(1963)**
**Journal of Medicinal Chemistry 11, 894 (1968)**

(73) Proprietor: **A.H. ROBINS COMPANY, INCORPORATED**
**1407 Cummings Drive P.O. Box 26609**
**Richmond Virginia 23261-6609 (US)**

(72) Inventor: **Chandler, Roy Taylor, Jr.**
**2715 Fenholloway Drive**
**Mechanicsville Virginia 23111 (US)**

(74) Representative: **Kearney, Kevin David Nicholas et al**
**KILBURN & STRODE 30 John Street**
**London, WC1N 2DD (GB)**

**EP 0 076 017 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to certain novel pyrido[1,4]benzodiazepines, intermediates for the production of pyrido[1,4]benzodiazepines, and pharmaceutical methods and compositions for treating depression in humans.

Wander, A., in British Patent 907,646 discloses preparation of certain dibenzodiazepines substituted with phenyl radicals on carbon and with alkyl or aminoalkyl radicals on the briding nitrogen atom between the phenyl rings.

Greig, M. E., et al., J. Med. Chem. *14* No. 2., page 153 (1971), disclose dibenzodiazepines similar to the foregoing Wander disclosure useful against anaphylactic shock.

Japanese Patent 74/43,520 (C.A. *80*: 133501n discloses 6-phenyl-2,3,4,4a-tetrahydro-11H-pyrido[2,3-b][1,4]benzodiazepines having anticonvulsant activity which are illustratively prepared from 2-amino-benzophenones and ornithine.

Burgers Medicinal Chemistry, 4th Edition, Pt III, Wiley New York, pp. 1018—1021 (1981) discloses certain dibenzodiazepines having anti-depressant activity but states that replacing a benzo-fused ring with a pyrido fused ring increases toxicity and reduces the CNS activity.

According to the present invention there are provided pyrido[1,4]benzodiazepines which are 6-aryl-11H-pyrido[2,3-b][1,4]benzodiazepines or the 5,6 dihydro derivatives thereof, and which have the formula $I_w$, namely

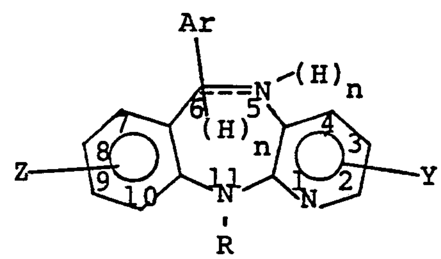

Formula $I_w$

which are 6-aryl-11H-pyrido[3,4-b][1,4]benzodiazepines or the 5,6 dihydro derivative thereof, and which have the formula $I_x$, namely

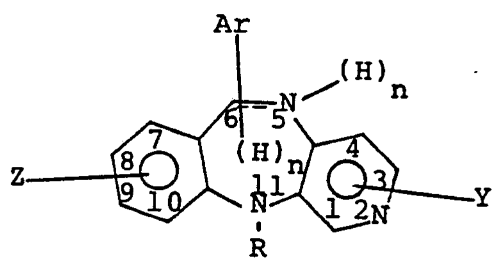

Formula $I_x$

or which are 10-aryl-5H-pyrido[4,3-b][1,4]benzodiazepines, or the 10,11 dihydro derivatives thereof, and which have the formula $I_y$, namely

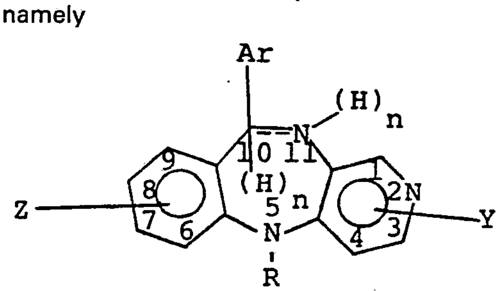

Formula $I_y$

or which are 10-aryl-5H-pyrido[3,2-b][1,4]benzodiazepines or the 10,11 dihydro derivatives thereof, and which have the formula $I_z$, namely

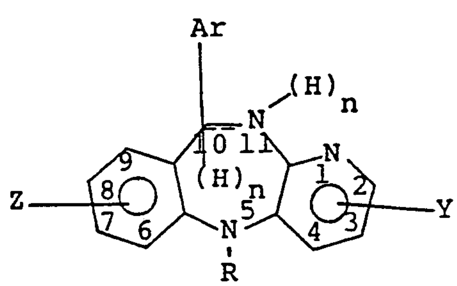

Formula $I_z$

wherein;

R represents a hydrogen atom, a $(C_1—C_8)$alkyl, an -alk$^1$—$NR^1R^2$ or an -alk$^1$-N=C—$OC_2H_5$ group;

$R^1$ and $R^2$ each represent a hydrogen atom, or a $C_2—C_8$ alkyl, or a —C(O)O—$(C_1—C_8)$ alkyl group, or $R^1$ and $R^2$ taken together with the adjacent nitrogen atom may form a 1-phthalimido, 1-piperidinyl, 1-pyrrolidinyl, 4-morpholino, 1-piperazino, or 4-substituted piperazin-1-yl residue.

Ar represents a 2, 3, or 4-pyridinyl, a 2 or 3-thienyl, or a phenyl or a substituted phenyl group, the said substituted phenyl group being substituted with 1 to 3 halo, loweralkyl loweralkoxy, trifluoromethyl or nitro radicals which may be the same or different;

Alk$^1$ represents a straight or branched hydrocarbon chain containing 1—8 carbon atoms;

Z represents a hydrogen atom, or a halogen atom or a $(C_1—C_8)$ alkyl, a $(C_1—C_8)$ alkoxy, a hydroxy or a nitro group;

Y represents a hydrogen atom or 1 or 2 $(C_1—C_8)$ alkyl, $(C_1—C_8)$ alkoxy or hydroxy radicals which may be the same or different;

n is 0 or 1 and when n is zero the dotted line is a double bond; and the acid addition salts thereof.

The compounds of Formulae $I_w$, $I_x$, $I_y$ and $I_z$ have utility as anti-depressants for treating depression or as intermediates in the preparation of other compounds of Formula I.

The novel [2-[amino-pyridinyl)amino]phenyl]arylmethanone intermediates (or precursors) which form in the reaction mixture prior to cyclization to diazepines and which have additional utility as anti-depressants for treating depression are represented by the formula

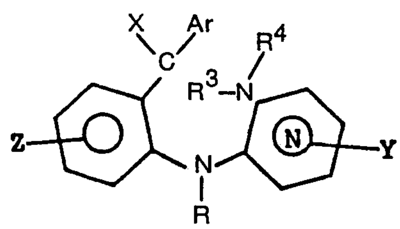

Formula II

wherein;

$R^1$ represents a hydrogen atom, a $(C_1—C_8)$ alkyl an -alk$^1$-$NR^1R^2$ or an -alk$^1$-N=CH—$OC_2H_5$ group;

$R^1$ and $R^2$ each represent a hydrogen atom, or a $(C_1—C_8)$ alkyl, or a —C(O)O—$(C_1—C_8)$ alkyl group, or $R^1$ and $R^2$ taken together with the adjacent nitrogen atom may form a 1-phthalimido, 1-piperidinyl, 1-pyrrolidinyl, 4-morpholino, 1-piperazino, or 4-substituted piperazin-1-yl heterocyclic residue;

Ar represents a 2, 3 or 4-pyridinyl, a 2 or 3-thienyl, or a phenyl or a substituted phenyl group, the said substituted phenyl group being substituted with 1 to 3 halo, $(C_1—C_8)$ alkyl, $(C_1—C_8)$ alkoxy, trifluoromethyl or nitro radicals which may be the same or different;

Alk$^1$ represents a straight or branched hydrocarbon chain containing 1—8 carbon atoms;

Z represents a hydrogen atom, or a halogen atom or a $(C_1—C_8)$ alkyl, a $(C_1—C_8)$ alkoxy, a hydroxy or a nitro group;

Y represents a hydrogen atom or 1 or 2 $(C_1—C_8)$ alkyl $(C_1—C_8)$ alkoxy or hydroxy radicals which may be the same or different;

and $R^3$ and $R^4$ each represent a hydrogen atom; and in which X represents =O or Q1 where Q1 represents a —C=O group or a carbonyl equivalent such as a ketal, thio-ketal or gem dihalide; and acid addition salts thereof.

In a preferred class of compounds of Formula II, R3 and R4 and R each represent a hydrogen atom. In a further modification R3 and R4 may each represent groups which can be split off, before or during the cyclization reaction, to leave groups (which may be the same or different) which do not interfere in the cyclization of compounds of Formula II to compounds of Formula I.

In the further definition of symbols in the formulas hereof and where they appear elsewhere throughout this specification and claims, the following terms have the following significance.

The "alk$^1$" straight or branched connecting hydrocarbon chain containing 1—8 carbons is exemplified by methylene (—$CH_2$—), ethylene (—$CH_2$—$CH_2$), propylene (—$CH_2CH_2CH_2$—), ethylidene

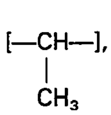, 1,2-propylene 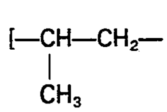 or 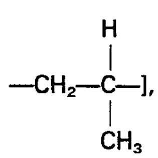, isopropylidine 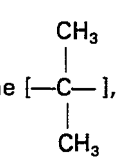, and 1,3-butylene

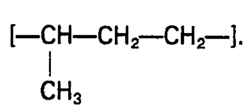.

The term "$(C_1—C_8)$alkyl" means straight and branched chain hydrocarbon radicals of up to eight

carbon atoms inclusive and is exemplified by such groups as methyl, ethyl, propyl, isopropyl, butyl, isobutyl, tertiary butyl, amyl, isoamyl, hexyl, heptyl, and octyl.

The term "halogen" means chlorine, bromine, fluorine, and iodine, preferably chlorine, bromine and fluorine.

The term "4-substituted-piperazin-1-yl" means piperazine substituted in the 4-position by a loweralkyl group or an alkyl-carbonyl blocking group which may subsequently be removed to give the unsubstituted piperazine.

"Pharmaceutically acceptable acid addition salts" means those salts formed by the pyrido-benzodiazepines of this invention with any acid which is physiologically compatible in warm blooded animals, such salts being formed either by strong or weak acids. Representative of strong acids are hydrochloric, sulphuric and phosphoric acids. Representative of weak acids are fumaric, maleic, succinic, oxalic, and cyclohexamic acids.

For the purpose of testing antidepressant activity of the compounds of the present invention, the procedure given by Englehardt, E. L., et al., J. Med. Chem. 11(2): 325 (1968) which has been indicative in the past of usefulness of compounds for treating human depression was used as follows: 20 mg/kg of the compound to be tested was administered to five adult female mice (ICR-DUB strain), intraperitoneally 30 minutes prior to the administration of a ptotic dose (32 mg/kg IP) of tetrabenazine (as the methane sulphonate salt). Thirty minutes later, the presence or absence of complete eyelid closure (ptosis) was assessed in each animal. An $ED_{50}$ (Median Effective Dose) may be established for each tested compound in blocking tetrabenazine induced depression in mice following the procedure given by Litchfield et al., J. Pharmacol. Exp. Therap. *96*: 99—113 (1949).

Compounds of the invention encompassed by Formula I which have antidepressant activity in the foregoing procedure have the Formula Ip

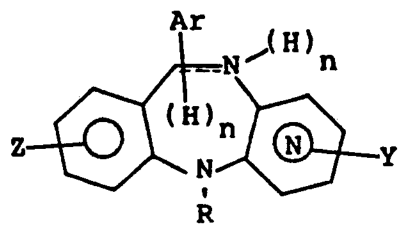

Formula $I_p$

wherein;

R represents a hydrogen atom, a loweralkyl or an -alk$^1$-N—$R^1R^2$ group;

$R^1$ and $R^2$ each represent a hydrogen atom or a loweralkyl group or $R^1$ and $R^2$ taken together with the adjacent nitrogen atom may form a 1-piperidinyl, 1-pyrrolidinyl, 4-morpholinyl, 1-piperazinyl or 4-loweralkylpiperzin-1-yl heterocyclic residue;

Ar represents a 2, 3 or 4-pyridinyl, a 2 or 3-thienyl, or a phenyl or a substituted phenyl group, the said substituted phenyl group being substituted with 1 to 3 halo, loweralkyl, loweralkoxy, trifluoromethyl or nitro radicals which may be the same or different;

Alk$^1$ represents a straight or branched hydrocarbon chain containing 1—8 carbon atoms;

Z represents a hydrogen atom, or a halogen atom, or a loweralkyl, a loweralkoxy, a hydroxy or a nitro group;

Y represents a hydrogen atom, or 1 or 2 loweralkyl, loweralkoxy or hydroxy radicals which may be the same or different;

n is 0 or 1 and when n is zero the dotted line is a double bond; and the pharmaceutically acceptable acid addition salts thereof.

The compounds of Formula Ip wherein R represents -alk$^1$-N$R^1R^2$ and $R^1$ and $R^2$ represent loweralkyl groups or hydrogen atoms have been shown to have low incidence of antihistaminic, anticholinergic and cardiotoxic side effects when tested in animals.

The preferred pyridobenzodiazepines useful in the method of treating depression are as follows.

Compound active ingredient (free base)

6-(4-fluorophenyl)-N,N-dimethyl-11H-pyrido[2,3-b][1,4]benzodiazepine-11-propanamine. (See Example 23)

6-phenyl-11H-pyrido[2,3-b][1,4]benzodiazepine-11-propanamine. (See Example 42)

N-methyl-6-phenyl-11H, pyrido[2,3-b][1,4]benzodiazepine-11-propanamine. (See Example 28)

6-(2-chlorophenyl)-N,N-dimethyl-11H-pyrido[2,3-b][1,4]benzodiazepine-11-propanamine. (See Example 52b).

Certain of the compounds of Formula I wherein the R moiety carries a phthalimido, chloro, carbamoyl or imidate component are intermediates rather than antidepressant agents.

It is therefore an object of the present invention to provide novel 6-aryl-11H-pyrido[1,4]benzodiazepines which have antidepressant activity.

Another object is to provide methods of treating depression and pharmaceutical compositions therefor.

Still another object is to provide novel intermediates for preparing aryl substituted-11H-pyrido[1,4]benzodiazepines which are antidepressants, certain of which intermediates also have anti-depressant activity.

The present invention encompasses the novel pyridobenzodiazepines and methanone intermediates as set forth herein in Formulas I and II as compositions of matter and utilization of these compounds as antidepressants or as intermediates in the preparation of other antidepressants.

*Description of Compound Preparation.* A reaction sequence set out as chemical equations for the preparation of the compounds of the invention is given in Chart 1. Alternative procedures for the preparation of certain compounds of Formulae $I_w$, $I_x$, $I_y$ and $I_z$ are set out as chemical equations in Charts 2, 3 and 4.

*Methanones, Formula II, See Chart 1.* The methanone intermediates are prepared by heating a mixture of the halo-amino pyridine and an aminobenzophenone for a shorter period of time than that required for cyclization to the pyridobenzodiazepine as indicated by chemical ionization mass spec. analysis. For the [2-(3-amino-2-pyridinyl)amino phenylmethanones, the conditions required are about 1 to 1.5 hr at 170—200°C. The methanones may be isolated as the predominant product, if desired, by cooling and adding a suitable organic solvent such as, for example, methylene chloride which will dissolve unreacted starting materials and some cyclized compound (Ia) followed by usual methods of isolating such as partitioning between the solvent and aqueous base or methanolic aqueous base followed by washing, drying, evaporating the solvent layer and recrystallizing from a suitable solvent.

*Unsubstituted Pyridobenzodiazepines, Formulas Ia and Ia-1 (R=H). See Chart 1.* The purified compounds of Formula II or crude compounds of formula II may be further heated in an aprotic solvent to cyclize to compounds of Formula Ia, removing water from the reaction mixture by conventional means; for example, under reflux using a Dean-Stark water trap. However, it is not necessary to stop heating at the intermediate stage; generally, it is sufficient to continue heating of the original reaction mixture; i.e. a mixture of compounds of formulas III + IV, for a longer period of time during which cyclization to compounds of formula Ia occurs. In the cyclization stage, whichever route is used, the temperature time relationship will vary to some extent depending on the reactants used, it being only necessary to heat for a time sufficient to produce the product desired as indicated by chemical ionization mass spec. The unsubstituted pyridobenzodiazepines may be purified by partitioning between a suitable solvent such as methylene chloride and aqueous base, washing and drying the solvent layer, evaporating and chromatographing in a suitable solvent system such as acetone-benzene. The corresponding dihydrodiazepine may be prepared by reduction with sodium boro-cyanohydrin.

*Substituted Pyridobenzodiazepines. Formulas Ib and Ib-1 (R=loweralkyl). See Chart 1.* Compounds of Formula Ia (or Ia-1) wherein R is a hydrogen atom are alkylaminated or radicals are introduced which will lead to alkylamination by reacting first with sodium hydride and then with an appropriate reagent represented by halo-alk$^1$Q wherein "alk$^1$" has the meaning as defined above and Q represents an —N-(loweralkyl)$_2$, 1-pyrrolidinyl, 1-piperidinyl, 4-substituted-piperazin-1-yl, 4-morpholino, 1-phthalimido,

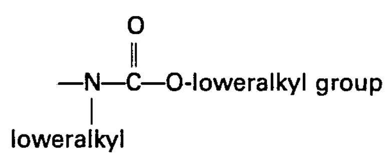

or a halogen atom (as defined in Chart 1.) The compounds suspended in a suitable solvent such as dimethyl formamide are added to a stirred suspension of sodium hydride in the same solvent. The halo-alk$^1$-Q reagent (alkylaminating agent or agent leading to alkylamination) is added at about room temperature and the reaction mixture is stirred for a period of time until reaction is complete as, for example, determined by thin layer chromatography. The unreacted sodium hydride is decomposed by adding to water and the product is extracted with a suitable solvent such as methylene chloride followed by aqueous acid extraction of the solvent layer and isolating the product from the aqueous layer by neutralization and re-extraction with methylene chloride followed by evaporation and precipitating, preferably as an addition salt such as a fumarate, hydrochloride, oxalate, or maleate. Generally, once having obtained and purified an acid addition salt, the free base may be regenerated by partitioning the salt between an aqueous base and a suitable solvent such as methylene chloride and evaporating the methylene chloride layer. The corresponding dihydrodiazepines may be prepared by reduction with sodium borocyanohydrin. Alternately, compounds of Formulas Ib wherein Q is a halogen atom may be converted to compounds wherein Q is —N-(loweralkyl)$_2$ by reacting with an appropriate dialkylamine as given in the reaction sequence of Chart 2.

The primary amines of Formula Ic; i.e., where R$^1$ and R$^2$ are both hydrogen atoms, may be prepared from the -alk$^1$-ω-(1-phthalimido) derivatives, as shown in Chart 1, by reacting with hydrazine hydrate, utilizing the method of Org. Syn. Coll. Vol. III, pp 151—153. Generally, about 2—3 hr reflux time is sufficient after which aqueous acid is added and the mixture is filtered. The primary -alk$^1$-amines may be isolated from suitable solvents such as isopropyl alcohol. Hydrochlorides and hydrochloride hydrates are preferred

salts in the isolation step. The corresponding dihydrodiazepines may be obtained by reduction with sodium borocyanohydrin.

The -alk$^1$-ω-monoalkylamines (Formula Ie); e.g., where $R^1$=methyl, $R^2$=hydrogen, may be prepared as shown in Chart 1 by reacting the primary -alk$^1$-$NH_2$ derivatives Formula Ic or Ic-1 with refluxing triethyl orthoformate for a period of time sufficient to form the methanimidic acid ester (formula I-d) which is then reacted with sodium borohydride. The unreacted borohydride is decomposed with water and the product extracted out with a suitable solvent such as ethyl acetate and may be purified by column chromatography and partitioning with basic solvent. Hydrochlorides are preferred salts in the isolation step. The method is more fully exemplified in Examples 43 and 44. The corresponding dihydrobenzodiazepines may then be prepared by reduction with sodium-borocyanohydrin.

-Alk$^1$-ω-monomethylamines may also be prepared by reaction of the primary amine with ethyl chloroformate as in Example 45, and thereafter reducing with lithium aluminium hydride as exemplified in Chart 3.

A further more generalized alternative for introduction of -alk$^1$-ω-monoloweralkyl amine radicals is via the radical:

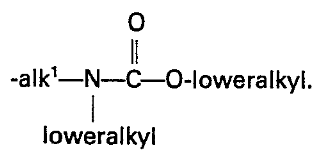

See Chart 1.

All the formulas Ia, Ia-1, Ib, Ib-1, Ib-2, Ib-3, Ib-4, Ic, Ic-1, Ic-2, Id, Ie, Ie-1 are encompassed by the Formula I.

Compounds of Formulae $I_w$, $I_x$, $I_y$ and $I_z$ wherein the —$NR^1R^2$ moiety is an unsubstituted 1-piperazinyl group may be obtained by hydrolyzing a compound of Formula I wherein —$NR^1R^2$ is piperazino substituted in the 4-position by an alkyl carbonyl such as t-butoxycarbonyl.

CHART 1

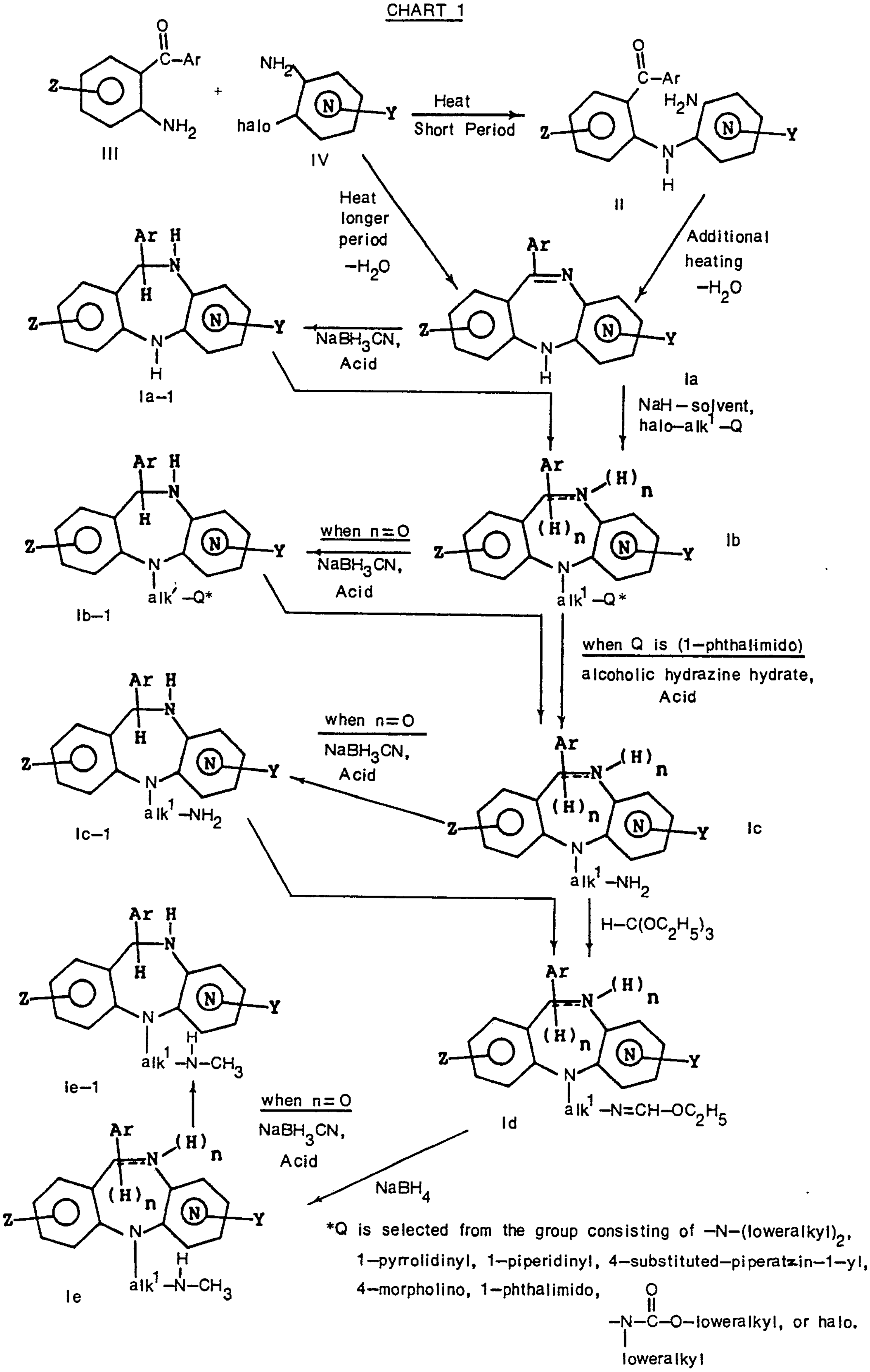


III

IV

II

Ia

Ia–1

Ib

Ib–1

Ic

Ic–1

Id

Ie

Ie–1

*Q is selected from the group consisting of –N–(loweralkyl)$_2$, 1–pyrrolidinyl, 1–piperidinyl, 4–substituted–piperazin–1–yl, 4–morpholino, 1–phthalimido, or halo.

CHART 2

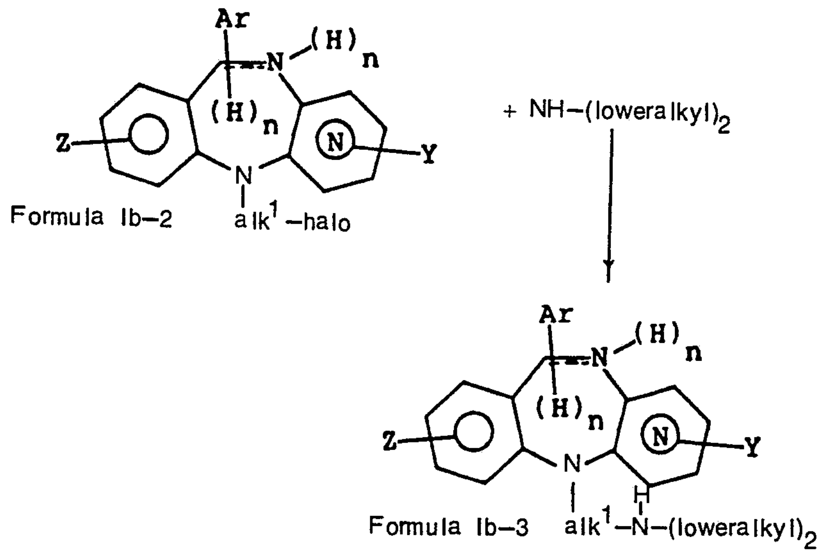


Formula Ib–2

Formula Ib–3

CHART 3

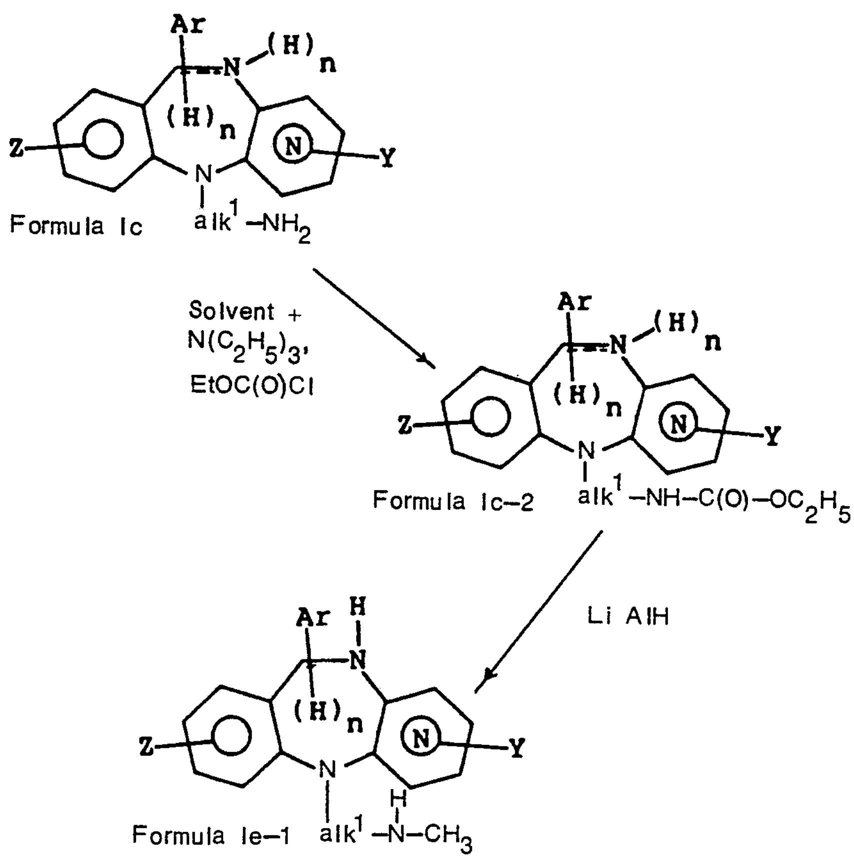


Formula Ic

Formula Ic–2

Formula Ie–1

CHART 4

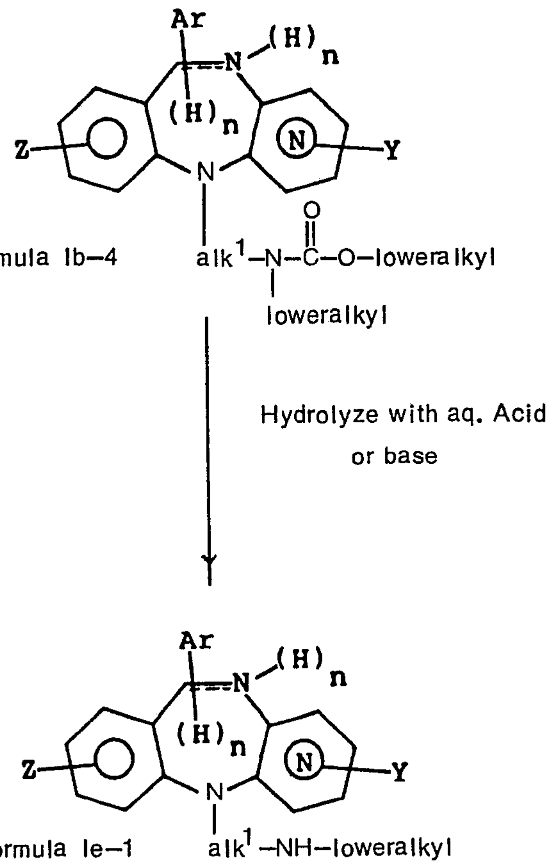


Formula Ib—4

Formula Ie—1

The preparation of the novel [(amino-pyridinyl)aminophenylaryl]methanones which are intermediates in the preparation of the phenyl substituted-pyrido[1,4]benzodiazepines are illustrated more fully in the following Examples 1 to 16. Structures of the intermediates are illustrated in Table 1.

## Preparation of Methanone Intermediates

### Example 1

[2-[(3-Amino-2-pyridinyl)amino]phenyl]phenylmethanone.

A stirred mixture of 39.4 g (0.20 mole) of 2-aminobenzophenone and 28.3 g (0.22 mole) of 3-amino-2-chloropyridine was heated at 180°C under a nitrogen atmosphere for 1.5 hr. The mixture was allowed to cool somewhat and 200 ml of methylene chloride was added slowly. After stirring for 3 hr and standing overnight at room temperature, 40.1 g of solid was filtered off and recrystallized twice from methanol-isopropyl ether giving 4.3 g, presumably the hydrochloride salt; m.p. 187—90°C. This solid was dissolved in a mixture of water-methanol, made basic with 3N sodium hydroxide and extracted with methylene chloride. The combined methylene chloride extracts were dried over magnesium sulphate and evaporated under reduced pressure. The residue was recrystallized from isopropyl ether (charcoal) to give 2.1 g of product; m.p. 91—3°C. Drying prior to analysis was overnight at room temperature and 0.02 mm Hg pressure.

| | | | |
|---|---|---|---|
| Analysis: Calculated for $C_{18}H_{15}N_3O$: | C, 74.72; | H, 5.23; | N, 14.52 |
| Found: | C, 74.94; | H, 5.23; | N, 14.69 |

### Example 2

[2-[(3-Amino-2-pyridinyl)amino]-4-chlorophenyl]phenylmethanone.

A stirred mixture of 23.2 g (0.1 mole) of 2-amino-4′-chlorobenzophenone and 14.2 g (0.11 mole) of 3-amino-2-chloropyridine was heated at 180°C under a nitrogen atmosphere for 2.5 hr. The mixture solidified upon cooling to room temperature and was broken up with a spatula. The solid was suspended in 100 ml of methylene chloride and collected by filtration. The filter cake was dissolved in a mixture of water-methanol, made basic with 3N sodium hydroxide and extracted twice with methylene chloride. The combined methylene chloride extracts were dried over magnesium sulphate and evaporated under reduced pressure. The residue which had crystallized was triturated in isopropyl ether and the solid (16.7 g) collected by filtration. A 3 g sample was recrystallized from isopropyl ether to give 1.6 g of product; m.p. 153—155°C.

Analysis Calculated for $C_{18}H_{14}ClN_3O$: C, 66.77; H, 4.36; N, 12.98
Found: C, 67.06; H, 4.36; N, 13.17

Example 3

[2-[(3-Amino-2-(pyridinyl)amino)phenyl](4-methylphenyl)methanone.

A stirred mixture of 20.0 g (0.095 mole) of 2-amino-4'-methylbenzophenone and 13.95 g (0.104 mole) of 3-amino-2-chloropyridine (96%) was heated under a nitrogen atmosphere at 180°C for 2.0 hr. The mixture cooled to a glassy solid which was broken up, triturated in methylene chloride and the mixture stirred overnight. The solid was collected by filtration and dissolved in warm methanol. The solution was made basic with 3N sodium hydroxide, diluted with 500 ml of water and extracted 3 times with 250 ml of methylene chloride. The combined methylene chloride extracts were dried over magnesium sulphate and evaporated under reduced pressure. The residue which crystallized on standing was recrystallized twice from benzene-isooctane to give 4.2 g of product, m.p. 126—127.5°C.

Analysis: Calculated for $C_{18}H_{17}N_3O$: C, 75.23; H, 5.65; N, 13.85
Found: C, 75.81; H, 5.69; N, 13.96

Example 4

[2-[(3-Amino-2-pyridinyl)amino]-5-chlorophenyl]-(2-chlorophenyl)methanone.

A stirred mixture of 20.0 g (0.156 mole) of 3-amino-2-chloropyridine and 37.3 g (0.14 mole) of 2-amino-2',5-dichlorobenzophenone was heated at 190°C under a nitrogen atmosphere for 5.5 hr. Thin layer chromatography (5% methyl alcohol-benzene on silica gel) indicated reaction had not substantially occurred. The mixture was stirred overnight at 190°C cooled somewhat, and 100 ml methylene chloride was added cautiously. The suspension was stirred for two hr and the black solid which formed was separated by filtration. The solid was suspended in 500 ml of methylene chloride and 300 ml of dilute sodium hydroxide was added. An emulsion formed and the mixture was filtered which allowed separation of layers. The methylene chloride layer was washed with two 250 ml portions of water by extraction, dried over magnesium sulphate and evaporated under reduced pressure. The residue was dissolved in benzene and filtered through a 300 g column of florisil to remove low $R_f$ material. All fractions were combined and evaporated under reduced pressure. Thin layer chromatography showed the presence of two major components with the lower $R_f$ spot predominating. The residue was dissolved in benzene and chromatographed on a 600 g column of florisil packed in benzene. The higher $R_f$ material was eluted with 1% acetone-benzene. On evaporation, the residue was triturated in benzene and recrystallized from benzene-isooctane to give 2.7 g of product; m.p. 162—4°C.

Analysis: Calculated for $C_{18}H_{13}Cl_2N_3O$: C, 60.35; H, 3.66; N, 11.73
Found: C, 60.67; H, 3.67; N, 11.77

Examples 5a to 5u

Following the procedures of Example 3 and substituting equal molar amounts of the following for 2-amino-4'-methylbenzophenone:

2-amino-4'-ethylbenzophenone,
2-amino-4'-isopropylbenzophenone,
2-amino-4'-bromobenzophenone,
2-amino-3'-fluorobenzophenone,
2-amino-4'-ethoxybenzophenone,
2-amino-4'-nitrobenzophenone,
2-amino-4'-trifluoromethylbenzophenone,
2-amino-3'-methylbenzophenone,
2-amino-3'-ethylbenzophenone,
2-amino-3'-methoxybenzophenone,
2-amino-3'-ethoxybenzophenone,
2-amino-2'-nitrobenzopheone,
2-amino-3'-trifluoromethylbenzophenone,
2-amino-2'-methylbenzophenone,
2-amino-2'-ethylbenzophenone,
2-amino-2'-methoxybenzophenone,
2-amino-2',4'-dichlorobenzophenone,
2-amino-3',4',5'-trimethoxybenzophenone,
2-amino-2'-fluorobenzophenone,
2-amino-2'-chlorobenzophenone, and
2-amino-2'-bromobenzophenone,

there were obtained:

a) [2-[(-3-amino-2-pyridinyl)amino]phenyl]-(4-ethylphenyl) methanone,
b) [2-[(3-amino-2-pyridinyl)amino]phenyl]-(4-isopropylphenyl)methanone,
c) [2-[(3-amino-2-pyridinyl)amino]phenyl]-(4-bromophenyl)methanone,

d) [2-[(3-amino-2-pyridinyl)amino]phenyl]-(3-fluorophenyl)methanone,
e) [2-[(3-amino-2-pyridinyl)amino]phenyl]-(4-ethoxyphenyl)methanone,
f) [2-[(3-amino-2-pyridinyl)amino]phenyl]-(4-nitrophenyl)methanone,
g) [2-[(3-amino-2-pyridinyl)amino]phenyl]-(4-trifluoromethylphenyl)methanone,
h) [2-[(3-amino-2-pyridinyl)amino]phenyl]-(3-methylphenyl)methanone,
i) [2-[(3-amino-2-pyridinyl)amino]phenyl]-(3-ethylphenyl)methanone,
j) [2-[(3-amino-2-pyridinyl)amino]phenyl]-(3-methoxyphenyl)methanone,
k) [2-[(3-amino-2-pyridinyl)amino]phenyl]-(3-ethoxyphenyl)methanone,
l) [2-[(3-amino-2-pyridinyl)amino]phenyl]-(2-nitrophenyl)methanone,
m) [2-[(3-amino-2-pyridinyl)amino]phenyl]-(3-trifluoromethylphenyl)methanone,
n) [2-[(3-amino-2-pyridinyl)amino]phenyl]-(2-methylphenyl)methanone,
o) [2-[(3-amino-2-pyridinyl)amino]phenyl]-(2-ethylphenyl)methanone,
p) [2-[(3-amino-2-pyridinyl)amino]phenyl]-(2-methoxyphenyl)methanone,
q) [2-[(3-amino-2-pyridinyl)amino]phenyl]-(2,4-dichlorophenyl)methanone,
r) [2-[(3-amino-2-pyridinyl)amino]phenyl]-(3,4,5-trimethoxyphenyl)methanone,
s) [2-[(3-amino-2-pyridinyl)amino]phenyl]-(2-fluorophenyl)methanone,
t) [2-[(3-amino-2-pyridinyl)amino]phenyl]-(2-chlorophenyl)methanone, and
u) [2-[(3-amino-2-pyridinyl)amino]phenyl]-(2-bromophenyl)methanone.

## Examples 6a to 6o

Following the procedure of Example 2 and substituting equal molar amounts of the following for 2-amino-4-chlorobenzophenone:

2-amino-5-chlorobenzophenone,
2-amino-6-chlorobenzophenone,
2-amino-4-fluorobenzophenone,
2-amino-4-bromobenzophenone,
2-amino-4-trifluoromethylbenzophenone,
2-amino-4-methylbenzophenone,
2-amino-5-methylbenzophenone,
2-amino-6-methylbenzophenone,
2-amino-4-ethylbenzophenone,
2-amino-4-methoxybenzophenone,
2-amino-4-ethoxybenzophenone,
2-amino-4-nitrobenzophenone,
2-amino-5-nitrobenzophenone,
2-amino-3-methylbenzophenone, and
2-amino-3-chlorobenzophenone,

there were obtained:

a) [2-[(3-amino-2-pyridinyl)amino]-5-chlorophenyl]phenylmethanone,
b) [2-[(3-amino-2-pyridinyl)amino]-6-chlorophenyl]phenylmethanone,
c) [2-[(3-amino-2-pyridinyl)amino]-4-fluorophenyl]phenylmethanone,
d) [2-[(3-amino-2-pyridinyl)amino]-4-bromophenyl]phenylmethanone,
e) [2-[(3-amino-2-pyridinyl)amino]-4-trifluoromethylphenyl]phenylmethanone,
f) [2-[(3-amino-2-pyridinyl)amino]-4-methylphenyl]phenylmethanone,
g) [2-[(3-amino-2-pyridinyl)amino]-5-methylphenyl]phenylmethanone,
h) [2-[(3-amino-2-pyridinyl)amino]-6-methylphenyl]phenylmethanone,
i) [2-[(3-amino-2-pyridinyl)amino]-4-ethylphenyl]phenylmethanone,
j) [2-[(3-amino-2-pyridinyl)amino]-4-methoxyphenyl]phenylmethanone,
k) [2-[(3-amino-2-pyridinyl)amino]-4-ethoxyphenyl]phenylmethanone,
l) [2-[(3-amino-2-pyridinyl)amino]-4-nitrophenyl]phenylmethanone,
m) [2-[(3-amino-2-pyridinyl)amino]-5-nitrophenyl]phenylmethanone,
n) [2-[(3-amino-2-pyridinyl)amino]-3-methylphenyl]phenylmethanone, and
o) [2-[(3-amino-2-pyridinyl)amino]-3-chlorophenyl]phenylmethanone.

## Examples 7a to 7c

Following the procedure of Example 1 and substituting equal molar amounts of the following for 3-amino-2-chloropyridine:

4-amino-3-chloropyridine,
3-amino-4-chloropyridine, and
2-amino-3-chloropyridine,

there were obtained:

a) [2-[(4-amino-3-pyridinyl)amino]phenyl]phenylmethanone,
b) [2-[(3-amino-4-pyridinyl)amino]phenyl]phenylmethanone, and
c) [2-[(2-amino-3-pyridinyl)amino]phenyl]phenylmethanone.

Example 8

[2-[(3-Amino-2-pyridinyl)amino]phenyl](3-chlorophenyl) methanone.

A stirred mixture of 35 g (0.152 mole) of 2-amino-3'-chlorobenzophenone and 23.4 g (0.182 mole) of 3-amino-2-chloropyridine was heated at 180°C for 2 hr. The hot melt was allowed to cool to 110°C, after which 100 ml of hot toluene was added dropwise with vigorous stirring. The mixture was allowed to cool while stirring to 30°C and 50 ml of methylene chloride was added. After stirring for an additional 1/2 hour, the mixture was filtered and the filter cake suspended in methylene chloride with stirring for 1/2 hr and methylene chloride was separated by filtration. The filter cake containing the product (25.4 g) was partially dissolved in hot methanol (total volume 150 ml) and 50% aqueous sodium hydroxide was added until the mixture was basic. Ice water was added and the solution was extracted with methylene chloride. This methylene chloride extract was washed with water and dried over magnesium sulphate and evaporated to dryness. The residue was dissolved in isopropyl alcohol and boiled with charcoal. The mixture was filtered, and reduced in volume to give a first crop of crystals weighing 16 g (33%). A portion of the crystals was recrystallized from isopropyl alcohol to give a brick red solid melting at 119—120°C.

| | | | |
|---|---|---|---|
| Analysis: Calculated for: | C, 66.77; | H, 4.36; | N, 12.98 |
| Found: | C, 66.78; | H, 4.42; | N, 12.94 |

Example 9

[2-[(3-Amino-2-pyridinyl)amino]phenyl](4-fluorophenyl)methanone.

A stirred mixture of 35 g (0.163 mole) of 2-amino-4'-fluorobenzophenone and 27 g (0.21 mole) of 3-amino-2-chloropyridine was heated at 175—180°C for 2.5 hr. The mixture was allowed to cool to 110°C, after which 100 ml of hot toluene was added. On cooling to 50°C, 50 ml of methylene chloride was added. The solvent layer was decanted, leaving a black solid mass which was dissolved in hot methanol. The solution volume was reduced by one half and allowed to stand overnight at room temperature. The mixture was filtered and the filter cake washed twice by suspending it in methylene chloride. The weight of crude solid produced was 22.5 g. The solid was dissolved in methanol and made basic with 50% aqueous sodium hydroxide. The mixture was extracted with methylene chloride and the extract dried and concentrated. The residue was twice crystallized from isopropyl alcohol, decolorizing by boiling with charcoal on the second crystallization, to give 14 g (28%) of solid which was red-orange in colour; m.p. 121.5—122.5°C.

| | | | |
|---|---|---|---|
| Analysis: Calculated for $C_{18}H_{14}N_3OF$: | C, 70.35; | H, 4.59; | N, 13.67 |
| Found: | C, 70.23; | H, 4.59, | N, 13.64 |

Example 10

[2-[(3-Amino-2-pyridinyl)amino]phenyl](2-thienyl)methanone.

In accordance with the procedure of Example 9, (2-aminophenyl)(2-thienyl)methanone, prepared by the method of Steinkopf & Gunther, Ann. *522,* 28—34 (1936), is reacted with 3-amino-2-chloropyridine to give the title compound.

Example 11

[2-[(3-Amino-2-pyridinyl)amino]phenyl(3-thienyl)methanone.

In accordance with the procedure of Example 9, (2-aminophenyl)(3-thienyl)methanone was reacted with 3-amino-2-chloropyridine to give the title compound.

Example 12

[2-[(3-Amino-2-pyridinyl)amino]phenyl](2-pyridinyl)methanone.

The title compound was prepared by reacting (2-aminophenyl)(2-pyridinyl)methanone, as prepared by Schofield, K., J. Chem. Soc. *1949,* 2408—12, with 3-amino-2-chloropyridine.

Example 13

[2-[(3-Amino-2-pyridinyl)amino]phenyl](3-pyridinyl)methanone.

The title compound was prepared by reacting (2-aminophenyl)(3-pyridinyl)methanone as prepared by Abramovitch R. A. & Tertzakian, G., *Tetrahedron Letters,* 1963, 1511—15 and Abramovitch, R. A. et al., Can. J. Chem. *43(4),* 725—31 (1965) with 3-amino-2-chloropyridine.

Example 14

[2-[(3-Amino-2-pyridinyl)amino]phenyl](4-pyridinyl)methanone.

The title compound was prepared by reacting (2-aminophenyl)(4-pyridinyl)methanone as prepared by Nann, A. J. and Schofield, K., J. Chem. Soc. *1952,* 583—9 with 3-amino-2-chloropyridine.

Examples 15a to 15g

Following the procedure of Example 1 and substituting equal molar amounts of the following for 3-amino-2-chloropyridine:

3-amino-2-chloro-4-methylpyridine,
3-amino-2-chloro-5-methylpyridine,
3-amino-2-chloro-6-methylpyridine,

3-amino-2-chloro-5,6-dimethylpyridine,
3-amino-2-chloro-6-methoxypyridine,
3-amino-4-chloro-2-methylpyridine, and
3-amino-2-chloro-5-methoxypyridine,

there were obtained:

a) [2-[(3-amino-4-methyl-2-pyridinyl)amino]phenyl]phenylmethanone,
b) [2-[(3-amino-5-methyl-2-pyridinyl)amino]phenyl]phenylmethanone,
c) [2-[(3-amino-6-methyl-2-pyridinyl)amino]phenyl]phenylmethanone,
d) [2-[(3-amino-5,6-dimethyl-2-pyridinyl)amino]phenyl]phenylmethanone,
e) [2-[(3-amino-6-methoxy-2-pyridinyl)amino]phenyl]phenylmethanone,
f) [2-[(3-amino-2-methyl-4-pyridinyl)amino]phenyl]phenylmethanone, and
g) [2-[(3-amino-5-methoxy-2-pyridinyl)amino]phenyl]phenylmethanone.

Examples 16a to 16f

Following the procedure of Example 1 and substituting equal molar amounts of the following for 3-amino-2-chloropyridine:

2-amino-3-chloro-5-methylpyridine,

TABLE 1

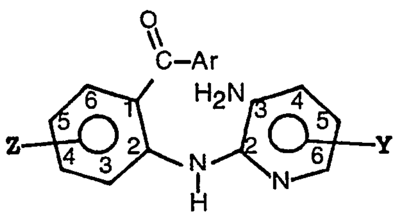

Formula $II_w$

| Example | Ar | Y | Z |
|---|---|---|---|
| | Substituents | | |
| 1 | $C_6H_5$— | H | H |
| 2 | $C_6H_5$— | H | 4-CL |
| 3 | 4-$CH_3$—$C_6H_4$— | H | H |
| 4 | 2-Cl—$C_6H_4$— | H | 5-Cl |
| 5a) | 4-$C_2H_5$—$C_6H_4$— | H | H |
| b) | 4-i-$C_3H_7$—$C_6H_4$— | H | H |
| c) | 4-Br-$C_6H_4$ | H | H |
| d) | 3-F—$C_6H_4$— | H | H |
| e) | 4-$OC_2H_5$—$C_6H_4$— | H | H |
| f) | 4-$NO_2$—$C_6H_4$— | H | H |
| g) | 4-$CF_3$—$C_6H_4$— | H | H |
| h) | 3-$CH_3$—$C_6H_4$— | H | H |
| i) | 3-$C_2H_5$—$C_6H_4$— | H | H |
| j) | 3-$OCH_3$—$C_6H_4$— | H | H |
| k) | 3-$OC_2H_5$—$C_6H_4$— | H | H |
| l) | 2-$NO_2$—$C_6H_4$— | H | H |
| m) | 3-$CF_3$—$C_6H_4$— | H | H |
| n) | 2-$CH_3$—$C_6H_4$— | H | H |

TABLE 1 (continued)

| Example | Substituents Ar | Y | Z |
|---|---|---|---|
| o) | 3-$CH_2H_5$—$C_6H_4$— | H | H |
| p) | 2-$OCH_3$—$C_6H_4$— | H | H |
| q) | 2,4-$Cl_2$—$C_6H_3$— | H | H |
| r) | 3,4,5-$(OCH_3)_3$—$C_6H_2$— | H | H |
| s) | 2-F—$C_6H_4$— | H | H |
| t) | 2-Cl-$C_6H_4$ | H | H |
| u) | 2-Br—$C_6H_4$ | H | H |
| 6a) | $C_6H_5$— | H | 5-Cl |
| b) | $C_6H_5$— | H | 6-Cl |
| c) | $C_6H_5$— | H | 4-F |
| d) | $C_6H_5$— | H | 4-Br |
| e) | $C_6H_5$— | H | 4-$CF_3$ |
| f) | $C_6H_5$— | H | 4-Me |
| g) | $C_6H_5$— | H | 5-Me |
| h) | $C_6H_5$— | H | 6-$CH_3$ |
| i) | $C_6H_5$— | H | 4-$CH_2H_5$ |
| j) | $C_6H_5$— | H | 4-$OCH_3$ |
| k) | $C_6H_5$— | H | 4-$OC_2H_5$ |
| l) | $C_6H_5$— | H | 4-$NO_2$ |
| m) | $C_6H_5$— | H | 5-$NO_2$ |
| n) | $C_6H_5$— | H | 3-$CH_3$ |
| o) | $C_6H_5$— | H | 3-Cl |
| 8 | 3-Cl—$C_6H_4$— | H | H |
| 9 | 4-F—$C_6H_4$— | H | H |
| 10 | 2-thienyl | H | H |
| 11 | 3-thienyl | H | H |
| 12 | 2-pyridinyl | H | H |
| 13 | 3-pyridinyl | H | H |
| 14 | 4-pyridinyl | H | H |
| 15a) | $C_6H_5$— | 4-$CH_3$ | H |

TABLE 1 (continued)

| Example | Ar | Y | Z |
|---|---|---|---|
| | Substituents | | |
| 15b) | $C_6H_5-$ | 5-$CH_3$ | H |
| 15c) | $C_6H_5-$ | 6-$CH_3-$ | H |
| 15d) | $C_6H_5-$ | 5,6-$(CH_3)_2$ | H |
| 15e) | $C_6H_5-$ | 6-$OCH_3$ | H |
| 15g) | $C_6H_5-$ | 5-$OCH_3$ | H |

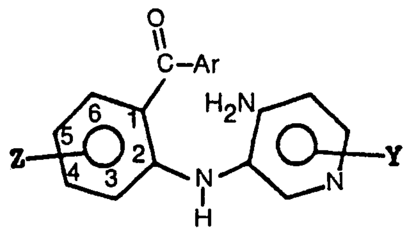

Formula $II_x$

| Example | Ar | Y | Z |
|---|---|---|---|
| 7a) | $C_6H_5-$ | H | H |
| 16d) | $C_6H_5-$ | 5-$CH_3$ | H |
| e) | $C_6H_5-$ | 6-$CH_3$ | H |
| f) | $C_6H_5-$ | 2-$CH_3$ | H |

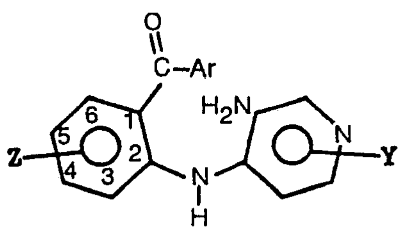

Formula $II_y$

| Example | Ar | Y | Z |
|---|---|---|---|
| 7b) | $C_6H_5-$ | H | H |
| 15f) | $C_6H_5-$ | 2-$CH_3$ | H |

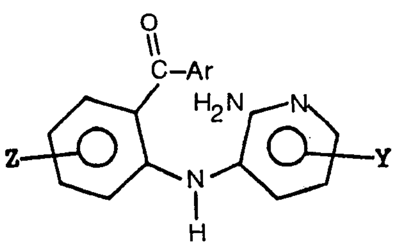

Formula $II_z$

| Example | Ar | Y | Z |
|---|---|---|---|
| 7c) | $C_6H_5-$ | H | H |
| 16a) | $C_6H_5-$ | 5-$CH_3$ | H |
| b) | $C_6H_5-$ | 4,6$(CH_3)_2$ | H |
| c) | $C_6H_5-$ | 5-$C_2H_5$ | H |

The preparation of novel phenyl-substituted pyrido[1,4]benzodiazepine compounds of the present invention and the novel process is exemplified more fully by the following examples 17 to 75. Structures of the compounds of these examples are illustrated in Table 2.

Example 17

6-Phenyl-11H-pyrido[2,3-b][1,4]benzodiazepine.

A mixture of 19.7 g (0.1 mole) of 2-aminobenzophenone and 15.0 g (0.12 mole) of 3-amino-2-chloropyridine was heated under a nitrogen atmosphere at 190°C. for 1.75 hr. The mixture was cooled to room temperature and partitioned between 3 N aqueous sodium hydroxide and methylene chloride. The combined methylene chloride extracts were washed with water, dried over magnesium sulphate and evaporated under reduced pressure. The residue, 32.7 g, was dissolved in benzene and chromatographed on a column of florisil packed in benzene, eluting with benzene and 1—2% acetone-benzene mixtures. After evaporation, the solid was crystallized from benzene to give 7.3 g of product; m.p. 106—108°C.

Analysis: Calculated for $C_{18}H_{13}N_3$: C, 79.68; H, 4.83; N, 15.49
Found: C, 79.70; H, 4.81; N, 15.42

Example 18

8-Chloro-6-phenyl-11H-pyrido[2,3-b][1,4]benzodiazepine

A mixture of 15.0 g (0.0647 mole) of 2-amino-5-chlorobenzophenone and 9.1 g (0.068 mole) of 3-amino-2-chloropyridine was heated at 200°C. (in an oil bath) for 0.75 hr under a nitrogen atmosphere. The mixture was cooled and methylene chloride added. The mixture was stirred for 1 hr then allowed to stand overnight. A brown solid precipitate weighing 8.7 g was separated by filtration. The filtrate was evaporated under reduced pressure. The residue was combined with the brown solid and partitioned with aqueous sodium hydroxide and methylene chloride and crude product isolated as in Example 17, except the crystallizing solvent was ethanol. Recrystallization from ethanol and drying overnight at 82°C at 0.1 mm Hg pressure gave 3.0 g of product; m.p. 156.5—158.5°C.

Analysis: Calculated for $C_{18}H_{12}ClN_3$: C, 70.71; H, 3.96; N, 13.74
Found: C, 70.24; H, 4.01; N, 13.76

Example 19

9-Chloro-6-phenyl-11H-pyrido[2,3-b][1,4]benzodiazepine.

A suspension of 6.6 g (0.02 mole) of [2-[(3-amino-2-pyridyl)amino]-4-chlorophenyl]-(phenyl)-methanone (see Example 2) in 200 ml of toluene was heated at reflux overnight under a nitrogen atmosphere. The reaction mixture was filtered hot and the filtrate heated back to reflux temperature. The precipitate formed in cooling to room temperature was separated by filtration and recrystallized from benzene and dried for 4 hours at 97—98°C at 0.1 mm Hg pressure and overnight temperature at 0.1 mm Hg pressure to give 3.7 g; m.p. 250.5 to 252°C. Elemental analysis for carbon was high and the product was re-dried at 139°C (xylenes in drying pistol) for 8 hr. Although the carbon analysis remained high, the proton nuclear magnetic resonance spectrum and mass spectrum was consistent with the proposed structure.

Analysis: Calculated for $C_{16}H_{12}ClN_3$: C, 70.71; H, 3.96; N, 13.74
Found: C, 71.46; H, 4.06; N, 13.46

Example 20

8-Chloro-6-(2-chlorophenyl)-5,6-dihydro-11H-pyrido[2,3-b][1,4]benzodiazepine.

Further elution of the florisil column in the preparation of Example 4 with 10—15% acetone in benzene and 5—25% methanol in benzene gave two fractions of the title product of this example, 6.4 g and 5.7 g, the second being quite impure. The 6.4 g fraction was recrystallized from benzene-isooctane to give 3.7 g of solid; m.p. 203—6°C. (decomposition) which was identified by chemical ionization mass spec. analysis, $^{1}H$ and $^{1s}C$ NMR as 8-chloro-6-(2-chlorophenyl)-5,6-dihydro-11H-pyrido[2,3-b](1,4]benzodiazepine.

Example 21

6-(4-Chlorophenyl)-11H-pyrido[2,3-b][1,4]benzodiazepine.

A mixture of 23.2 g (0.10 mole) of 2-amino-4'-chlorobenzophenone and 14.7 g (0.11 mole) of 3-amino-2-chloropyridine (96%) were heated for 1.5 hr at 180°C under a nitrogen atmosphere. The mixture was cooled to room temperature and methylene chloride added. After stirring for 30 min., solids were separated by filtration and triturated in hot 190 proof ethanol. The remaining insoluble material was collected by filtration and recrystallized from benzene-isooctane to give 2.7 g product, m.p. 203—204.5°C. Drying conditions prior to analyses were: overnight at 97—98°C at 0.1 mm Hg pressure.

Analysis: Calculated for $C_{18}H_{12}ClN_3$: C, 70.71; H, 3.96; N, 13.74
Found: C, 70.76; H, 3.92; N, 13.95

Example 22

6-(4-Methylphenyl)-11H-pyrido(2,3-b][1,4]benzodiazepine

A solution of 3.6 g (0.012 mole) of [2-[(3-amino-2-pyridyl)amino]phenyl](4-methylphenyl)methanone in 100 ml of anhydrous toluene was treated with a catalytic amount of para toluene sulphonic acid and refluxed overnight while separating water with a Dean-Stark trap. The reaction mixture was filtered while hot. The product precipitated as the filtrate cooled to room temperature and was collected by filtration. The weight of solid after the solvent had evaporated was 2.5 g, m.p. 203.5—205°C. (decomp.).

Analysis: Calculated for $C_{19}H_{15}N_3$: C, 79.98; H, 5.30; N, 14.73
Found: C, 79.95; H, 5.27; N, 14.76

Example 23

6-(4-Methoxyphenyl)-11H-pyrido[2,3-b][1,4]benzodiazepine

A stirred mixture of 20.0 g (0.088 mole) of 2-amino-4'-methoxybenzophenone and 13.0 g (0.097 mole) of 3-amino-2-chloropyridine (96%) was heated at 180°C under a nitrogen atmosphere for 2.0 hr. The reaction mixture was cooled to approximately 70°C and 100 ml of methylene chloride was added slowly. After the mixture had cooled to room temperature another 50 ml of methylene chloride was added and the mixture was stirred overnight. The suspended solid was collected by filtration, air dried, dissolved in methanol and made basic with 3 N sodium hydroxide. The suspension was diluted with 500 ml water and extracted with three 250 ml portions of methylene chloride. The combined methylene chloride extracts were dried over magnesium sulphate and evaporated under reduced pressure. Analysis by mass spectra (EI and CI) indicated the residue was a mixture of [2-[(3-amino-2-pyridyl)-amino]phenyl](4-methoxyphenyl)methanone and the title compound. The residue-mixture was dissolved in 250 ml toluene with a catalytic amount of para toluene sulphonic acid and the solution was refluxed overnight under nitrogen atmosphere while separating water in a Dean-Stark trap. The reaction mixture was filtered while hot. The product precipitated as the filtrate cooled to room temperature and was collected by filtration. After recrystallization from benzene the product weighed 1.8 g, m.p. 198.5—200—5°C. (d).

| | | | |
|---|---|---|---|
| Analysis: Calculated for $C_{19}H_{15}N_3O$: | C, 75.73; | H, 5.02; | N, 13.94 |
| Found: | C, 75.65; | H, 4.98; | N, 14.03 |

Example 24

8-Chloro-N,N-dimethyl-6-phenyl-11H-pyrido[2,3-b][1,4]benzodiazepine-11-propanamine oxalate [1:1]

To a stirred suspension of 1.05 g (0.044 mole) of sodium hydride (in mineral oil) in 50 ml of anhydrous dimethylformamide, under a nitrogen atmosphere was added portionwise, 6.1 g (0.02 mole) of 8-chloro-6-phenyl-11H-pyrido[2,3-b][1,4]benzodiazepine. The reaction mixture was stirred at room temperature for 1.5 hr, during which evolution of hydrogen ceased. To the mixture was added, portionwise, 3.5 g (0.022 mole) of 3-dimethylaminopropylchloride hydrochloride. After stirring overnight at room temperature, the reaction mixture was poured into 1600 ml water and the combination extracted with three 250 ml portions of methylene chloride. The combined methylene chloride extract was washed with two 250 ml portions of water, dried over magnesium sulphate, and evaporated under reduced pressure. The residue was dissolved in benzene and chromatographed with acetone-benzene on a 300 g column of florisil packed in benzene. Starting material, 1.6 g., was recovered in the benzene elution and 3.6 g, containing the product as free base was obtained from the acetone-benzene elution on evaporation of solvent. A portion of the crude free base, 2.5 g., was dissolved in hot isopropyl alcohol and reacted with 0.8 g (0.0064 mole) of oxalic acid dihydrate. The oxalate salt, which precipitated on cooling, was collected by filtration and recrystallized from ethanol to give 2.2 g of product m.p. 206—208°C. Drying conditions prior to analyses were: 5 hr at 97—98°C at 0.02 mm Hg pressure; over night at room temperature at 0.02 mm Hg pressure.

| | | | |
|---|---|---|---|
| Analysis: Calculated for $C_{25}H_{25}ClN_4O_4$: | C, 62.43; | H, 5.24; | N, 11.65 |
| Found: | C, 62.52; | H, 5.23; | N, 11.76 |

Example 25

N,N-Dimethyl-6-phenyl-11H-pyrido[2,3-b][1,4]benzodiazepine-11-propanamine fumarate [1:1]

To a stirred suspension of 1.68 g (0.070 mole) of sodium hydride (in mineral oil) in 25 ml of anhydrous dimethylformamide, under a nitrogen atmosphere, was added, portionwise, a suspension of 8.0 g (0.029 mole) of 6-phenyl-11H-pyrido[2,3-b][1,4]benzodiazepine in 20 ml of anhydrous dimethylformamide. The mixture was stirred for 30 min after addition was complete, warmed to 65°C for 15 min and cooled again to room temperature. To the mixture was added 5.6 g (0.035 mole) of 3-dimethylaminopropyl chloride hydrochloride. After stirring overnight at room temperature, thin-layer chromatography indicated the reaction was nearly completed. The reaction mixture was poured into 1500 ml water and extracted with 250 ml of methylene chloride. The methylene chloride extract was washed with three 250 ml portions of water, dried over magnesium sulphate and evaporated under reduced pressure. The residue was dissolved in methylene chloride and extracted with 100 ml and 150 ml portions of 3 N hydrochlorid acid. Unreacted 6-phenyl-11H-pyrido[2,3-b][1,4]benzodiazepine starting material precipitated from the aqueous acidic solution and was separated by carefully decanting the liquid from the solid. The aqueous solution was basified with 3 N sodium hydroxide and extracted with three 100 ml portions of methylene chloride. The combined methylene chloride extracts were dried over magnesium sulphate and evaporated under reduced pressure to give 7.7 g of residue, the free base of the title compound. A solution of 6.6 g of the residue in hot isopropyl alcohol was reacted with 2.15 g of fumaric acid and the mixture heated until dissolution was completed. On standing for 48 hr, the salt which had precipitated was collected by filtration. After recrystallization from isopropyl alcohol-isopropyl ether, 5.9 g of product was obtained, m.p. 171—173°C. Drying conditions prior to analyses were: 4 hr at 90°C at 0.1 mm Hg pressure; overnight at room temperature at 0.1 mm Hg pressure.

| | | | |
|---|---|---|---|
| Analysis: Calculated for $C_{27}H_{28}H_4O_4$: | C, 68.63; | H, 5.97; | N, 11.86 |
| Found: | C, 68.37; | H, 6.05; | N, 11.73 |

Example 26

N,N-Dimethyl-6-phenyl-11H-pyrido[2,3-b][1,4]benzodiazepin-11-ethanamine fumarate [1:1]

To a stirred suspension of 1.48 g (0.062 mole) of sodium hydride (in mineral oil) in 35 ml of anhydrous dimethylformamide, under nitrogen atmosphere, was added portionwise, 7.0 g (0.026 mole) of 6-phenyl-11H-pyrido[2,3-b][1,4]benzodiazepine. After cooling the reaction mixture to room temperature, 4.46 g (0.031 mole) of 2-dimethylaminoethyl chloride hydrochloride was added portionwise and stirring continued overnight. The reaction mixture was poured into 1500 ml of water and the resultant mixture extracted with 250 ml of methylene chloride. The methylene chloride extract was washed with three 500 ml portions of water, dried over magnesium sulphate and evaporated under reduced pressure to give 8.6 g of an oil, the free base of the title compound. Part of the oil, 6.9 g, was reacted with an equal molar amount of fumaric acid in isopropyl alcohol. Addition of isopropyl ether gave an oily solid. The mixture was evaporated under reduced pressure and the residue crystallized on standing. The crystals were triturated with acetone and recrystallized from acetone-isopropyl ether to give 4.3 g of the fumarate salt, m.p. 175—177.5°C.

| | | | |
|---|---|---|---|
| Analysis: Calculated for $C_{26}H_{26}N_4O_4$: | C, 68.11; | H, 5.72; | N, 12.22 |
| Found: | C, 67.88; | H, 5.72; | N, 12.17 |

Example 27

11-[3-(4-Morpholinyl)propyl]-6-phenyl-11H-pyrido[2,3-b][1,4]benzodiazepine fumarate [1:1]

To a stirred suspension of 1.10 g (0.046 mole) of sodium hydride (in mineral oil) in 25 ml of anhydrous dimethylformamide under nitrogen pressure was added, portionwise, 5.0 g (0.0184 mole) of 6-phenyl-11H-pyrido[2,3-b][1,4]benzodiazepine. The reaction mixture was stirred at room temperature for 15 min, warmed at 65—70°C for 10 min and allowed to cool to room temperature. To the mixture was added portionwise, 4.1 g (0.02 mole) of 4-(3-chloropropyl)morpholine hydrochloride. The reaction mixture was stirred at room temperature for 16 hr and then poured into 800 ml of water. This mixture was extracted twice with 200 ml portions of methylene chloride. The combined methylene chloride extracts were extracted with 150 ml and 75 ml portions of 3 N hydrochloric acid and the combined aqueous extracts were made basic with 3 N sodium hydroxide. The resulting suspension was extracted with two 150 ml portions of methylene chloride and these latter two extracts combined, dried over magnesium sulphate and evaporated under reduced pressure. The residue, the free base of the title compound, was reacted with an equal molar amount of fumaric acid in warm isopropyl alcohol and the mixture treated with isopropyl ether. The fumarate salt was collected by filtration and recrystallized from ethanol-ethyl acetate to give 5.6 g, m.p. 154—7°C. Drying conditions prior to analyses were: 4 hr at 97—98°C at 0.1 mm Hg pressure; overnight at room temperature at 0.1 mm Hg pressure.

| | | | |
|---|---|---|---|
| Analysis: Calculated for $C_{29}H_{30}N_4O_5$: | C, 67.69; | H, 5.88; | N, 10.88 |
| Found: | C, 67.52; | H, 5.84; | N, 10.90 |

Example 28

N,N-Diethyl-6-phenyl-11H-pyrido[2,3-b][1,4]benzodiazepine-11-propanamine oxalate [1:1]

To a stirred suspension of 1.10 g (0.0461 mole) of sodium hydride (in mineral oil) in 25 ml of anhydrous dimethylformamide under a nitrogen atmosphere was added, portionwise, 5.0 g (0.0184 mole) of 6-phenyl-11H-pyrido[2,3-b][1,4]benzodiazepine. The reaction mixture was stirred at room temperature for 0.5 hr, warmed to 65—70°C. and cooled slowly to room temperature. To the mixture was added, portionwise, 3.77 g (0.020 mole) of 3-diethylaminopropylchloride hydrochloride and the reaction mixture stirred at room temperature for 16 hr. The mixture was poured into 750 ml of water and extracted with three 150 ml portions of methylene chloride. The combined methylene chloride extracts were extracted with 150 ml and 75 ml portions of 3 N hydrochloric acid. The combined aqueous extracts were made basic with 3 N sodium hydroxide and then extracted with three 100 ml portions of methylene chloride. The combined methylene chloride extracts were dried over magnesium sulphate and evaporated under reduced pressure to give 7.5 g of the free base of the title compound. A portion, 5.6 g, was reacted with an equal molar amount of oxalic acid dihydrate in hot isopropyl alcohol. The oxalate salt was collected by filtration to give 5.5 g of product, m.p. 196—199°C. Drying conditions prior to analayses were: 1 hr at 97—98°C at 0.1 mm Hg pressure.

| | | | |
|---|---|---|---|
| Analysis: Calculated for $C_{27}H_{30}N_4O_4$: | C, 68.34; | H, 6.37; | N, 11.81 |
| Found: | C, 68.31; | H, 6.43; | N, 11.86 |

Example 29

9-Chloro-N,N-dimethyl-6-phenyl-11H-pyrido[2,3-b][1,4]benzodiazepine-11-propanamine fumarate [1:1]

To a stirred suspension of 0.98 g (0.041 mole) of sodium hydride (in mineral oil) in 25 ml of anhydrous dimethylformamide under a nitrogen atmosphere was added portionwise, 5.0 g (0.016 mole) of 9-chloro-6-phenyl-11H-pyrido[2,3-b][1,4]benzodiazepine over a 45 min period. The reaction mixture was stirred at room temperature for 1 hr, warmed to 70°C and then cooled slowly to room temperature. To the mixture was added, portionwise, over a 30 min period, 2.84 g (0.018 mole); of 3-dimethylaminopropyl chloride hydrochloride and the reaction mixture stirred at room temperature for 17 hr. The mixture was poured into 750 ml of water and extracted with 150 ml and two 100 ml portions of methylene chloride. The combined methylene chloride extracts were washed with 100 ml portions of water followed by extraction with 100 ml

and 75 ml portions of 3 N hydrochloric acid. The acidic extracts were combined and filtered to remove the precipitate which had formed and the filtrate was made basic with 3 N sodium hydroxide and extracted with three 100 ml portions of methylene chloride. The combined methylene chloride extracts were dried over magnesium sulphate and evaporated under reduced pressure. The residue was dissolved in methylene chloride and filtered through a 50—60 g bed of florisil in a sintered glass funnel. The bed was washed in succession with 1%, 2%, 3% and 5% methanol-methylene chloride mixtures, the filtrates combined and evaporated under reduced pressure to give the free base of the title compound. The free base was reacted with an equal molar amount of fumaric acid in hot isopropanol to give 3.3 g of fumarate, m.p. 199—202°C/

Analysis: Calculated for $C_{27}H_{27}N_4O_4Cl$: C, 63.96 H, 5.37; N, 11.05
Found: C, 63.63; H, 5.36; N 11.00

Example 30

6-Phenyl-11-[3-(1-piperidinyl)propyl]-11H-pyrido[2,3-b][1,4]benzodiazepine fumarate [1:1]

To a stirred suspension of 1.10 g (0.0461 mole) of sodium hydride (in mineral oil) in 25 ml of anhydrous dimethylformamide under a nitrogen atmosphere was added, portionwise, 5.0 g (0.018 mole) of 6-phenyl-11H-pyrido[2,3-b][1,4]benzodiazepine. The reaction mixture was stirred for 30 min, warmed to 70°C and cooled to room temperature. To the mixture was added, portionwise, 4.14 g (0.0203 mole) of N-(3-chlorophenyl)piperidine hydrochloride and the reaction mixture was stirred at room temperature for 16 hr. The mixture was poured into 750 ml of water, and extracted with 150 ml of methylene chloride by stirring for 15 min. The aqueous layer was extracted with two additional 100 ml portions of methylene chloride. The combined methylene chloride extracts were extracted with 150 ml and 75 ml portions of 3 N hydrochloric acid and the combined acid extracts made basic with 3 N sodium hydroxide and then extracted with three 100 ml portions of methylene chloride. The methylene chloride extracts were combined, dried over magnesium sulphate and evaporated under reduced pressure. The residue was dissolved in a minimum of methylene chloride and filtered through a 100 g bed of florisil in a sintered glass funnel. The bed was washed successively with methylene chloride, 1%, 2%, 3% and 5% methanol-methylene chloride mixtures. All the filtrates were combined and evaporated under reduced pressure. The residue was reacted with 1.3 g of fumaric acid in hot isopropanol and isopropyl ether added. An amorphous precipitate was formed. The entire mixture was evaporated to dryness and the residue dissolved in 200 ml of ethanol. The solution was warmed to reflux, filtered and isopropyl ether added to the filtrate. Crystals which formed overnight were filtered off to give 4.1 g of fumarate salt, m.p. 153—6°C. Drying conditions prior to analyses were: 4 hr at 97—98°C at 0.1 mm Hg pressure.

Analysis: Calculated for $C_{30}H_{32}N_4O_4$: C, 70.29; H, 6.29; N, 10.93
Found: C, 70.38; H, 6.32; N, 10.92

Example 31

6-(4-Chlorophenyl)-N,N-dimethyl-11H-pyrido[2,3-b][1,4]benzodiazepine-11-propanamine fumarate [1:1]

To a stirred suspension of 1.57 g (0.065 mole) of sodium hydride (in mineral oil) in 25 ml of anhydrous dimethylformamide was added under a nitrogen atmosphere, 8.0 g (0.026 mole) of 6-(4-chlorophenyl)-11H-pyrido[2,3-b][1,4]benzodiazepine. The reaction mixture was stirred for 1 hr at room temperature, warmed at 80°C for 15 min and cooled to room temperature. To the mixture was added, portionwise, 4.55 g (0.029 mole); of 3-dimethylaminopropyl chloride hydrochloride and the mixture was stirred overnight at room temperature. The mixture was poured into 750 ml of water and stirred for 30 min with 150 ml of methylene chloride. The aqueous layer was extracted further with three 100 ml portions of methylene chloride. The combined methylene chloride extracts were dried over magnesium sulphate and evaporated under reduced pressure to give the free base of the title compound. The free base was reacted with an equal molar amount of fumaric acid in hot isopropanol. On cooling, 3.6 g of the fumarate salt precipitated, m.p. 200.5—202.5°C. The product was air dried further prior to analysis.

Analysis: Calculated for $C_{27}H_{27}ClN_4O_4$: C, 63.96; H, 5.37; N, 11.05
Found: C, 64.18; H, 5.33; N, 11.07

Example 32

8-Chloro-N,N-dimethyl-6-phenyl-11H-pyrido[2,3-b][1,4]benzodiazepine-11-ethanamine oxalate [1:1]

To a stirred suspension of 1.05 g (0.044 mole) of sodium hydride (in mineral oil) in 50 ml of anhydrous dimethylformamide was added, portionwise, 6.1 g (0.02 mole) of 8-chloro-6-phenyl-11H-pyrido[2,3-b][1,4]benzodiazepine. The reaction mixture was stirred at room temperature for 1.5 hr, during which time evolution of hydrogen ceased. The reaction mixture was cooled to 5°C and 3.2 g (0.022 mole) of 2-dimethylaminoethyl chloride hydrochloride was added, portionwise, followed by stirring at room temperature for about 60 hr. The reaction mixture was poured into 1600 ml of water and the mixture extracted three times with 500 ml portions of methylene chloride. The combined extracts were washed with two 500 ml portions of water, dried over magnesium sulphate and evaporated under reduced pressure. Thin layer chromatography (20% methanol/benzene on silica gel) indicated the presence of free base of the title compound and of starting material. The residue was dissolved in benzene and chromatographed on a 200 g column of florisil packed in benzene. Starting material, 1.3 g of 8-chloro-6-phenyl-11H-pyrido[2,3-d][1,4]benzodiazepine was eluted with benzene and the free base of the title compound was eluted with mixtures of acetone in benzene. The free base was reacted with an equal molar amount of oxalic acid dihydrate in refluxing isopropyl alcohol and product recrystallized from isopropyl alcohol weighed 1.6 g, m.p. 228—5—232°C. Drying conditions prior to analysis were 6 hr at 82°C at 0.1 mm Hg pressure; overnight at room temperature.

| | | | |
|---|---|---|---|
| Analysis: Calculated for $C_{24}H_{23}ClN_4O_4$: | C, 61.74; | H, 4.96; | N, 12.00 |
| Found: | C, 61.62; | H, 4.95; | N, 11.98 |

Example 33

8-Chloro-11-methyl-6-phenyl-11H-pyrido[2,3-b][1,4]benzodiazepine

To a stirred suspension of 0.25 g (0.01 mole) of sodium hydride (in mineral oil) in 15 ml of anhydrous dimethylformamide was added, portionwise, 3.05 g (0.01 mole) of 8-chloro-6-phenyl-11H-pyrido[2,3-b][1,4]benzodiazepine. The mixture was warmed at about 60°C for one hr. A solution of 1.42 g (0.01 mole) of methyl iodide in 10 ml of anhydrous dimethylformamide was added dropwise over a 0.5 hr period and the reaction mixture stirred overnight at room temperature after which it was poured into 400 ml of water and stirred for 2 hr. The precipitated solid was recrystallized twice from isopropyl alcohol to give 2.0 g of product, m.p. 153—6°C. Drying conditions prior to analyses were 1 hr at 82°C at 0.1 mm Hg pressure.

| | | | |
|---|---|---|---|
| Analysis: Calculated for $C_{19}H_{14}ClN_3$: | C, 71.36; | H, 4.41; | N, 13.14 |
| Found: | C, 71.64; | H, 4.43; | N, 13.32 |

Example 34

N,N-Dimethyl-6-(4-methylphenyl)-11H-pyrido[2,3-b][1,4]benzodiazepine-11-propanamine fumarate [1:1]

To a stirred suspension of 0.51 g (0.022 mole) of sodium hydride in 25 ml of anhydrous dimethylformamide under a nitrogen atmsophere was added, portionwise, 4.2 g (0.0147 mole) of 6-(4-methylphenyl)-11H-pyrido[2,3-b][1,4]benzodiazepine over a 45 min period. The mixture was stirred for 1 hr at room temperature, warmed at 75—80°C for 1 hr, cooled to room temperature and a solution of 0.0184 mole of 3-dimethylaminopropyl chloride in 10 ml of anhydrous dimethylformamide was added dropwise. The mixture was stirred overnight at room temperature and poured into 1000 ml of water. The suspension was extracted with three 150 ml portions of methylene chloride and the combined methylene chloride extracts were extracted with two 150 ml portions of 3 N hydrochloric acid. A precipitate formed in the acidic solution which was removed by filtration and discarded. The filtrate was made basic with 3 N NaOH and extracted with three 100 ml portions of methylene chloride. The combined methylene chloride extracts were dried over magnesium sulphate evaporated under reduced pressure to give an oil, the free base of the title compound. This residual oil was dissolved in hot isopropyl alcohol and reacted with an equal molar amount of fumaric acid. The fumarate salt crystallized as the solution cooled to room temperature and was recrystallized twice from isopropyl alcohol-isopropyl ether to give 1.7 g of product, m.p. 187—189°C (decomp).

| | | | |
|---|---|---|---|
| Analysis: Calculated for $C_{28}H_{30}N_4O_4$: | C, 69.12; | H, 6.22; | N, 11.52 |
| Found: | C, 68.86; | H, 6.32; | N, 11.36 |

Example 35

6(4-Methoxyphenyl)-N,N-dimethyl-11H-pyrido[2,3-b][1,4]benzodiazepine-11-propanamine fumarate [1:1]

To a stirred suspension of 0.45 g (0.0187 mole) of sodium hydride in 25 ml of anhydrous dimethylformamide under a nitrogen atmosphere was added 4.5 g (0.015 mole) of 6-(4-methoxyphenyl)-11H-pyrido[2,3-b][1,4]benzodiazepine over a 30 min period. The mixture was stirred for 30 min at room temperature followed by warming at 80—90°C for one hr, cooling to room temperature and a solution of 0.019 mole of 3-dimethylaminopropyl chloride in 5 ml of anhydrous dimethylformamide was added dropwise. The reaction mixture was stirred overnight at room temperature and poured into 800 ml of water. The suspension was extracted with two 150 ml portions of methylene chloride. The combined extracts were washed with 500 ml of water then extracted with two 100 ml portions of 3 N hydrochloric acid. The solid which precipitated from the combined acidic extracts was filtered off and discarded. The filtrate was made basic with 3N sodium hydroxide and extracted with three 100 ml portions of methylene chloride. The combined methylene chloride extracts were dried over magnesium sulphate and evaporated under reduced pressure. The residual oil had partially crystallized and was triturated in methylene chloride and filtered, leaving a residue of 0.32 g. The filtrate was evaporated under reduced pressure and the residual oil triturated in hot benzene and filtered, leaving a residue of 0.8 g. The benzene filtrate was evaporated under reduced pressure and the residual oil was reacted with 1.02 g fumaric acid in hot isopropyl alcohol. Upon cooling, an oil separated from solution. The supernatant liquid was decanted and the oil seeded. After crystallizing partially, the mixture was filtered to give 2.5 g solid, m.p. 157—60°C. An attempted recrystallization from isopropyl alcohol-isopropyl ether again produced an oil-solid mixture. The mixture was reheated with additional isopropyl alcohol, solubilized, filtered, seeded and cooled. The fumarate salt precipitated and was collected by filtering to give 2.0 g, m.p. 159—161°C.

| | | | |
|---|---|---|---|
| Analysis: Calculated for $C_{28}H_{30}N_4O_5$: | C, 66.92; | H, 6.02; | N, 11.15 |
| Found: | C, 66.90; | H, 6.08; | N, 11.08 |

Example 36

6-(3-Chlorophenyl)-11H-pyrido[2,3-b][1,4]benzodiazepine

A mixture of 14 g (0.0433 mole) of [2-[(3-amino-2-pyridinyl)amino]phenyl](3-chlorophenyl)-methanone and 0.3 g of para toluene sulphonic acid in 500 ml of toluene were heated at reflux overnight using a Dean-Stark trap to collect water. At the end of the reflux time, some of the toluene (ca 250 ml) was distilled off and the hot solution was filtered. Petroleum ether (30—60°C) was added to the cloud point. The solution was refrigerated overnight and filtered to give, after air drying, 10 g (76%) of gold coloured crystals. A portion was recrystallized from isopropyl alcohol-isopropyl ether, m.p. 160—160.5°C.

| | | | |
|---|---|---|---|
| Analysis: Calculated for $C_{18}H_{12}N_3Cl$: | C, 70.71; | H, 3.96; | N, 13.74 |
| Found: | C, 70.47; | H, 3.98; | N, 13.62 |

Example 37

6-(3-Chlorophenyl)-N,N-dimethyl-11H-pyrido[2,3-b][1,4]benzodiazepine-11-propanamine fumarate [1:1]

To a stirred suspension of 3.4 g (0.07 mole) of sodium hydride (in mineral oil) in 250 ml of anhydrous dimethylformamide was added under a nitrogen atmosphere in portions 8.5 g (0.028 mole) of 6-(3-chlorophenyl)-11H-pyrido[2,3-b][1,4]benzodiazepine. The mixture was stirred for 30 min at room temperature. The temperature was raised to 80°C for 3 hr and thereafter allowed to cool to room temperature. To the reaction mixture was added dropwise a solution of 4.9 g (0.031 mole) of 3-dimethylaminopropyl chloride hydrochloride in 30 ml of dimethylformamide over a 20 minute period. The reaction mixture was allowed to stir at room temperature overnight under nitrogen atmosphere. Thin layer chromatography indicated some starting material was present. Additional sodium hydride 1.4 g (0.03 mole) was added and after 15 min 4.7 g (0.03 mole) of 3-dimethylaminopropyl chloride hydrochloride was added followed by stirring for 4.5 hr. Water (20 ml) was added dropwise and the reaction mixture filtered and concentrated on a rotary evaporator. The residue was partitioned between diethyl ether and dilute sodium hydroxide. The ether layer was washed with water 3 times and extracted with dilute aqueous hydrochloric acid. The water layer was made basic with sodium hydroxide pellets and extracted with methylene chloride. The methylene chloride layer was dried and concentrated to give a residue of 7.5 g of product. The free base was reacted with fumaric acid and the fumarate salt recrystallized from ethyl acetate-ethanol, m.p. 167.5—168.5°C.

| | | | |
|---|---|---|---|
| Analysis: Calculated for $C_{23}H_{23}N_4Cl$: | C, 63.96; | H, 5.47; | N, 11.05 |
| Found: | C, 63.95; | H, 5.39; | N, 11.00 |

Example 38

6-(4-Fluorophenyl)-11H-pyrido[2,3-b][1,4]benzodiazepine

A mixture of 11.5 g (0.037 mole) of [2-[(3-amino-2-pyridinyl)amino]phenyl](4-fluorophenyl)methanone and 0.6 g of para toluene sulphonic acid in toluene was refluxed for 24 hr using a Dean-Stark trap to collect water. At the end of reflux, some of the toluene (300 ml) was distilled off and the hot solution was filtered. Petroleum ether (30—60°C) was added to the cloud point. The solution was refrigerated overnight (0°C) and filtered to give 10.7 g crystals. A portion of the material was recrystallized from isopropyl alcohol and dried *in vacuo* overnight at 65°C, m.p. 203—205°C.

| | | | |
|---|---|---|---|
| Analysis: Calculated for $C_{18}H_{12}N_3F$: | C, 74.73; | H, 4.18; | N, 14.52 |
| Found: | C, 74.61; | H, 4.17; | N, 14.54 |

Example 39

6-(4-Fluorophenyl)-N,N-dimethyl-11H-pyrido[2,3-b][1,4]benzodiazepine-11-propanamine hydrochloride hemihydrate

To a stirred suspension of 3.6 g (0.075 mole) of sodium hydride (in mineral oil) in 250 ml of anhydrous dimethylformamide was added under a nitrogen atmosphere in portions of 8.7 g (0.03 mole) of 6-(4-fluorophenyl)-11H-pyrido[2,3-b][1,4]benzodiazepine. The mixture was stirred for 30 min at room temperature. The temperature was raised to 80°C for 3.5 hr and thereafter allowd to cool to 45°C. To the reaction mixture was added dropwise a solution of 5.2 g (0.033 mole) of 3-dimethylaminopropyl chloride hydrochloride in 30 ml of dimethylformamide. After stirring overnight at room temperature, thin layer chromatography indicated the presence of starting material. Additional sodium hydride, 3.6 g (0.075 mole) was added and after 45 min stirring, the reaction mixture was heated to 50—60°C for ½ hr. A green colour developed with formation of gas. The mixture was stirred at room temperature for 3 hr. A solution of 5.2 g (0.033 mole) of 3-dimethylaminopropyl chloride in 30 ml of dimethylformamide was added dropwise. (About half way through the addition, a green colour developed and addition was halted temporarily for about an hour). The reaction mixture was stirred overnight at room temperature. To the mixture was added 30 ml of water while cooling. After gas evolution had stopped the mixture was filtered and concentrated in a rotary evaporator. The residue was partitioned bewteen diethyl ether and water and the ether layer extracted with dilute aqueous hydrochloric acid solution. The aqueous layer was filtered after 1.5 hr to remove solid. The filtrate was made basic with sodium hydroxide pellets and extracted with methylene chloride. The extract was dried and concentrated. The residue was divided into two equal parts and purified by dry column chromatography on two 20″ × 1.5″ (51 × 3.8 cm) columns of silica gel which had been deactivated by the development solvent (10% methanol, 1% concentrated ammonium hydroxide, 89% methylene chloride). The centre portion of the column was cut out and extracted with the development solvent. The combined

extracts were concentrated under reduced pressure and the residue dissolved in ethyl acetate-ethanol mixture and acidified with concentrated hydrochloric acid. The hydrochloric acid salt was recrystallized from an ethanol-ethyl acetate mixture. The solid obtained by filtration was dried at 99°C for 48 hr to give the title compound as the monohydrochloride hemihydrate, m.p. 120—123°C.

Analysis: Calculated for $C_{46}H_{50}N_8F_2Cl_2O$: C, 65.78; H, 6.00; N, 13.34
Found: C, 65.58; H, 5.77; N, 13.47

Example 40

11[3-(1,3-Dihydro-1,3-dioxo-2H-isoindol-2-yl)propyl]-6-phenyl-11H-pyrido[2,3-b][1,4]benzodiazepine

To a stirred suspension of 0.56 g (0.023 mole) of sodium hydride in 25 ml of anhydrous dimethylformamide was added, portionwise, 5.0 g (0.0184 mole) of 6-phenyl-11H-pyrido[2,3-b][1,4]benzodiazepine. The reaction mixture was warmed to 80° ± 2°C for 1 hr and cooled to room temperature. A solution of 5.55 g (0.020 mole) of N-(3-bromopropyl) phthalimide in 10 ml of anhydrous dimethylformamide was added dropwise and after stirring for 16 hr the reactioon mixture was poured into 650 ml of water and stirred for 30 min. The yellow solid was collected by filtration and recrystallized three times from isopropyl alcohol to give 3.7 g of product, m.p. 170—172°C.

Analysis: Calculated for $C_{29}H_{22}N_4O_2$: C, 75.97; H, 4.84; N, 12.22
Found: C, 76.25; H, 4.87; N, 12.34

Example 41

6-Phenyl-11H-pyrido[2,3-b][1,4]benzodiazepine-11-propanamine, dihydrochloride, hemihydrate

A mixture of 16.2 g (0.035 mole) of 6-phenyl-11-[3-(phthalimido)propyl]-11H-pyrido[2,3-b][1,4]benzodiazepine and 2.29 g (0.0387 mole) of hydrazine hydrate, 85% in 175 ml of 190 proof ethyl alcohol was refluxed for 2.5 hr and allowed to stand for 72 hr. A solution of 10 ml conc. hydrochloric acid in 50 ml of water was added to the mixture. The mixture was stirred overnight. The solid precipitate was collected by filtration and discarded. The filtrate was evaporated under reduced pressure. The residue, slightly wet, was suspended in 200 ml of water, the mixture was stirred for 2 hr and filtered through celite. The filtrate was evaporated under reduced pressure and the residue suspended in 100 ml of 200 proof ethyl alcohol and evaporated under reduced pressure. The latter procedure was repeated. The crude, damp residue (42.1 g) was recrystallized from isopropanol with standing for about 15 hr. The solid, collected by filtration, was dried at 82° over phosphorous anhydride at 0.1 mm Hg pressure for 3 hr; m.p. 210—220°C (decomp.).

Analysis: Calculated for $C_{42}H_{46}Cl_4N_8O$: C, 61.47; H, 5.65; N, 13.65
Found: C, 61.36; H, 5.72; N, 13.90

Example 42

6-Phenyl-11H-pyrido[2,3-b][1,4]benzodiazepine-11-propanamine

A portion of the 6-phenyl-11H-pyrido[2,3-b][1,4]benzodiazepine-11-propanamine, dihydrochloride hemihydrate obtained in Example 41 was dissolved in water, made basic with dilute sodium hydroxide, and extracted with three portions of methylene chloride. The combined methylene chloride extracts were filtered through a 50—60 g bed of florisil in a sintered glass funneel. The bed was washed in succession with 1%, 2%, 3% and 5% methanol-methylene chloride mixtures, the filtrates combined and evaporated under reduced pressure to give the free base, the title compound.

Example 43

N-[3-[6-Phenyl-11H-pyrido[2,3-b][1,4]benzodiazepin-11-yl]propyl]methanimidic acid ethyl ester

A solution of 8.8 g (0.021 mole) of 6-phenyl-11H-pyrido[2,3-b][1,4]benzodiazepine-11-propanamine in 150 ml of triethylorthoformate was heated at reflux for 4.5 hr and allowed to stand overnight. The mixture was concentrated *in vacuo*, the residue washed with petroleum ether (30—60°C). Chemical ionization mass spec indicated the product was a mixture containing the title compound.

Example 44

N-Methyl-6-phenyl-11H-pyrido[2,3-b][1,4]benzodiazepine-11-propanamine, dihydrochloride

*Preparation of Imidate Ester*

[Procedure of Crochet, T. A. & Blanton, C. D., Jr. Synthesis 1974 (1) 55—56]

6-Phenyl-11H-pyrido[2,3-b][1,4]benzodiazepine-11-propanamine dihydrochloride hemihydrate, 25 g (0.06 mole) from Example 41 was converted to the free base by partitioning between dilute sodium hydroxide and methylene chloride, drying and concentrating the methylene chloride layer to dryness, adding dry benzene and again concentrating to drive off the benzene. The resulting free base was dissolved in 300 ml (267 g; 1.8 mole) of freshly distilled triethyl orthoformate with refluxing for 9 hr. The mixture was concentrated *in vacuo*, ethanol was added and the mixture concentrated again.

Conversion of Amidate to Amine

The 23.4 g (0.061 mole) of the amidate prepared in the foregoing was dissolved in 200 ml of ethanol and sodium borohydrate added with stirring at 15—20°C. until thin layer chromatography indicated reaction was essentially complete as indicated by substantial absence of starting material. Fifty ml of water

was added slowly with stirring and cooling continued for 15 min after the water addition. The mixture was then flooded with 2 litres of water and extracted with ethyl acetate. The ethyl acetate layer was washed with water until a neutral wash was obtained and was then saturated with sodium chloride. The resulting ethyl acetate layer was dried and concentrated. Diethyl ether was added and the mixture chilled. Some insoluble material was filtered off and discarded. The ether layer was concentrated and the product chromatographed on an alumina column (neutral, activity-1) eluting with ethyl acetate + methanol + traces of triethyl amine. The fractions containing substantial product (TLC) were partitioned between ethyl acetate and aqueous sodium hydroxide. Ethereal hydrogen chloride was added to the ethyl acetate layer and the crystalline product recrystallized from an acetonitrile-water mixture. Melting point of the product was 139—141°C.

| | | | |
|---|---|---|---|
| Analysis: Calculated for $C_{22}H_{24}N_4Cl_2$: | C, 63.62; | H, 5.82; | N, 13.49 |
| Found: | C, 63.81; | H, 6.15; | N, 13.60 |

Example 45

N-[3-[6-Phenyl-11H-pyrido[2,3-b][1,4]benzodiazepine-11-yl]propyl]carbamic acid ethyl ester.

To a solution of 1.6 g (0.0045 mole) of 6-phenyl-11H-pyrido[2,3-b][1,4]benzodiazepine-11-propanamine in dry methylene chloride was added 0.53 g (0.0052) of triethylamine. To this solution was added dropwise, while cooling, 0.54 g (0.0050 mole) of ethyl chloroformate. The mixture was stirred at room temperature for 2 hr. The methylene chloride solution of the product (as indicated by chemical ionization mass spec) was washed with dilute sodium hydroxide-sodium chloride saturated aqueous solution and dried and concentrated to dryness. The residue was triturated in isopropyl ether. Yield was 1.5 g of the title product.

Example 46

5,6-Dihydro-N,N-dimethyl-6-phenyl-11H-pyrido[2,3-b][1,4]benzodiazepine-11-propanamine, dihydrochloride hemihydrate

A solution of 3.0 g (0.0064 mole) of N,N-dimethyl-6-phenyl-11H-pyrido[2,3-b][1,4]benzodiazepine-11-propanamine in absolute methanol was adjustd to pH 5.6 with methanolic hydrogen chloride solution. To this solution was added at one time, 0.7 g (0.011 mole) of $NaBH_3CN$ and the reaction mixture was refluxed for 20 min. The ethanol was removed *in vacuo* and the residue was partitioned between dilute sodium hydroxide and methylene chloride. The methylene chloride layer was dried over magnesium sulphate and concentrated to leave a residue which was twice crystallized from 2-propanol and isopropyl ether. A yellow solid, 1.6 g (57%) was obtained which loses its crystalline structure on heating starting at 156—160°C with decomposition at 180—195°C.

| | | | |
|---|---|---|---|
| Analysis: Calculated for $C_{46}H_{58}N_8OCl_4$: | C, 62.73; | H, 6.64; | N, 12.72 |
| Found: | C, 62.40; | H, 6.90; | N, 12.61 |

Examples 47a to 47r

Following the procedure of Example 22, the following methanone compounds:
[2-[(3-amino-2-pyridinyl)amino]phenyl]-(4-ethylphenyl)methanone,
[2-[(3-amino-2-pyridinyl)amino]phenyl]-(4-isopropylphenyl)methanone,
[2-[(3-amino-2-pyridinyl)amino]phenyl]-(4-bromophenyl)methanone,
[2-[(3-amino-2-pyridinyl)amino]phenyl]-(4-fluorophenyl)methanone,
[2-[(3-amino-2-pyridinyl)amino]phenyl]-(4-ethoxyphenyl)methanone,
[2-[(3-amino-2-pyridinyl)amino]phenyl]-(4-nitrophenyl)methanone,
[2-[(3-amino-2-pyridinyl)amino]phenyl]-(4-trifluoromethylphenyl)methanone,
[2-[(3-amino-2-pyridinyl)amino]phenyl]-(3-methylphenyl)methanone,
[2-[(3-amino-2-pyridinyl)amino]phenyl]-(3-ethylphenyl)methanone,
[2-[(3-amino-2-pyridinyl)amino]phenyl]-(3-methoxyphenyl)methanone,
[2-[(3-amino-2-pyridinyl)amino]phenyl]-(3-ethoxyphenyl)methanone,
[2-[(3-amino-2-pyridinyl)amino]phenyl]-(2-nitrophenyl)methanone,
[2-[(3-amino-2-pyridinyl)amino]phenyl]-(3-trifluoromethylphenyl)methanone,
[2-[(3-amino-2-pyridinyl)amino]phenyl]-(2-methylphenyl)methanone,
[2-[(3-amino-2-pyridinyl)amino]phenyl]-(2-ethylphenyl)methanone,
[2-[(3-amino-2-pyridinyl)amino]phenyl]-(2-methoxyphenyl)methanone,
[2-[(3-amino-2-pyridinyl)amino]phenyl]-(2,4-dichlorophenyl)methanone, and
[2-[(3-amino-2-pyridinyl)amino]phenyl]-(3,4,5-trimethoxyphenyl)methanone,
were cyclized to the following pyridobenzodiazepines:
a) 6-(4-ethylphenyl)-11H-pyrido[2,3-b][1,4]benzodiazepine,
b) 6-(4-isopropylphenyl)-11H-pyrido[2,3-b][1,4]benzodiazepine,
c) 6-(4-bromophenyl)-11H-pyrido[2,3-b][1,4]benzodiazepine,
d) 6-(4-fluorophenyl)-11H-pyrido[2,3-b][1,4]benzodiazepine,
e) 6-(4-ethoxyphenyl)-11H-pyrido[2,3-b][1,4]benzodiazepine,
f) 6-(4-nitrophenyl)-11H-pyrido[2,3-b][1,4]benzodiazepine,
g) 6-(4-trifluoromethylphenyl)-11H-pyrido[2,3-b][1,4]benzodiazepine,
h) 6-(3-methylphenyl)-11H-pyrido[2,3-b][1,4]benzodiazepine,

i) 6-(3-ethylphenyl)-11H-pyrido[2,3-b][1,4]benzodiazepine,
j) 6-(3-methoxyphenyl)-11H-pyrido[2,3-b][1,4]benzodiazepine,
k) 6-(3-ethoxyphenyl)-11H-pyrido[2,3-b][1,4]benzodiazepine,
l) 6-(2-nitrophenyl)-11H-pyrido[2,3-b][1,4]benzodiazepine,
m) 6-(3-trifluoromethylphenyl)-11H-pyrido[2,3-b][1,4]benzodiazepine,
n) 6-(2-methylphenyl)-11H-pyrido[2,3-b][1,4]benzodiazepine,
o) 6-(2-ethylphenyl)-11H-pyrido[2,3-b][1,4]benzodiazepine,
p) 6-(2-methoxyphenyl)-11H-pyrido[2,3-b][1,4]benzodiazepine,
q) 6-(2,4-dichlorophenyl)-11H-pyrido[2,3-b][1,4]benzodiazepine, and
r) 6-(3,4,5-trimethoxyphenyl)-11H-pyrido[2,3-b][1,4]benzodiazepine.

Examples 48a to 48o

Following the procedure of Example 19, the following methanone compounds:
[2-[(3-amino-2-pyridinyl)amino]-5-chlorophenyl](phenyl)methanone,
[2-[(3-amino-2-pyridinyl)amino]-6-chlorophenyl](phenyl)methanone,
[2-[(3-amino-2-pyridinyl)amino]-4-bromophenyl](phenyl)methanone,
[2-[(3-amino-2-pyridinyl)amino]-4-fluorophenyl](phenyl)methanone,
[2-[(3-amino-2-pyridinyl)amino]-4-trifluoromethylphenyl](phenyl)methanone,
[2-[(3-amino-2-pyridinyl)amino]-4-methylphenyl](phenyl)methanone,
[2-[(3-amino-2-pyridinyl)amino]-5-methylphenyl](phenyl)methanone,
[2-[(3-amino-2-pyridinyl)amino]-6-methylphenyl](phenyl)methanone,
[2-[(3-amino-2-pyridinyl)amino]-4-ethylphenyl](phenyl)methanone,
[2-[(3-amino-2-pyridinyl)amino]-4-methoxyphenyl](phenyl)methanone,
[2-[(3-amino-2-pyridinyl)amino]-4-ethoxyphenyl](phenyl)methanone,
[2-[(3-amino-2-pyridinyl)amino]-4-nitrophenyl](phenyl)methanone,
[2-[(3-amino-2-pyridinyl)amino]-5-nitrophenyl](phenyl)methanone,
[2-[(3-amino-2-pyridinyl)amino]-3-methylphenyl](phenyl)methanone, and
[2-[(3-amino-2-pyridinyl)amino]-3-chlorophenyl](phenyl)methanone,
were cyclized to the following benzodiazepines:
a) 8-chloro-6-phenyl-11H-pyrido[2,3-b][1,4]benzodiazepine,
b) 7-chloro-6-phenyl-11H-pyrido[2,3-b][1,4]benzodiazepine,
c) 9-bromo-6-phenyl-11H-pyrido[2,3-b][1,4]benzodiazepine,
d) 9-fluoro-6-phenyl-11H-pyrido[2,3-b][1,4]benzodiazepine,
e) 6-phenyl-9-trifluoromethyl-11H-pyrido[2,3-b][1,4]benzodiazepine,
f) 9-methyl-6-phenyl-11H-pyrido[2,3-b][1,4]benzodiazepine,
g) 8-methyl-6-phenyl-11H-pyrido[2,3-b][1,4]benzodiazepine,
h) 7-methyl-6-phenyl-11H-pyrido[2,3-b][1,4]benzodiazepine,
i) 9-ethyl-6-phenyl-11H-pyrido[2,3-b][1,4]benzodiazepine,
j) 9-methoxy-6-phenyl-11H-pyrido[2,3-b][1,4]benzodiazepine,
k) 9-ethhoxy-6-phenyl-11H-pyrido[2,3-b][1,4]benzodiazepine,
l) 9-nitro-6-phenyl-11H-pyrido[2,3-b][1,4]benzodiazepine,
m) 8-nitro-6-phenyl-11H-pyrido[2,3-b][1,4]benzodiazepine,
n) 10-methyl-6-phenyl-11H-pyrido[2,3-b][1,4]benzodiazepine, and
o) 10-chloro-6-phenyl-11H-pyrido[2,3-b][1,4]benzodiazepine.

Examples 49a to 49r

Utilizing the procedure of Example 31 but substituting equal molar amounts of each of the compounds prepared in Example 47, the following 6-phenyl-substituted pyrido-benzodiazepines were prepared:
a) 6-(4-ethylphenyl)-N,N-dimethyl-11H-pyrido[2,3-b][1,4]benzodiazepine-11-propanamine,
b) N,N-dimethyl-6-[4-(1-methylethyl)phenyl]-11H-pyrido[2,3-b][1,4]benzodiazepine-11-propanamine,
c) 6-(4-bromophenyl)-N,N-dimethyl-11H-pyrido[2,3-b][1,4]benzodiazepine-11-propanamine,
d) 6-(4-fluorophenyl)-N,N-dimethyl-11H-pyrido[2,3-b][1,4]benzodiazepine-11-propanamine,
e) 6-(4-ethoxyphenyl)-N,N-dimethyl-11H-pyrido[2,3-b][1,4]benzodiazepine-11-propanamine,
f) N,N-dimethyl-6-(4-nitrophenyl)-11H-pyrido[2,3-b][1,4]benzodiazepine-11-propanamine,
g) N,N-dimethyl-6-[4-(trifluoromethyl)phenyl]-11H-pyrido[2,3-b][1,4]benzodiazepine-11-propanamine,
h) N,N-dimethyl-6-(3-methylphenyl)-11H-pyrido[2,3-b][1,4]benzodiazepine-11-propanamine,
i) 6-(3-ethylphenyl)-N,N-dimethyl-11H-pyrido[2,3-b][1,4]benzodiazepine-11-propanamine,
j) 6-(3-methoxyphenyl)-N,N-dimethyl-11H-pyrido[2,3-b][1,4]benzodiazepine-11-propanamine,
k) 6-(3-ethoxyphenyl)-N,N-dimethyl-11H-pyrido[2,3-b][1,4]benzodiazepine-11-propanamine,
l) N,N-dimethyl-6-(2-nitrophenyl)-11H-pyrido[2,3-b][1,4]benzodiazepine-11-propanamine,
m) N,N-dimethyl-6-[4-(trifluoromethyl)phenyl]-11H-pyrido[2,3-b][1,4]benzodiazepine-11-propanamine,
n) N,N-dimethyl-6-(2-methylphenyl)-11H-pyrido[2,3-b][1,4]benzodiazepine-11-propanamine,
o) 6-(2-ethylphenyl)-N,N-dimethyl-11H-pyrido[2,3-b][1,4]benzodiazepine-11-propanamine,
p) 6-(2-methoxyphenyl)-N,N-dimethyl-11H-pyrido[2,3-b][1,4]benzodiazepine-11-propanamine,
q) 6-(2,4-dichlorophenyl)-N,N-dimethyl-11H-pyrido[2,3-b][1,4]benzodiazepine-11-propanamine, and
r) N,N-dimethyl-6-(3,4,5-trimethoxyphenyl)-11H-pyrido[2,3-b][1,4]benzodiazepine-11-propanamine.

Examples 50a to 50o

Utilizing the procedure of Example 29 but substituting equal molar amounts of the compounds prepared in Example 48 for 9-chloro-6-phenyl-11H-pyrido[2,3-b][1,4]benzodiazepine, the following pyridobenzodiazepines were prepared:

a) 8-chloro-N,N-dimethyl-6-phenyl-11H-pyrido[2,3-b][1,4]benzodiazepine-11-propanamine,
b) 7-chloro-N,N-dimethyl-6-phenyl-11H-pyrido[2,3-b][1,4]benzodiazepine-11-propanamine,
c) 9-bromo-N,N-dimethyl-6-phenyl-11H-pyrido[2,3-b][1,4]benzodiazepine-11-propanamine,
d) 9-fluoro-N,N-dimethyl-6-phenyl-11H-pyrido[2,3-b][1,4]benzodiazepine-11-propanamine,
e) N,N,dimethyl-6-phenyl-9-(trifluoromethyl)-11H-pyrido[2,3-b][1,4]benzodiazepine-11-propanamine,
f) N,N,9-trimethyl-6-phenyl-11H-pyrido[2,3-b][1,4]benzodiazepine-11-propanamine,
g) N,N,8-trimethyl-6-phenyl-11H-pyrido[2,3-b][1,4]benzodiazepine-11-propanamine,
h) N,N,7-trimethyl-6-phenyl-11H-pyrido[2,3-b][1,4]benzodiazepine-11-propanamine,
i) 9-ethyl-N,N-dimethyl-6-phenyl-11H-pyrido[2,3-b][1,4]benzodiazepine-11-propanamine,
j) 9-methoxy-N,N-dimethyl-6-phenyl-11H-pyrido[2,3-b][1,4]benzodiazepine-11-propanamine,
k) 9-ethoxy-N,N-dimethyl-6-phenyl-11H-pyrido[2,3-b][1,4]benzodiazepine-11-propanamine,
l) N,N-dimethyl-9-nitro-6-phenyl-11H-pyrido[2,3-b][1,4]benzodiazepine-11-propanamine,
m) N,N-dimethyl-8-nitro-6-phenyl-11H-pyrido[2,3-b][1,4]benzodiazepine-11-propanamine,
n) N,N-10,trimethyl-6-phenyl-11H-pyrido[2,3-b][1,4]benzodiazepine-11-propanamine, and
o) 10-chloro-N,N-dimethyl-6-phenyl-11H-pyrido[2,3-b][1,4]benzodiazepine-11-propanamine.

Examples 51a to 51c

Following the procedure of Example 17 and substituting equal molar amounts of the following for 3-amino-2-chloropyridine:

4-amino-3-chloropyridine,
3-amino-4-chloropyridine, and
2-amino-3-chloropyridine,

there were obtained:

a) 6-phenyl-11H-pyrido[3,4-b][1,4]benzodiazepine,
b) 10-phenyl-5H-pyrido[4,3-b][1,4]benzodiazepine, and
c) 10-phenyl-5H-pyrido[3,2-b][1,4]benzodiazepine.

Examples 52a to 52c

Following the procedure of Example 19, the following:

[2-[(4-amino-3-pyridinyl)amino]phenylmethanone,
[2-[(3-amino-4-pyridinyl)amino]phenylmethanone, and
[2-[(2-amino-3-pyridinyl)amino]phenylmethanone,

were converted to:

a) 6-phenyl-11H-pyrido[3,4-b][1,4]benzodiazepine,
b) 10-phenyl-5H-pyrido[4,3-b][1,4]benzodiazepine, and
c) 10-phenyl-5H-pyrido[3,2-b][1,4]benzodiazepine.

Examples 53a to 53c

Following the procedure of Example 25 and substituting equal molar amounts of the following for 6-phenyl-11H-pyrido[2,3-b][1,4]benzodiazepine,

6-phenyl-11H-pyrido[3,4-b][1,4]benzodiazepine,
10-phenyl-5H-pyrido[4,3-b][1,4]benzodiazepine, and
10-phenyl-5H-pyrido[3,2-b][1,4]benzodiazepine.

there were obtained:

a) N,N-dimethyl-6-phenyl-11H-pyrido[3,4-b][1,4]benzodiazepine-11-propanamine fumarate,
b) N,N-dimethyl-10-phenyl-5H-pyrido[4,3-b][1,4]benzodiazepine-5-propanamine fumarate, and
c) N,N-dimethyl-10-phenyl-5H-pyrido[3,2-b][1,4]benzodiazepine-5-propanamine fumarate.

Example 54

5,6-Dihydro-6-phenyl-N-methyl-11H-pyrido[2,3-b][1,4]benzodiazepine-11-propanamine

To a solution of 1.4 g (0.0035 mole) of N-[3-[(6-phenyl)-11H-pyrido[2,3-b][1,4]benzodiazepine-11-yl]propyl]carbamic acid ethyl ester (from Example 45) in tetrahydrofuran under nitrogen gas was added 0.4 g (0.0105 mole) of lithium aluminum hydride and slight exothermic reaction occurred. The mixture was cooled to prevent overheating. The mixture was stirred at reflux temperature for 16 hr. Thin layer chromatography indicated only partial conversion had occurred. An additional 0.4 g (0.0105 mole) of lithium aluminum hydride was added and the mixture refluxed overnight. Thin-layer chromatography indicated the product was predominantly the title compound.

Example 55

6-(2-Thienyl)-11H-pyrido[2,3-b][1,4]benzodiazepine

Following the procedure of Example 36, [2-[(3-amino-2-pyridinyl)amino]phenyl](2-thienyl)methanone

was heated with para toluene sulphonic acid catalyst in organic solvent while removing water in a Dean-Stark strap to give the title compound.

Example 56

6-(3-Thienyl)-11H-pyrido[2,3-b][1,4]benzodiazepine

Following the procedure of Example 36, [2-[(3-amino-2-pyridinyl)amino]phenyl](3-thienyl)methanone was heated with para toluene sulphonic acid catalyst in organic solvent while removing water in a Dean-Stark trap to give the title compound.

Example 57

6-(2-Pyridinyl)-11H-pyrido[2,3-b][1,4]benzodiazepine

Following the procedure of Example 19, [2-[(3-amino-2-pyridinyl)amino]phenyl](2-pyridinyl)methanone was cyclized to the title compound.

Example 58

6-(3-Pyridinyl)-11H-pyrido[2,3-b][1,4]benzodiazepine

Following the procedure of Example 19, [2-[(3-amino-2-pyridinyl)amino]phenyl](3-pyridinyl)methanone was cyclized to the title compound.

Example 59

6-(4-Pyridinyl)-11H-pyrido[2,3-b][1,4]benzodiazepine

Following the procedure of Example 19, [2-[(3-amino-2-pyridinyl)amino]phenyl](4-pyridinyl)methanone was cyclized to the title compound.

Example 60

N,N-Dimethyl-6-(2-thienyl)-11H-pyrido[2,3-b][1,4]benzodiazepine-11-propanamine

Following the procedure of Example 39, 6-(2-thienyl)-11H-pyrido[2,3-b][1,4]benzodiazepine was reacted with sodium hydride followed by reaction with 3-dimethylaminopropyl chloride to give the title compound.

Example 61

N,N-Dimethyl-6-(3-thienyl)-11H-pyrido[2,3-b][1,4]benzodiazepine-11-propanamine

Following the procedure of Example 39, 6-(3-thienyl)-11H-pyrido[2,3-b][1,4]benzodiazepine was reacted with sodium hydride followed by reaction with 3-dimethylaminopropyl chloride to give the title compound.

Example 62

N,N-Dimethyl-6-(2-pyridinyl)-11H-pyrido[2,3-b][1,4]benzodiazepine-11-propanamine

Following the procedure of Example 39, 6-(2-pyridinyl)-11H-pyrido[2,3-b][1,4]benzodiazepine was reacted with sodium hydride followed by reaction with 3-dimethylaminopropyl chloride to give the title compound.

Example 63

N,N-Dimethyl-6-(3-pyridinyl)-11H-pyrido[2,3-b][1,4]benzodiazepine-11-propanamine

Following the procedure of Example 39, 6-(3-pyridinyl)-11H-pyrido[2,3-b][1,4]benzodiazepine was reacted with sodium hydride followed by reaction with 3-dimethylaminopropyl chloride to give the title compound.

Example 64

N,N-Dimethyl-6-(4-pyridinyl)-11H-pyrido[2,3-b][1,4]benzodiazepine-11-propanamine

Following the procedure of Example 39, 6-(4-pyridinyl)-11H-pyrido[2,3-b][1,4]benzodiazepine was reacted with sodium hydride followed by reaction with 3-dimethylaminopropyl chloride to give the title compound.

Examples 65a to 65g

Following the procedure of Example 22, the following methanone compounds of Example 15:

[2-[(3-amino-4-methyl-2-pyridinyl)amino]phenyl]phenylmethanone,
[2-[(3-amino-5-methyl-2-pyridinyl)amino]phenyl]phenylmethanone,
[2-[(3-amino-6-methyl-2-pyridinyl)amino]phenyl]phenylmethanone,
[2-[(3-amino-5,6-dimethyl-2-pyridinyl)amino]phenyl]phenylmethanone,
[2-[(3-amino-6-methoxy-2-pyridinyl)amino]phenyl]phenylmethanone,
[2-[(3-amino-2-methyl-4-pyridinyl)amino]phenyl]phenylmethanone, and
[2-[(3-amino-5-methoxy-2-pyridinyl)amino]phenyl]phenylmethanone,

were converted to the following pyridobenzodiazepines:

a) 4-methyl-6-phenyl-11H-pyrido[2,3-b][1,4]benzodiazepine,
b) 3-methyl-6-phenyl-11H-pyrido[2,3-b][1,4]benzodiazepine,
c) 2-methyl-6-phenyl-11H-pyrido[2,3-b][1,4]benzodiazepine,
d) 2,3-dimethyl-6-phenyl-11H-pyrido[2,3-b][1,4]benzodiazepine,
e) 2-methoxy-6-phenyl-11H-pyrido[2,3-b][1,4]benzodiazepine,
f) 1-methyl-10-phenyl-5H-pyrido[4,3-b][1,4]benzodiazepine, and
g) 3-methoxy-6-phenyl-11H-pyrido[2,3-b][1,4]benzodiazepine.

Examples 66a to 66g

Following the procedure of Example 39, the pyridobenzodiazepines prepared in Example 65 are reacted with sodium hydride and 3-dimethylaminopropyl chloride to give the following:

a) N,N,4-trimethyl-6-phenyl-11H-pyrido[2,3-b][1,4]benzodiazepine-11-propanamine,
b) N,N,3-trimethyl-6-phenyl-11H-pyrido[2,3-b][1,4]benzodiazepine-11-propanamine,
c) N,N,2-trimethyl-6-phenyl-11H-pyrido[2,3-b][1,4]benzodiazepine-11-propanamine,
d) N,N,2,3-tetramethyl-6-phenyl-11H-pyrido[2,3-b][1,4]benzodiazepine-11-propanamine,
e) 2-methoxy-N,N-dimethyl-6-phenyl-11H-pyrido[2,3-b][1,4]benzodiazepine-11-propanamine,
f) N,N,1-trimethyl-10-phenyl-5H-pyrido[4,3-b][1,4]benzodiazepine-5-propanamine, and
g) 3-methoxy-N,N-dimethyl-6-phenyl-11H-pyrido[2,3-b][1,4]benzodiazepine-11-propanamine.

Examples 67a to 67g

Following the procedure of Example 38 but substituting the following for [2-[(3-amino-2-pyridinyl)amino]phenyl](4-fluorophenyl)methanone:

[2-[(3-amino-2-pyridinyl)amino]phenyl](2-fluorophenyl)methanone,
[2-[(3-amino-2-pyridinyl)amino]phenyl](2-chlorophenyl)methanone, and
[2-[(3-amino-2-pyridinyl)amino]phenyl](2-bromophenyl)methanone,

there were obtained:

a) 6-(2-fluorophenyl)-11H-pyrido[2,3-b][1,4]benzodiazepine,
b) 6-(2-chlorophenyl)-11H-pyrido[2,3-b][1,4]benzodiazepine, and
c) 6-(2-bromophenyl)-11H-pyrido[2,3-b][1,4]benzodiazepine.

Examples 68a to 68c

Following the procedure of Example 39, substituting the following pyrido[1,4]benzodiazepines for 6-(4-fluorophenyl)-11H-pyrido[2,3-b][1,4]benzodiazepine:

6-(2-fluorophenyl)-11H-pyrido[2,3-b][1,4]benzodiazepine,
6-(2-chlorophenyl)-11H-pyrido[2,3-b][1,4]benzodiazepine,
6-(2-bromophenyl)-11H-pyrido[2,3-b][1,4]benzodiazepine,

there were obtained:

a) 6-(2-fluorophenyl)-N,N-dimethyl-11H-pyrido[2,3-b][1,4]benzodiazepine-11-propanamine, m.p. 92—94°C; recrystallizing solvent isopropyl alcohol-isopropyl ether,

b) 6-(2-chlorophenyl)-N,N-dimethyl-11H-pyrido[2,3-b][1,4]benzodiazepine-11-propanamine, m.p. 104—105°C; recrystallizing solvent: isopropyl ether, and

c) 6-(2-bromophenyl)-N,N-dimethyl-11H-pyrido[2,3-b][1,4]benzodiazepine-11-propanamine, m.p. 96—98°C; recrystallizing solvent: isopropyl ether.

Examples 69a and 69b

Following the procedure of Example 25, the following were substituted for 3-dimethylaminopropyl chloride:

3-dimethylamino-2-methylpropyl chloride, and
4-dimethylaminobutyl chloride,

there were obtained:

a) N,N,β-trimethyl-6-phenyl--11H-pyrido[2,3-b][1,4]benzodiazepine-11-propanamine fumarate, and
b) N,N-dimethyl-6-phenyl-11H-pyrido[2,3-b][1,4]benzodiazepine-11-butanamine fumarate.

Examples 70a and 70b

When in the procedure of Example 27 the following were substituted for 4-(3-chloropropyl)morpholine hydrochloride:

1-(3-chloropropyl)pyrrolidine hydrochloride, and
1-(3-chloropropyl)-4-methylpiperazine hydrochloride,

there are obtained:

6-phenyl-11-[3-(1-pyrrolidinyl)propyl]-11H-pyrido[2,3-b][1,4]benzodiazepine, and
6-phenyl-11-[3-(4-methyl-1-piperazinyl)propyl]-11H-pyrido[2,3-b][1,4]benzodiazepine.

Examples 71a to 71c

When in the procedure of Example 25 the following were substituted for 6-phenyl--11H-pyrido[2,3-b][1,4]benzodiazepine:

8-methyl-6-phenyl-11H-pyrido[3,4-b][1,4]benzodiazepine,
6-(4-chlorophenyl)-11H-pyrido[3,4-b][1,4]benzodiazepine, and
3-methoxy-6-phenyl-11H-pyrido[3,4-b][1,4]benzodiazepine,

there are obtained:

a) N,N,8-trimethyl-6-phenyl--11H-pyrido[3,4-b][1,4]benzodiazepine-11-propanamine, and
b) 6-(4-chlorophenyl)-N,N-dimethyl-11H-pyrido[3,4-b][1,4]benzodiazepine-11-propanamine, and
c) 3-methoxy-N,N-dimethyl-6-phenyl-11H-pyrido[3,4-b][1,4]benzodiazepine-11-propanamine.

Examples 72a to 72d

When in the procedure of Example 33 the following were substituted for 8-chloro-6-phenyl--11H-pyrido[2,3-b][1,4]benzodiazepine:

6-phenyl-11H-pyrido[2,3-b][1,4]benzodiazepine,
8-chloro-6-(2-nitrophenyl)-11H-pyrido[2,3-b][1,4]benzodiazepine,
8-chloro-6-(2-chlorophenyl)-11H-pyrido[2,3-b][1,4]benzodiazepine, and
8-chloro-6-(2-bromophenyl)-11H-pyrido[2,3-b][1,4]benzodiazepine,

there were obtained:

a) 11-methyl-6-phenyl-11H-pyrido[2,3-b][1,4]benzodiazepine,

b) 8-chloro-11-methyl-6-(2-nitrophenyl)-11H-pyrido[2,3-b][1,4]benzodiazepine, m.p. 165—166°C, recrystallizing solvent: ethyl alcohol,

c) 8-chloro-6-(2-chlorophenyl)-11-methyl-11H-pyrido[2,3-b][1,4]benzodiazepine, m.p. 150—152°C, recrystallizing solvent: isopropyl alcohol-isopropyl ether, and

d) 6-(2-bromophenyl)-8-chloro-11-methyl-11H-pyrido[2,3-b][1,4]benzodiazepine, m.p. 121—123°C, recrystallizing solvent: isopropyl ether.

Example 73

N-Methyl-N-[3-(11H-pyrido[2,3-b][1,4]benzodiazepine-11-yl)propyl]carbamic acid methyl ester.

The title compound was prepared by reacting 6-phenyl-11H-pyrido[2,3-b][1,4]benzodiazepine and (3-chloropropyl) methylcarbamic acid methyl ester.

Example 74

9-Hydroxy-N,N-dimethyl-6-phenyl-11H-pyrido[2,3-b][1,4]benzodiazepine-11-propanamine.

The title compound was prepared by reacting 11-[3-(dimethylamino)propyl]-9-methoxy-6-phenyl-11H-pyrido[2,3-b][1,4]benzodiazepine with hydrogen iodide and glacial acetic acid.

Example 75

3-Hydroxy-N,N-dimethyl-6-phenyl-11H-pyrido[2,3-b][1,4]benzodiazepine-11-propanamine

The title compound was prepared by reacting 3-methoxy-N,N-dimethyl-6-phenyl-11H-pyrido[2,3-b]-[1,4]benzodiazepine-11-propanamine with hydrogen iodide and glacial acetic acid.

TABLE 2

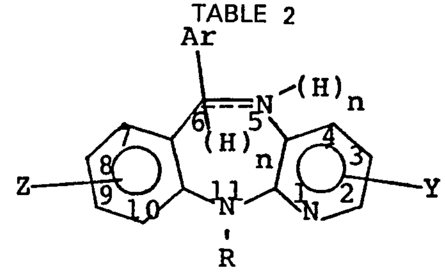

Formula $I_w$

Substituents

| Example | R | Ar | Y | Z | n | Salt |
|---|---|---|---|---|---|---|
| 17 | H | $C_8H_5$— | H | H | 0 | — |
| 18 | H | $C_8H_5$ | H | 8-Cl | 0 | — |
| 19 | H | $C_8H_5$ | H | 9-Cl | 0 | — |
| 20 | H | 2-Cl—$C_8H_4$— | H | 8-Cl | 1 | — |
| 21 | H | 4-Cl—$C_8H_4$ | H | H | 0 | — |
| 22 | H | 4-$CH_3$—$C_8H_4$— | H | H | 0 | — |
| 23 | H | 4-$OCH_3$—$C_8H_4$— | H | H | 0 | — |
| 24 | —$(CH_2)_3$—$N(CH_3)_2$ | $C_8H_5$— | H | 8-Cl | 0 | oxalate |
| 25 | —$(CH_2)_3$—$N(CH_3)_2$ | $C_8H_5$— | H | H | 0 | fumarate |
| 26 | —$(CH_2)_2$—$N(CH_3)_2$ | $C_8H_5$— | H | H | 0 | fumarate |
| 27 | —$(CH_2)_3$—4-morpholinyl | $C_8H_5$— | H | H | 0 | fumarate |
| 28 | —$(CH_2)_3$—$N(C_2H_5)_2$ | $C_8H_5$— | H | H | 0 | oxolate |
| 29 | —$(CH_2)_3$—$N(CH_3)_2$ | $C_8H_5$— | H | 9-Cl | 0 | fumarate |
| 30 | —$(CH_2)_3$—1-piperidinyl | $C_8H_5$— | H | H | 0 | fumarate |
| 31 | —$(CH_2)_3$—$N(CH_3)_2$ | 4-Cl—$C_8H_4$— | H | H | 0 | fumarate |

TABLE 2 (continued)

Substituents

| Example | R | Ar | Y | Z | n | Salt |
|---|---|---|---|---|---|---|
| 32 | $—(CH_2—N(CH_3)_2$ | $C_8H_5$ | H | 8-Cl | 0 | oxalate |
| 33 | $—CH_3$ | $C_8H_5—$ | H | 8-Cl | 0 | — |
| 34 | $—(CH_2)_3—N(CH_3)_2$ | $4\text{-}CH_3—C_8H_4—$ | H | H | 0 | fumarate |
| 35 | $—(CH_2)_3—N(CH_3)_2$ | $4\text{-}OCH_3—C_8H_4—$ | H | H | 0 | fumarate |
| 36 | H | $3\text{-}Cl—C_8H_4—$ | H | H | 0 | — |
| 37 | $—(CH_2)_3—N(CH_3)_2$ | $3\text{-}Cl—C_8H_4—$ | H | H | 0 | fumarate |
| 38 | H | $4\text{-}F—C_7H_4—$ | H | H | 0 | — |
| 39 | $—(CH_2)_3—N(CH_3)_2$ | $4\text{-}F—C_8H_4—$ | H | H | 0 | HCl, $\frac{1}{2}$ $H_2O$ |
| 40 | $—(CH_2)_3—$1-phthalimidoyl | $C_8H_5—$ | H | H | 0 | — |
| 41 | $—(CH_2)_3—NH_2$ | $C_8H_5—$ | H | H | 0 | 2 HCl, 2 $H_2O$ |
| 42 | $—(CH_2)_3—NH_2$ | $C_8H_5—$ | H | H | 0 | — |
| 43 | $—(CH_2)_3—N{=}CH—OC_2H_5$ | $C_8H_5—$ | H | H | 0 | — |
| 44 | $—(CH_2)_3—NHCH_3$ | $C_8H_5—$ | H | H | 0 | 2 HCl |
| 45 | $—(CH_2)_3—NHC(O)—OC_2H_5$ | $C_8H_5—$ | H | H | 0 | — |
| 46 | $—(CH_2)_3—N(CH_3)_2$ | $C_8H_5—$ | H | H | 1 | 2 HCl, 0.5 $H_2O$ |
| 47a) | H | $4\text{-}C_2H_5—C_8H_4$ | H | H | 0 | — |
| b) | H | $4\text{-}i—CH_6H_7—C_8H_4$ | H | H | 0 | — |
| c) | H | $4\text{-}Br—C_8H_4$ | H | H | 0 | — |
| d) | H | $4\text{-}F—C_8H_4$ | H | H | 0 | — |

TABLE 2 (continued)

| Example | R | Ar | Y | Z | n | Salt |
|---|---|---|---|---|---|---|
| | | Substituents | | | | |
| e) | H | $4\text{-}OC_2H_5—C_8H_4$ | H | H | 0 | — |
| f) | H | $4\text{-}NO_2—C_8H_4$ | H | H | 0 | — |
| g) | H | $4\text{-}CF_3—C_8H_4$ | H | H | 0 | — |
| h) | H | $4\text{-}CH_3—C_8H_4$ | H | H | 0 | — |
| i) | H | $3\text{-}C_2H_5—C_8H_4$ | H | H | 0 | — |
| j) | H | $3\text{-}OCH_3—C_8H_4$ | H | H | 0 | — |
| k) | H | $3\text{-}OC_2H_5—C_8H_4$ | H | H | 0 | — |
| l) | H | $2\text{-}NO_2—C_8H_4$ | H | H | 0 | — |
| m) | H | $3\text{-}CF_3—C_8H_4$ | H | H | 0 | — |
| n) | H | $2\text{-}CH_3—C_8H_4$ | H | H | 0 | — |
| o) | H | $2\text{-}C_2H_5—C_8H_4$ | H | H | 0 | — |
| p) | H | $2\text{-}OCH_3—C_8H_4$ | H | H | 0 | — |
| q) | H | $2,4(Cl)_2—C_8H_3$ | H | H | 0 | — |
| r) | H | $3,4,5\text{-}(OCH_3)_3C_8H_2—$ | H | H | 0 | — |
| 48a) | H | $C_8H_5—$ | H | 8-Cl | 0 | — |
| b) | H | $C_8H_5—$ | H | 7-Cl | 0 | — |
| c) | H | $C_8H_5—$ | H | 9-Br | 0 | — |
| d) | H | $C_8H_5—$ | H | 9-F | 0 | — |
| e) | H | $C_8H_5—$ | H | $9\text{-}CF_3$ | 0 | — |

TABLE 2 (continued)

Substituents

| Example | R | Ar | Y | Z | n | Salt |
|---|---|---|---|---|---|---|
| f) | H | $C_8H_5$— | H | 9-$CH_3$ | 0 | — |
| g) | H | $C_8H_5$— | H | 8-$CH_3$ | 0 | — |
| h) | H | $C_8H_5$— | H | 7-$CH_3$ | 0 | — |
| i) | H | $C_8H_5$— | H | 9-$C_2H_5$ | 0 | — |
| j) | H | $C_8H_5$— | H | 9-$OCH_3$ | 0 | — |
| k) | H | $C_8H_5$— | H | 9-$OC_2H_5$ | 0 | — |
| l) | H | $C_8H_5$— | H | 9—$NO_2$ | 0 | — |
| m) | H | $C_8H_5$— | H | 8-$NO_2$ | 0 | — |
| n) | H | $C_8H_5$— | H | 10-$CH_3$ | 0 | — |
| o) | H | $C_8H_5$— | H | 10-Cl | 0 | — |
| 49a) | —$(CH_2)_3$—$N(CH_3)_2$ | 4-$C_2H_5$—$C_8H_4$— | H | H | 0 | — |
| b) | —$(CH_2)_3$—$N(CH_3)_2$ | 4-i-$C_3H$——$C_8H_4$— | H | H | 0 | — |
| c) | —$(CH_2)_3$—$N(CH_3)_2$ | 4-Br—$C_8H_4$— | H | H | 0 | — |
| d) | —$(CH_2)_3$—$N(CH_3)_2$ | 4-F—$C_8H_4$— | H | H | 0 | — |
| e) | —$(CH_2)_3$—$N(CH_3)_2$ | 4-$OC_2H_5$—$C_8H_4$— | H | H | 0 | — |
| f) | —$(CH_2)_3$—$N(CH_3)_2$ | 4-$NO_2$—$C_8H_4$— | H | H | 0 | — |
| g) | —$(CH_2)_3$—$N(CH_3)_2$ | 4-$CF_3$—$C_8H_4$— | H | H | 0 | — |
| h) | —$(CH_2)_3$—$N(CH_3)_2$ | 3-$CH_3$—$C_8H_4$— | H | H | 0 | — |
| i) | —$(CH_2)_3$—$N(CH_3)_2$ | 3-$C_2H_5$—$C_8H_4$— | H | H | 0 | — |

TABLE 2 (continued)

Substituents

| Example | R | Ar | Y | Z | n | Salt |
|---|---|---|---|---|---|---|
| j) | $-(CH_2)_3-N(CH_3)_2$ | $3\text{-}OCH_3-C_8H_4-$ | H | H | 0 | — |
| k) | $-(CH_2)_3-N(CH_3)_2$ | $3\text{-}OC_2H_5-C_8H_4-$ | H | H | 0 | — |
| l) | $-(CH_2)_3-N(CH_3)_2$ | $2\text{-}NO_2-C_8H_4-$ | H | H | 0 | — |
| m) | $-(CH_2)_3-N(CH_3)_2$ | $3\text{-}CF_3-C_8H_4-$ | H | H | 0 | — |
| n) | $-(CH_2)_3-N(CH_3)_2$ | $2\text{-}CH_3-C_8H_4-$ | H | H | 0 | — |
| o) | $-(CH_2)_3-N(CH_3)_2$ | $2\text{-}C_2H_5-C_8H_4-$ | H | H | 0 | — |
| p) | $-(CH_2)_3-N(CH_3)_2$ | $2\text{-}OCH_3-C_8H_4-$ | H | H | 0 | — |
| q) | $-(CH_2)_3-N(CH_3)_2$ | $2,4\text{-}(Cl)_2-C_8H_3-$ | H | H | 0 | — |
| r) | $-(CH_2)_3-N(CH_3)_2$ | $3,4,5\text{-}(OCH_3)_3-C_8H_2-$ | H | H | 0 | — |
| 50a) | $-(CH_2)_3-N(CH_3)_2$ | $C_8H_5-$ | H | 8-Cl | 0 | — |
| b) | $-(CH_2)_3-N(CH_3)_2$ | $C_8H_5-$ | H | 7-Cl | 0 | — |
| c) | $(CH_2)_3-N(CH_3)_2$ | $C_8H_5-$ | H | 9-Br | 0 | — |
| d) | $-(CH_2)_3-N(CH_3)_2$ | $C_8H_5-$ | H | 9-F | 0 | — |
| e) | $-(CH_2)_3-N(CH_3)_2$ | $C_8H_5-$ | H | $9\text{-}CF_3$ | 0 | — |
| f) | $-(CH_2)_3-N(CH_3)_2$ | $C_8H_5-$ | H | $9\text{-}CH_3$ | 0 | — |
| g) | $-(CH_2)_3-N(CH_3)_2$ | $C_8H_5-$ | H | $8\text{-}CH_3$ | 0 | — |
| h) | $-(CH_2)_3-N(CH_3)_2$ | $C_8H_5-$ | H | $7\text{-}CH_3$ | 0 | — |
| i) | $-(CH_2)_3-N(CH_3)_2$ | $C_8H_5-$ | H | $9\text{-}C_2H_5$ | 0 | — |
| j) | $-(CH_2)_3-N(CH_3)_2$ | $C_8H_5-$ | H | $9\text{-}OCH_3$ | 0 | — |

TABLE 2 (continued)

Substituents

| Example | R | Ar | Y | Z | n | Salt |
|---|---|---|---|---|---|---|
| k) | $-(CH_2)_3-N(CH_3)_2$ | $C_8H_5-$ | H | $9\text{-}OC_2H_5$ | 0 | — |
| l) | $-(CH_2)_3-N(CH_3)_2$ | $C_8H_5-$ | H | $9\text{-}NO_2$ | 0 | — |
| m) | $-(CH_2)_3-N(CH_3)_2$ | $C_8H_5-$ | H | $8\text{-}NO_2$ | 0 | — |
| n) | $-(CH_2)_3-N(CH_3)_2$ | $C_8H_5-$ | H | $10\text{-}CH_3$ | 0 | — |
| o) | $-(CH_2)_3-N(CH_3)_2$ | $C_8H_5-$ | H | 10-Cl | 0 | — |
| 51 | $-(CH_2)_3NHCH_3$ | $C_8H_5-$ | H | H | 1 | — |
| 52 | H | 2-thienyl | H | H | 0 | — |
| 53 | H | 3-thienyl | H | H | 0 | — |
| 54 | H | 2-pyridinyl | H | H | 0 | — |
| 55 | H | 3-pyridinyl | H | H | 0 | — |
| 56 | H | 4-pyridinyl | H | H | 0 | — |
| 57 | $-(CH_2)_3-N(CH_3)_2$ | 2-thienyl | H | H | 0 | — |
| 58 | $-(CH_2)_3-N(CH_3)_2$ | 3-thienyl | H | H | 0 | — |
| 59 | $-(CH_2)_3-N(CH_3)_2$ | 2-pyridinyl | H | H | 0 | — |
| 60 | $-(CH_2)_3-N(CH_3)_2$ | 3-pyridinyl | H | H | 0 | — |
| 61 | $-(CH_2)_3-N(CH_3)_2$ | 4-pyridinyl | H | H | 0 | — |
| 62a) | H | $C_8H_5-$ | $4\text{-}CH_3$ | H | 0 | — |
| b) | H | $C_8H_5-$ | $3\text{-}CH_3$ | H | 0 | — |
| c) | H | $C_8H_5-$ | $2\text{-}CH_3$ | H | 0 | — |

TABLE 2 (continued)

Substituents

| Example | R | Ar | Y | Z | n | Salt |
|---|---|---|---|---|---|---|
| d) | H | $C_8H_5$— | 2,3-$(CH_3)_2$ | H | | |
| e) | H | $C_8H_5$— | 2-$OCH_3$ | H | 0 | — |
| g) | H | $C_8H_5$— | 3-$OCH_3$ | H | 0 | — |
| 63a) | —$(CH_2)_3$—$N(CH_3)_2$ | $C_8H_5$— | 4-$CH_3$ | H | 0 | — |
| b) | —$(CH_2)_3$—$N(CH_3)_2$ | $C_8H_5$— | 3-$CH_3$ | H | 0 | — |
| c) | —$(CH_2)_3$—$N(CH_3)_2$ | $C_8H_5$— | 2-$CH_3$ | H | 0 | — |
| d) | —$(CH_2)_3$—$N(CH_3)_2$ | $C_8H_5$— | 2,3-$(CH_3)_2$ | H | 0 | — |
| e) | —$(CH_2)_3$—$N(CH_3)_2$ | $C_8H_5$— | 2-$OCH_3$ | H | 0 | — |
| g) | —$(CH_2)_3$—$N(CH_3)_2$ | $C_8H_5$— | 3-$OCH_3$ | H | 0 | — |
| 64a) | H | 2-F—$C_8H_4$— | H | H | 0 | — |
| b) | H | 2-CL—$C_8H_4$— | H | H | 0 | — |
| c) | H | 2-BR—$C_8H_4$— | H | H | 0 | — |
| 65a) | —$(CH_2)_3$—$N(CH_3)_2$ | 2-F—$C_8H_4$— | H | H | 0 | — |
| b) | —$(CH_2)_3$—$N(CH_3)_2$ | 2-Cl—$C_8H_4$— | H | H | 0 | — |
| c) | —$(CH_2)_3$—$N(CH_3)_2$ | 2-Br—$C_8H_4$— | H | H | 0 | — |
| 66a) | —$CH_2CH(CH_3)CH_2$—$N(CH_3)_2$ | $C_8H_5$— | H | H | 0 | fumarate |
| b) | —$(CH_2)_4$—$N(CH_3)_2$ | $C_8H_5$— | H | H | 0 | fumarate |
| 67a) | —$(CH_2)_3$—1-pyrrolidinyl | $C_8H_5$— | H | H | 0 | — |
| b) | —$(CH_2)_3$—4-methylpiperazin-1-yl | $C_8H_5$— | H | H | 0 | — |

TABLE 2 (continued)

Substituents

| Example | R | Ar | Y | Z | n | Salt |
|---|---|---|---|---|---|---|
| 72a) | $-CH_3$ | $C_8H_4-$ | H | H | 0 | — |
| b) | $-CH_3$ | $2\text{-}NO_2-C_8H_4-$ | H | 8-Cl | 0 | — |
| c) | $-CH_3$ | $2\text{-}Cl-C_8H_4-$ | H | 8-Cl | 0 | — |
| d) | $-CH_3$ | $2\text{-}Br-C_8H_4-$ | H | 8-Cl | 0 | — |
| 73 | $-(CH_2)_3-N(CH_3)$ $-C(O)OCH_3$ | $C_8H_5-$ | H | H | 0 | — |
| 74 | $-(CH_2)_3-N(CH_3)_2$ | $C_8H_5-$ | H | 9-OH | 0 | — |
| 75 | $-(CH_2)_3-N(CH_3)_2$ | $C_8H_5-$ | 3-OH | H | 0 | — |

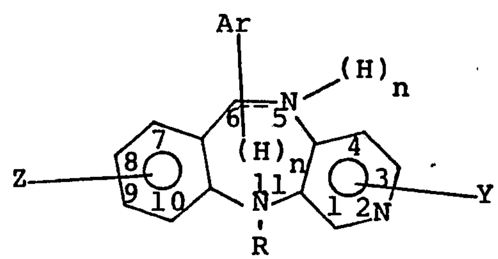

Formula $I_x$

| | | | | | | |
|---|---|---|---|---|---|---|
| 51a) | H | $C_8H_5-$ | H | H | 0 | — |
| 52a) | H | $C_8H_5-$ | H | H | 0 | — |
| 53a) | $-(CH_2)_3-N(CH_3)_2$ | $C_8H_5-$ | H | H | 0 | fumarate |
| 71a) | $-(CH_2)_3-N(CH_3)_2$ | $C_8H_5-$ | H | $8\text{-}CH_3$ | 0 | — |
| b) | $-(CH_2)_3-N(CH_3)_2$ | $C_8H_5-$ | $3\text{-}OCH_3$ | H | 0 | — |
| c) | $-(CH_2)_3-N(CH_3)_2$ | $C_8H_5-$ | H | H | 0 | — |

TABLE 2 (continued)

Substituents

| Example | R | Ar | Y | Z | n | Salt |
|---|---|---|---|---|---|---|
| | | 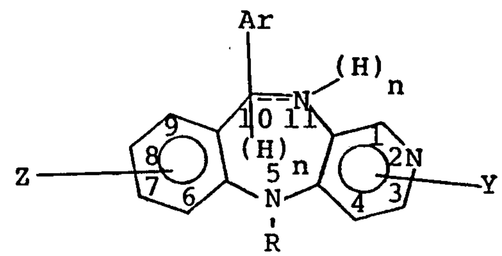 | | Formula $I_y$ | | |
| 51b) | H | $C_8H_5$— | H | H | 0 | — |
| 52b) | H | $C_8H_5$— | H | H | 0 | — |
| 53b) | $-(CH_2)_3-N(CH_3)_2$ | $C_8H_5$— | H | H | 0 | fumarate |
| 62f) | H | $C_8H_5$— | 1-$CH_3$ | H | 0 | — |
| 63f) | $-(CH_2)_3-N(CH_3)_2$ | $C_8H_5$— | 1-$CH_3$ | H | 0 | — |
| | | 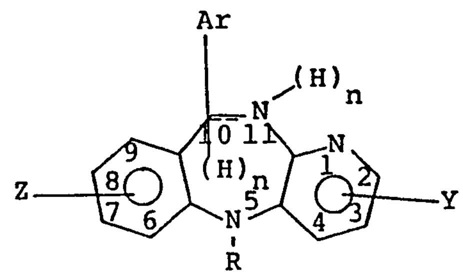 | | Formula $I_z$ | | |
| 51c) | H | $C_8H_5$— | H | H | 0 | — |
| 52c) | H | $C_8H_5$— | H | H | 0 | — |
| 53c) | $-(CH_2)_3-N(CH_3)_2$ | $C_8H_5$— | H | H | 0 | fumarate |

Formulation and Administration

Effective quantities of the foregoing pharmacologically active compounds of Formula Ip or Formula II may be administered to humans for therapeutic purposes according to usual modes of administration and in usual forms, such as orally in solutions, emulsions, suspensions, pills, tablets or capsules, in pharmaceutically acceptable carriers or parenterally in the form of sterile solutions.

Exemplary of solid carriers for oral administration are such as lactose, magnesium, stearate, terra alba, sucrose, talc, stearic acid, gelatin, agar, pectin or acacia.

Exemplary of liquid carriers for oral administration are vegetable oils and water.

For intramuscular administration the carrier or excipient may be a sterile, parenterally acceptable liquid; e.g., water or a parenterally acceptable oil; e.g. arachis oil contained in ampoules.

Although very small quantities of the active materials of the present invention are effective when minor therapy is involved or in cases of administration to subjects having a relatively low body weight, unit dosages are usually from five milligrams or above and preferably 10, 25, 50, or 100 milligrams or even higher, preferably administered three or four times per day, depending, of course, upon the emergency of the situation, the compound used, and the particular result desired. Twenty-five to 200 milligrams appears optimum per unit dose or usual broader ranges appear to be about 10 to 500 milligrams per unit dose. Daily dosages usually required should range from about 0.3 to about 20 mg/kg/day, preferably 0.3 to 10 mg/kg for the more active compunds. The active ingredients of the invention may be combined with other compatible pharmacologically active agents. It is only necessary that the active ingredient constitute an effective amount; i.e., such that a suitable effective dosage will be obtained consistent with the dosage form employed. Obviously, several unit dosage forms may be administered at about the same time. The exact individual dosages as well as daily dosages will, of course, be determined according to standard medical principles under the direction of a physician.

The following formulations are representative for the pharmacologically active compounds of this invention.

FORMULATIONS

Examples 76a to 76e

Capsules of 10 mg and 50 mg of active ingredient per capsule are prepared. With the higher amounts of active ingredient, reduction may be made in the amount of lactose.

| Example | 76a | 76b |
|---|---|---|
| Typical Blend for encapsulation | 10 mg Per Capsule | 50 mg Per Capsule |
| Active ingredient, as salt | 10 | 50 |
| Lactose | 259 | 219 |
| Starch | 126 | 126 |
| Magnesium stearate | 4 | 4 |
| Total | 399 | 399 |

Additional capsule formulations preferably contain a higher dosage of active ingredient and are as follows:

| Example | 76c | 76d | 76e |
|---|---|---|---|
| Ingredients | 100 mg. per Capsule | 250 mg. per Capsule | 500 mg. per Capsule |
| Active ingredient, as salt | 100 | 250 | 500 |
| Lactose | 214 | 163 | 95 |
| Starch | 87 | 81 | 47 |
| Magnesium stearate | 4 | 6 | 8 |
| Total | 299 | 500 | 650 |

In each case, the selected active ingredient was uniformly blended with the lactose, starch, and magnesium stearate and the blend then encapsulated.

Example 77

A typical formulation for a tablet containing 5.0 mg of active ingreident per tablet follows. The formulation may be used for other strengths of active ingredient by adjustment of the weight of dicalcium phosphate.

| | Per Tablet, mg. |
|---|---|
| 1. Active ingredient | 10.0 |
| 2. Corn starch | 15.0 |
| 3. Corn starch (paste) | 12.0 |
| 4. Lactose | 35.0 |
| 5. Dicalcium phosphate | 132.0 |
| 6. Calcium stearate | 2.0 |
| Total | 202.0 |

Ingredients 1, 2, 4 and 5 were first uniformly blended. Ingredient 3 was prepared as a 10% paste in water. The blend was granulated with the starch paste and the wet mass passed through an 8 mesh (U.S. sieve series) screen. The wet granulation was dried and sized through a 12 mesh (U.S. sieve series) screen. The dried granules were blended with the calcium stearate and compressed. 8 mesh has openings of 2.38 mm; 12 mesh of 1.68 mm.

Example 78

An injectable sterile 2% solution was made up as follows:

| | Per cc |
|---|---|
| Active ingredient mg. | 20 |
| Preservative, e.g. chlorobutanol, w/vol. percent | 0.5 |
| Water for injection q.s. | |

The solution was prepared, clarified by filtration, filled into vials, sealed and autoclaved.

**Claims for the Contracting States: BE FR DE IT LU NL SE CH LI GB**

1. A compound having the formula

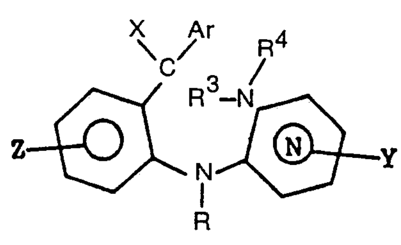

wherein;

R represents a hydrogen atom, a $(C_1—C_8)$alkyl, an -alk$^1$-NR$^1$R$^2$ or an -alk$^1$-N=CH—$OC_2H_5$ group;

R$^1$ and R$^2$ each represent a hydrogen atom, or a $C_1—C_8$ alkyl, or a —C(O)O—$(C_1—C_8)$alkyl group, or R$^1$ and R$^2$ taken together with the adjacent nitrogen atom may form a 1-phthlimido, 1-piperidinyl, 1-pyrrolidinyl, 4-morpholino, 1-piperazino, or 4-substituted piperazin-1-yl heterocyclic residue;

Ar represents a 2, 3 or 4-pyridinyl, a 2 or -3-thienyl, or a phenyl or a substituted phenyl group, the said substituted phenyl group being substituted with 1 to 3 halo, $(C_1—C_8)$alkyl, $(C_1—C_8)$alkoxy, trifluoromethyl or nitro radicals which may be the same or different;

Alk$^1$ represents a straight or branched hydrocarbon chain containing 1—8 carbon atoms;

Z represents a hydrogen atom, or a halogen atom or a $(C_1—C_8)$alkyl, a $(C_1—C_8)$alkoxy, a hydroxy or a nitro group;

Y represents a hydrogen atom or one or two ($C_1$—$C_8$)alkyl, ($C_1$—$C_8$)alkoxy or hydroxy radicals which may be the same or different; and

R3 and R4 each represent a hydrogen atom

X represents =0 or Q1 where Q1 represents a —C=O group or a carbonyl equivalent such as a ketal, thioketal or gem dihalide; and

acid addition salts thereof.

2. A compound having the formula

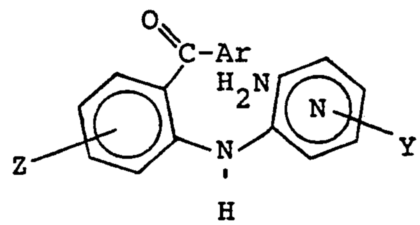

wherein;

Ar represents a 2, 3 or 4-pyridinyl, a 2 or 3-thienyl, a phenyl or a substituted phenyl group, the said substituted phenyl group being substituted with one to three halo, ($C_1$—$C_8$)alkyl, ($C_1$—$C_8$)alkoxy, trifluoromethyl or nitro radicals which may be the same or different;

Z represents a hydrogen atom, or a halogen atom, or a ($C_1$—$C_8$)alkyl, a ($C_1$—$C_8$)alkoxy, a hydroxy, or a nitro group;

Y represents a hydrogen atom, or one or two ($C_1$—$C_8$) alkyl ($C_1$—$C_8$)alkoxy or hydroxy radicals which may be the same or different; and the pharmaceutically acceptable acid addition salt thereof.

3. The compound of Claim 1 which is [2-[(3-amino-2-pyridinyl)amino]phenyl]phenylmethanone.

4. The compound of Claim 1 which is [2-[(3-amino-2-pyridinyl)amino]-4-chlorophenyl]phenylmethanone.

5. The compound of Claim 1 which is [2-[(3-amino-2-pyridinyl)amino]phenyl](4-methylphenyl)-methanone.

6. The compound of Claim 1 which is [2-[(3-amino-2-pyridinyl)amino]-5-chlorophenyl](2-chlorophenyl)methanone.

7. The compound of Claim 1 which is [2-[(3-amino-2-pyridinyl)amino]-3-chlorophenyl]phenylmethanone.

8. The compound of Claim 1 which is [2-[(3-amino-2-pyridinyl)amino]-4-fluorophenyl]phenylmethanone.

9. The compound of Claim 1 which is [2-[(3-amino-2-pyridinyl)amino]phenyl](3-chlorophenyl)methanone.

10. The compound of Claim 1 which is [2-[(3-amino-2-pyridinyl)amino]phenyl](4-fluorophenyl)methanone.

11. The compound of Claim 1 which is [2-[(3-amino-2-pyridinyl)amino]phenyl]-(3-trifluoromethylphenyl)methanone.

12. The compound of Claim 1 which is [2-[(3-amino-2-pyridinyl)amino]phenyl]-(3-fluorophenyl)methanone.

13. The compound of Claim 1 which is [2-[(3-amino-2-pyridinyl)amino]phenyl]-(2-fluorophenyl)methanone.

14. The compound of Claim 1 which is [2-[(3-amino-2-pyridinyl)amino]phenyl]-(2-chlorophenyl)methanone.

15. The compound of Claim 1 which is [2-[(3-amino-2-pyridinyl)amino]phenyl]-(2-bromophenyl)methanone.

16. A compound as claimed in any one of Claims 1 to 15 for use in treating depression.

17. A pharmaceutical composition for treating depression in unit dosage form comprising (a) an effective amount of a compound as claimed in any one of Claims 2 to 15 and (b) a pharmaceutical carrier therefor.

18. Pyrido[1,4]benzodiazepines which are 6-aryl-11H-pyrido[2,3-b][1,4]benzodiazepines or the 5,6 dihydro derivatives thereof, and which have the formula $I_w$, namely

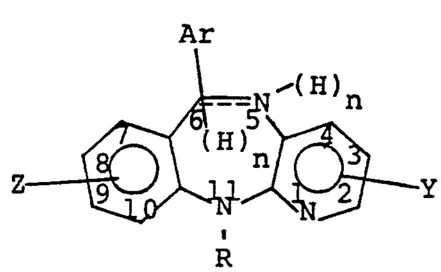

which are 6-aryl-11H-pyrido[3,4-b][1,4]benzodiazepines or the 5,6 dihydro derivative thereof, and which have the formula $I_x$, namely

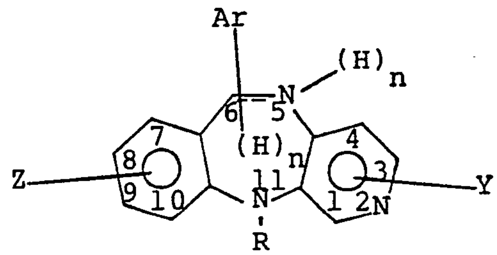

or which are 10-aryl-5H-pyrido[4,3-b][1,4]benzodiazepines, or the 10,11 dihydro derivatives thereof, and which have the formula $I_y$, namely

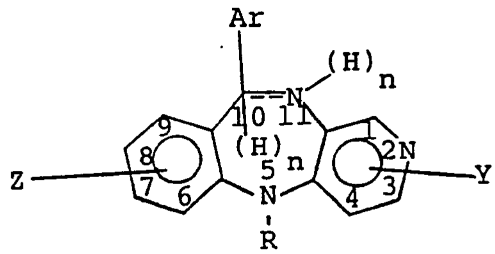

or which are 10-aryl-5H-pyrido[3,2-b][1,4]benzodiazepines or the 10,11 dihydro derivatives thereof, and which have the formula $I_z$, namely

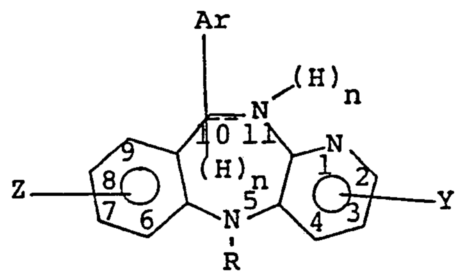

wherein;

R represents a hydrogen atom, $(C_1-C_8)$alkyl, an -alk$^1$-NR$^1$R$^2$ or an -alk$^1$-N=CH—$OC_2H_5$ group;

R$^1$ and R$^2$ each represent a hydrogen atom, or a $(C_1-C_8)$alkyl, or a —C(O)O—$(C_1-C_8)$alkyl group, or R$^1$ and R$^2$ taken together with the adjacent nitrogen atom may form a 1-phthalimido, 1-piperidinyl, 1-pyrrolidinyl, 4-morpholino, 1-piperazino, or 4-substituted piperazin-1-yl residue;

Ar represents a 2, 3 or 4-pyridinyl, a 2 or 3-thienyl, or a phenyl or a substituted phenyl group, the said substituted phenyl group being substituted with one to three halo, $(C_1-C_8)$alkyl, $(C_1-C_8)$alkoxy, trifluoromethyl or nitro radicals which may be the same or different;

Alk$^1$ represents a straight or branched hydrocarbon chain containing 1—8 carbon atoms;

Z represents a hydrogen atom, or a halogen atom or a $(C_1-C_8)$alkyl, a $(C_1-C_8)$alkoxy, a hydroxy or a nitro group;

Y represents a hydrogen atom or 1 or 2 $(C_1-C_8)$alkyl, $(C_1-C_8)$alkoxy or hydroxy radicals which may be the same or different;

n is 0 or 1 and when n is zero the dotted line is a double bond; and the acid addition salts thereof, but excluding the compound which has the formula $I_w$, wherein Z and Y both represent a hydrogen atom, R represents a $(CH_2)_3$—$N(CH_3)_2$ group and Ar represents a $C_6H_5$— group.

19. A compound as claimed in Claim 18 in which R represents a $(C_1-C_8)$alkyl or an -alk$^1$-NR$^1$R$^2$ group;

R$^1$ and R$^2$ each represent a hydrogen atom, or a $(C_1-C_8)$alkyl group, or R$^1$ and R$^2$ taken together with the adjacent nitrogen atom may form a 1-piperidinyl, 1-pyrrolidinyl, 4-morpholinyl, or 4-loweralkyl-1-piperazinyl residue,

Ar represents a 2, 3 or 4-pyridinyl, a 2 or 3-thienyl, or a phenyl or substituted phenyl group, the said substituted phenyl group being substituted with one to three halo $(C_1-C_8)$alkyl, $(C_1-C_8)$alkoxy, trifluoromethyl or nitro radicals which may be the same or different;

alk$^1$ represents a straight or branched hydrocarbon chain containing 1—8 carbon atoms;

Z represents a hydrogen atom, or a halogen atom, or a $(C_1-C_8)$alkyl, a $(C_1-C_8)$alkoxy, a hydroxy, or a nitro group;

Y represents a hydrogen atom, or one or two $(C_1-C_8)$alkyl, $(C_1-C_8)$alkoxy, or hydroxy radicals which may be the same or different;

n is 0 or 1 and when n is zero the dotted line is a double bond; and the pharmaceutically acceptable acid addition salts thereof.

20. A compound as claimed in Claim 18 which is a 6-aryl-11H-pyrido[2,3-b][1,4]benzodiazepine or a 5,6 dihydro derivative thereof and has the formula

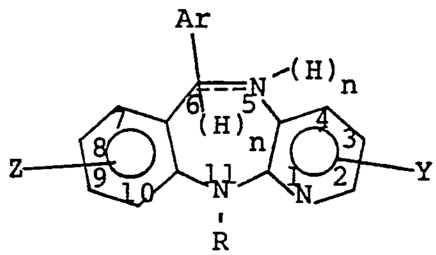

21. The compound of Claim 18 which is 6-phenyl-11H-pyrido[2,3-b][1,4]benzodiazepine.
22. The compound of Claim 18 which is 8-chloro-6-phenyl-11H-pyrido[2,3-b][1,4]benzodiazepine.
23. The compound of Claim 18 which is 9-chloro-6-phenyl-11H-pyrido[2,3-b][1,4]benzodiazepine.
24. The compound of Claim 18 which is 6-(4-chlorophenyl)-11H-pyrido[2,3-b][1,4]benzodiazepine.
25. The compound of Claim 18 which is 6-(4-methylpheny)-11H-pyrido[2,3-b][1,4]benzodiazepine.
26. The compound of Claim 18 which is 6-(4-methoxyphenyl)-11H-pyrido[2,3-b][1,4]benzodiazepine.
27. The compound of Claim 18 which is 8-chloro-N,N-dimethyl-6-phenyl-11H-pyrido[2,3-b][1,4]benzodiazepine-11-propanamine.
28. The compound of Claim 18 which is 8-chloro-N,N-dimethyl-6-phenyl-11H-pyrido[2,3-b][1,4]benzodiazepine-11-propanamine oxalate [1:1].
29. The compound of Claim 18 which is N,N-dimethyl-6-phenyl-11H-pyrido[2,3-b][1,4]benzodiazepine-11-ethanamine.
30. The compound of Claim 18 which is N,N-dimethyl-6-phenyl-11H-pyrido[2,3-b][1,4]benzodiazepine-11-ethanamine fumarate [1:1].
31. The compound of Claim 18 which is 11-[3-(4-morpholinyl)propyl]-6-phenyl-11H-pyrido[2,3-b][1,4]benzodiazepine.
32. The compound of Claim 18 which is 11-[3-(4-morpholinyl)propyl]-6-phenyl-11H-pyrido[2,3-b][1,4]benzodiazepine fumarate [1:1].
33. The compound of Claim 18 which is N,N-diethyl-6-phenyl-11H-pyrido[2,3-b][1,4]benzodiazepine-11-propanamine.
34. The compound of Claim 18 which is N,N-diethyl-6-phenyl-11H-pyrido[2,3-b][1,4]benzodiazepine-11-propanamine oxalate [1:1].
35. The compound of Claim 18 which is 9-chloro-N,N-dimethyl-6-phenyl-11H-pyrido[2,3-b][1,4]benzodiazepine-11-propanamine.
36. The compound of Claim 18 which is 9-chloro-N,N-dimethyl-6-phenyl-11H-pyrido[2,3-b][1,4]benzodiazepine-11-propanamine fumarate [1:1].
37. The compound of Claim 18 which is 6-phenyl-11-[3-(1-piperidinyl)propyl]-11H-pyrido[2,3-b][1,4]benzodiazepine.
38. The compound of Claim 18 which is 6-phenyl-11-[3-(1-piperidinyl)propyl]-11H-pyrido[2,3-b][1,4]benzodiazepine fumarate [1:1].
39. The compound of Claim 18 which is 6-(4-chlorophenyl)-N,N-dimethyl-11H-pyrido[2,3[b][1,4]benzodiazepine-11-propanamine.
40. The compound of Claim 18 which is 6-(4-chlorophenyl)-N,N-dimethyl-11H-pyrido[2,3-b][1,4]benzodiazepine-11-propanamine fumarate [1:1].
41. The compound of Claim 18 which is 8-chloro-N,N-dimethyl-6-phenyl-11H-pyrido[2,3-b][1,4]benzodiazepine-11-ethanamine.
42. The compound of Claim 18 which is 8-chloro-N,N-dimethyl-6-phenyl-11H-pyrido[2,3-b][1,4]benzodiazepine-11-ethanamine oxalate [1:1].
43. The compound of Claim 18 which is 8-chloro-11-methyl-6-phenyl-11H-pyrido[2,3-b][1,4]benzodiazepine.
44. The compound of Claim 18 which is N,N-dimethyl-6-(4-methylphenyl)-11H-pyrido[2,3-b][1,4]benzodiazepine-11-propanamine.
45. The compound of Claim 18 which is N,N-dimethyl-6-(4-methylphenyl)-11H-pyrido[2,3-b[1,4]benzodiazepine-11-propanamine fumarate [1:1].
46. The compound of Claim 18 which is 6-(4-methoxyphenyl)-N,N-dimethyl-11H-pyrido[2,3-b][1,4]benzodiazepine-11-propanamine.
47. The compound of Claim 18 which is 6-(4-methoxyphenyl)-N,N-dimethyl-11H-pyrido[2,3-b][1,4]benzodiazepine-11-propanamine fumarate [1:1].
48. The compound of Claim 18 which is 6-(3-chlorophenyl)-11H-pyrido[2,3-b][1,4]benzodiazepine.
49. The compound of Claim 18 which is 6-(3-chlorophenyl)-N,N-dimethyl-11H-pyrido[2,3-b][1,4]benzodiazepine-11-propanamine.

50. The compound of Claim 18 which is 6-(4-fluorophenyl)-11H-pyrido[2,3-b][1,4]benzodiazepine.

51. The compound of Claim 18 which is 6-(4-fluorophenyl)-N,N-dimethyl-11H-pyrido[2,3-b][1,4]benzodiazepine-11-propanamine.

52. The compound of Claim 18 which is 11-[3-(1,3-dihydro-1,3-dioxo-2H-isoindol-2-yl)propyl]6-phenyl-11H-pyrido[2,3-b][1,4]benzodiazepine.

53. The compound of Claim 18 which is 6-phenyl-11H-pyrido[2,3-b][1,4]benzodiazepine-11-propanamine.

54. The compound of Claim 18 which is 6-phenyl-11H-pyrido[2,3-b][1,4]benzodiazepine-11-propanamine dihydrochloride hemihydrate.

55. The compound of Claim 18 which is N-[3-[6-phenyl-11H-pyrido[2,3-b][1,4]benzodiazepine-11-yl]propyl]methanimidic acid ethyl ester.

56. The compound of Claim 18 which is N-methyl-6-phenyl-11H-pyrido[2,3-b][1,4]benzodiazepine-11-propanamine.

57. The compound of Claim 18 which is N-methyl-6-phenyl-11H-pyrido[2,3-b][1,4]benzodiazepine-11-propanamine dihydrochloride.

58. The compound of Claim 18 which is N-[3-[6-phenyl-11H-pyrido[2,3-b][1,4]benzodiazepine-11-yl]propyl]carbamic acid ethyl ester.

59. The compound of Claim 18 which is 5,6-dihydro-N,N-dimethyl-6-phenyl-11H-pyrido[2,3-b[1,4]benzodiazepine-11-propanamine.

60. The compound of Claim 18 which is 5,6-dihydro-N,N-dimethyl-6-phenyl-11H-pyrido[2,3-b][1,4]benzodiazepine-11-propanamine, dihydrochloride hemihydrate.

61. The compound of Claim 18 which is 8-chloro-6-(2-chlorphenyl)-5,6-dihydro-11H-pyrido[2,3-b][1,4]benzodiazepine.

62. The compound of Claim 18 which is 5,6-dihydro-N-methyl-6-phenyl-11H-pyrido[2,3-b][1,4]benzodiazepine-11-propanamine.

63. The compound of Claim 18 which is 6-(2-fluorophenyl)-N,N-dimethyl-11H-pyrido[2,3-b][1,4]benzodiazepine-11-propanamine.

64. The compound of Claim 18 which is 6-(2-chlorophenyl)-N,N-dimethyl-11H-pyrido[2,3-b][1,4]benzodiazepine-11-propanamine.

65. The compound of Claim 18 which is 6-(2-bromophenyl)-N,N-dimethyl-11H-pyrido[2,3-b][1,4]benzodiazepine-11-propanamine.

66. The compound of Claim 18 which is 8-chloro-11-methyl-6-(2-nitrophenyl)-11H-pyrido[2,3-b][1,4]benzodiazepine.

67. The compound of Claim 18 which is 8-chloro-6-(2-chlorophenyl)-11-methyl-11H-pyrido[2,3-b][1,4]benzodiazepine.

68. The compound of Claim 18 which is 6-(2-bromophenyl)-8-chloro-11-methyl-11H-pyrido[2,3-b][1,4]benzodiazepine.

69. The compound of Claim 18 which is 6-(3-chlorophenyl)-N,N-dimethyl-11H-pyrido[2,3-b[1,4]benzodiazepine-11-propanamine fumarate [1:1].

70. The compound of Claim 18 which is 6-(4-fluorophenyl)-N,N-dimethyl-11H-pyrido[2,3-b][1,4]benzodiazepine-11-propanamine hydrochloride hemihydrate.

71. The compound as claimed in Claim 18 which is a 6-aryl-11H-pyrido[3,4-b][1,4]benzodiazepine or a 5,6 dihydro derivative thereof and has the formula

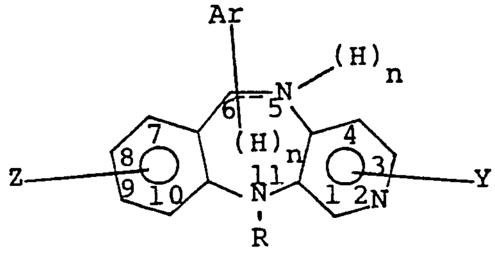

72. The compound as claimed in Claim 18 which is a 10-aryl-5H-pyrido[4,3-b][1,4]benzodiazepine or a 10, 11 dihydro derivative thereof and has the formula

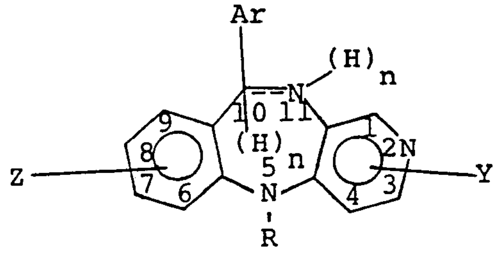

73. The compound as claimed in Claim 18 which is a 10-aryl-5H-pyrido[3,2-b][1,4]benzodiazepine or a 10, 11 dihydro derivative thereof and has the formula

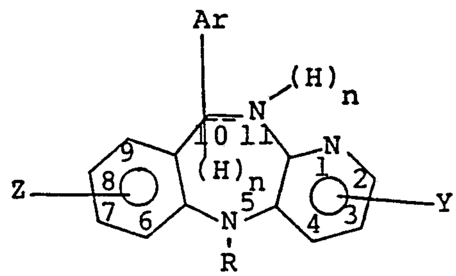

74. A compound as claimed in any one of Claims 18 to 73 for use in treating depression.

75. A pharmaceutical composition for treating depression in unit dosage form comprising (a) an effective amount of a compound as claimed in any one of Claims 18 to 74, and (b) a pharmaceutical carrier therefor.

76. The pharmaceutical composition of Claim 75 wherein the compound is a pharmaceutically acceptable salt of N-methyl-6-phenyl-11H-pyrido[2,3-b][1,4]benzodiazepine-11-propanamine.

77. The pharmaceutical composition of Claim 75 wherein the compound is a pharmaceutically acceptable salt of 6-phenyl-11H-pyrido[2,3-b[1,4]benzodiazepine-11-propanamine.

78. The pharmaceutical composition of Claim 75 wherein the compound is 8-chloro-N,N-dimethyl-6-phenyl-11H-pyrido[2,3-b][1,4]benzodiazepine-11-propanamine.

79. The pharmaceutical composition of Claim 75 wherein the compound is 6-(3-chlorophenyl)-N,N-dimethyl-11H-pyrido[2,3-b][1,4]benzodiazepine-11-propanamine fumarate [1:1].

80. The pharmaceutical composition of Claim 75 wherein the compound is a pharmaceutically acceptable acid addition salt of 6-(4-fluorophenyl)-N,N-dimethyl-11H-pyrido[2,3-b][1,4]benzodiazepine-11-propanamine.

81. The pharmaceutical composition of Claim 75 wherein the compound is 6-(2-chlorophenyl)-N,N-dimethyl-11H-pyrido[2,3-b][1,4]benzodiazepine-11-propanamine.

82. The use of a compound as claimed in Claim 1 in the preparation of an antidepressant agent.

83. The use of a compound as claimed in any one of Claims 1 to 15 and 18 to 73 in the preparation of an antidepressant agent.

84. A compound as claimed in Claim 1 for use in medicine.

85. A compound as claimed in any one of Claims 1 to 15 and 18 to 73 for use in medicine.

**Claims for the Contracting State: AT**

1. A process for the preparation of a compound having the formula

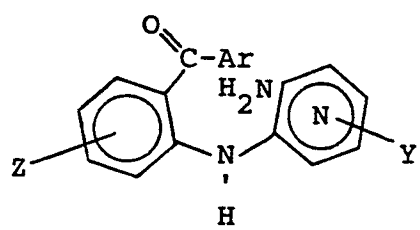

wherein;

Ar represents a 2, 3 or 4-pyridinyl, a 2 or 3-thienyl, or a phenyl or a substituted phenyl group, the said substituted phenyl group being substituted with 1 to 3 halo, $(C_1—C_8)$alkyl, $(C_1—C_8)$alkoxy, trifluoromethyl or nitro radicals which may be the same or different;

Z represents a hydrogen atom, or a halogen atom, or a $(C_1—C_8)$alkyl, a $(C_1—C_8)$alkoxy, a hydroxy, or a nitro group;

Y represents a hydrogen atoms or one or two $(C_1—C_8)$alkyl, $(C_1—C_8)$alkoxy or hydroxy radicals which may be the same or different; or a pharmaceutically acceptable acid addition salt thereof;

the process comprising reacting a compound of general formula III

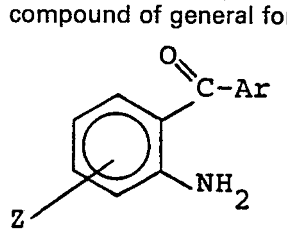

wherein Ar and Z are as defined above with a compound of general formula IV

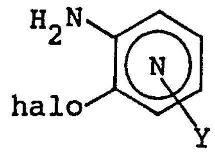

wherein Y is as defined above and halo represents a halogen atom, and optionally forming a salt of the compound.

2. A process as claimed in Claim 1 for preparing
[2-[(3-amino-2-pyridinyl)amino]phenyl]phenylmethanone.
[2-[(3-amino-2-pyridinyl)amino]-4-chlorophenyl]phenylmethanone,
[2-[(3-amino-2-pyridinyl)amino]phenyl](4-methylphenyl)-methanone,
[2-[(3-amino-2-pyridinyl)amino]-5-chlorophenyl](2-chlorophenyl)methanone,
[2-[(3-amino-2-pyridinyl)amino]-3-chlorophenyl]phenylmethanone,
[2-[(3-amino-2-pyridinyl)amino]-4-fluorophenyl]phenylmethanone,
[2-[(3-amino-2-pyridinyl)amino]phenyl](3-chlorophenyl)methanone,
[2-[(3-amino-2-pyridinyl)amino]phenyl](4-fluorophenyl)methanone,
[2-[(3-amino-2-pyridinyl)amino]phenyl]-(3-trifluoromethylphenyl)methanone,
[2-[(3-amino-2-pyridinyl)amino]phenyl]-(3-fluorophenyl)methanone,
[2-[(3-amino-2-pyridinyl)amino]phenyl]-(2-fluorophenyl)methanone,
[2-[(3-amino-2-pyridinyl)amino]phenyl-(2-chlorophenyl)methanone, or
[2-[(3-amino-2-pyridinyl)amino]phenyl]-(2-bromophenyl)methanone.

3. A process for the preparation of a pyrido[1,4]benzodiazepine which is a 6-aryl-11H-pyrido[2,3-b][1,4]-benzodiazepine or a 5,6 dihydro derivative thereof, and which has the formula $I_w$, namely

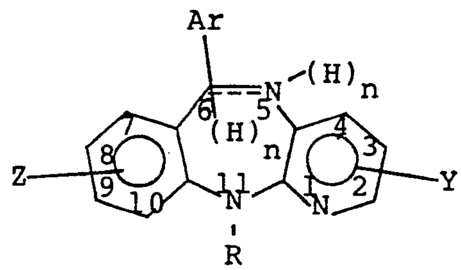

or which is a 6-aryl-11H-pyrido[3,4-b][1,4]benzodiazepine or the 5,6 dihydro derivative thereof, and which has the formula $I_x$, namely

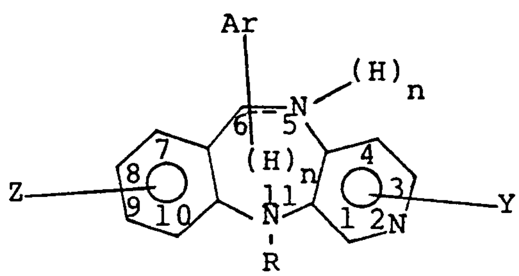

or which is a 10-aryl-5H-pyrido[4,3-b][1,4]benzodiazepine or a 10,11 dihydro derivative thereof, and which has the formula $I_y$, namely

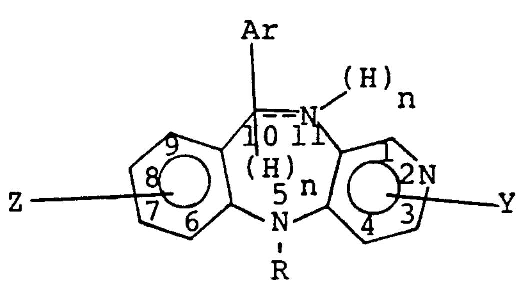

or which is a 10-aryl-5H-pyrido[3,2-b][1,4]benzodiazepine or a 10,11 dihydro derivative thereof, and which has the formula $I_z$, namely

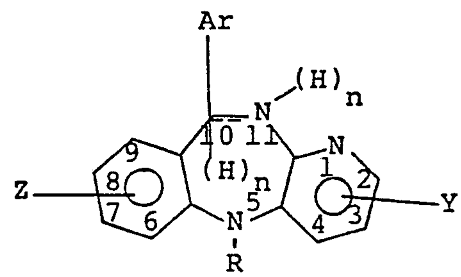

wherein;

R represents a hydrogen atom, ($C_1$—$C_8$)alkyl, an -alk$^1$-NR$^1$R$^2$ or an -alk$^1$-N=CH—OC$_2$H$_5$ group;

R$^1$ and R$^2$ each represent a hydrogen atom, or a ($C_1$—$C_8$)alkyl, or a —C(O)O—($C_1$—$C_8$)alkyl group, or R$^1$ and R$^2$ taken together with the adjacent nitrogen atom may form a 1-phthalimido, 1-piperidinyl, 1-pyrrolidinyl, 4-morpholino, 1-piperazino, or 4-substituted piperazin-1-yl residue;

Ar represents a 2, 3 or 4-pyridinyl, a 2 or 3-thienyl, or a phenyl or a substituted phenyl group, the said substituted phenyl group being substituted with one to three halo, ($C_1$—$C_8$)alkyl, ($C_1$—$C_8$)alkoxy, trifluoromethyl or nitro radicals which may be the same or different;

Alk$^1$ represents a straight or branched hydrocarbon chain containing 1—8 carbon atoms;

Z represents a hydrogen atom, or a halogen atom or a ($C_1$—$C_8$)alkyl, a ($C_1$—$C_8$)alkoxy, a hydroxy or a nitro group;

Y represents a hydrogen atom or 1 or 2 ($C_1$—$C_8$)alkyl, ($C_1$—$C_8$)alkoxy or hydroxy radicals which may be the same or different;

n is 0 or 1 and when n is zero the dotted line is a double bond; and the acid addition salts thereof, but excluding the compound which has the formula $I_w$, wherein Z and Y both represent a hydrogen atom, R represents a $(CH_2)_3$—$N(CH_3)_2$ group and Ar represents a $C_6H_5$— group.

the process comprising reacting a compound of the general formula III

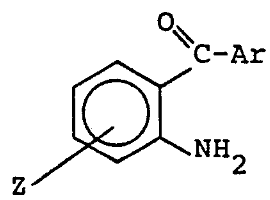

wherein Ar and Z are as defined for general formulae $I_w$ to $I_z$,

with a compound of gehneral formula IV

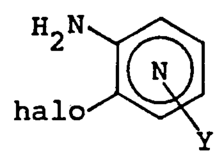

wherin Y is as defined for general formulae $I_w$ to $I_z$ and halo represents halogen atom,

to form a compound of general formulae $I_w$ to $I_z$ where R represents a hydrogen atom and n is zero,

and optionally thereafter converting the compound of general formulae $I_w$ to $I_z$ so formed to another compound of general formulae $I_w$ to $I_z$,

and optionally forming a pharmacuetically acceptable salt thereof.

4. A process as claimed in Claim 3 in which R represents a ($C_1$—$C_8$)alkyl or an -alk$^1$-NR$^1$R$^2$ group;

R$^1$ and R$^2$ each represent a hydrogen atom, or a ($C_1$—$C_8$)alkyl group, or R$^1$ and R$^2$ taken together with the adjacent nitrogen atom may form a 1-piperidinyl, 1-pyrrolidinyl, 4-morpholinyl, or 4-loweralkyl-1-piperazinyl residue,

Ar represents a 2, 3 or 4-pyridinyl, a 2 or 3-thienyl, or a phenyl or substituted phenyl group, the said substituted phenyl group being substituted with one to three halo ($C_1$—$C_8$)alkyl, ($C_1$—$C_8$)alkoxy, trifluoromethyl or nitro radicals which may be the same or different;

alk$^1$ represents a straight or branched hydrocarbon chain containing 1—8 carbon atoms;

Z represents a hydrogen atom, or a halogen atom, or a ($C_1$—$C_8$)alkyl, a ($C_1$—$C_8$)alkoxy, a hydroxy, or a nitro group;

Y represents a hydrogen atom, or one or two ($C_1$—$C_8$)alkyl, ($C_1$—$C_8$)alkoxy, or hydroxy radicals which may be the same or different; and

n is 0 or 1 and when n is zero the dotted line is a double bond.

5. A process as claimed in Claim 3 for preparing a 6-aryl-11H-pyrido((2,3-b][1,4]benzodiazepine or a 5,6 dihydro derivative thereof which has the general formula $I_w$.

6. A process as claimed in Claim 3 for preparing
6-phenyl-11H-pyrido[2,3-b][1,4]benzodiazepine;
8-chloro-6-phenyl-11H-pyrido[2,3-b][1,4]benzodiazepine,
9-chloro-6-phenyl-11H-pyrido[2,3-b][1,4]benzodiazepine;
6-(4-chlorophenyl)-11H-pyrido[2,3-b][1,4]benzodiazepine;
6-(4-methylpheny)-11H-pyrido[2,3-b][1,4]benzodiazepine;
6-(4-methoxyphenyl)-11H-pyrido[2,3-b][1,4]benzodiazepine;
8-chloro-N,N-dimethyl-6-phenyl-11H-pyrido[2,3-b][1,4]benzodiazepine-11-propanamine;
8-chloro-N,N-dimethyl-6-phenyl-11H-pyrido[2,3-b][1,4]benzodiazepine-11-propanamine oxalate [1:1];
N,N-dimethyl-6-phenyl-11H-pyrido[2,3-b][1,4]benzodiazepine-11-ethanamine;
N,N-dimethyl-6-phenyl-11H-pyrido[2,3-b][1,4]benzodiazepine-11-ethanamine fumarate [1:1];
11-[3-(4-morpholinyl)propyl]-6-phenyl-11H-pyrido[2,3-b][1,4]benzodiazepine;
11-[3-(4-morpholinyl)propyl]-6-phenyl-11H-pyrido[2,3-b][1,4]benzodiazepine fumarate [1:1];
N,N-diethyl-6-phenyl-11H-pyrido[2,3-b][1,4]benzodiazepine-11-propanamine;
N,N-diethyl-6-phenyl-11H-pyrido[2,3-b][1,4]benzodiazepine-11-propanamine oxalate [1:1];
9-chloro-N,N-dimethyl-6-phenyl-11H-pyrido[2,3-b][1,4]benzodiazepine-11-propanamine;
9-chloro-N,N-dimethyl-6-phenyl-11H-pyrido[2,3-b][1,4]benzodiazepine-11-propanamine fumarate [1:1];
6-phenyl-11-[3-(1-piperidinyl)propyl]-11H-pyrido[2,3-b][1,4]benzodiazepine;
6-phenyl-11-[3-(1-piperidinyl)propyl]-11H-pyrido[2,3-b][1,4]benzodiazepine fumarate [1:1];
6-(4-chlorophenyl)-N,N-dimethyl-11H-pyrido[2,3b][1,4]benzodiazepine-11-propanamine;
6-(4-chlorophenyl)-N,N-dimethyl-11H-pyrido[2,3-b][1,4]benzodiazepine-11-propanamine fumarate [1:1];
8-chloro-N,N-dimethyl-6-phenyl-11H-pyrido[2,3b][1,4]benzodiazepine-11-ethanamine;
8-chloro-N,N-dimethyl-6-phenyl-11H-pyrido[2,3-b][1,4]benzodiazepine-11-ethanamine oxalate [1:1];
8-chloro-11-methyl-6-phenyl-11H-pyrido[2,3-b][1,4]benzodiazepine;
N,N-dimethyl-6-(4-methylphenyl)-11H-pyrido[2,3-b][1,4]benzodiazepine-11-propanamine;
N,N-dimethyl-6-(4-methylphenyl)-11H-pyrido[2,3-b[1,4]benzodiazepine-11-propanamine fumarate [1:1];
6-(4-methoxyphenyl)-N,N-dimethyl-11H-pyrido[2,3-b][1,4]benzodiazepine-11-propanamine;
6-(4-methoxyphenyl)-N,N-dimethyl-11H-pyrido[2,3-b][1,4]benzodiazepine-11-propanamine;
6-(4-methoxyphenyl)-N,N-dimethyl-11H-pyrido[2,3-b][1,4]benzodiazepine-11-propanamine fumarate [1:1];
6-(3-chlorophenyl)-11H-pyrido[2,3-b][1,4]benzodiazepine;
6-(3-chlorophenyl)-N,N-dimethyl-11H-pyrido[2,3-b][1,4]benzodiazepine-11-propanamine;
6-(4-fluorophenyl)-11H-pyrido[2,3-b][1,4]benzodiazepine;
6-(4-fluorophenyl)-N,N-dimethyl-11H-pyrido[2,3-b][1,4]benzodiazepine-11-propanamine;
11-[3-(1,3-dihydro-1,3-dioxo-2H-isoindol-2-yl)propyl]6-phenyl-11H-pyrido[2,3-b][1,4]benzodiazepine;
6-phenyl-11H-pyrido[2,3-b][1,4]benzodiazepine-11-propanamine;
6-phenyl-11H-pyrido[2,3-b][1,4]benzodiazepine-11-propanamine dihydrochloride hemihydrate;
N-[3-[6-phenyl-11H-pyrido[2,3-b][1,4]benzodiazepine-11-yl]propyl]methanimidic acid ethyl ester;
N-methyl-6-phenyl-11H-pyrido[2,3][1,4]benzodiazepine-11-propanamine;
N-methyl-6-phenyl-11H-pyrido[2,3-b][1,4]benzodiazepine-11-propanamine dihydrochloride;
N-[3-[6-phenyl-11H-pyrido[2,3-b][1,4]benzodiazepine-11-yl]propyl]carbamic acid ethyl ester;
5,6-dihydro-N,N-dimethyl-6-phenyl-11H-pyrido[2,3-b][1,4]benzodiazepine-11-propanamine;
5,6-dihydro-N,N-dimethyl-6-phenyl-11H-pyrido[2,3-b][1,4]benzodiazepine-11-propanamine, dihydrochloride hemihydrate;
8-chloro-6-(2-chlorophenyl)-5,6-dihydro-11H-pyrido[2,3-b][1,4]benzodiazepine;
5,6-dihydro-N-methyl-6-phenyl-11H-pyrido[2,3-b][1,4]benzodiazepine-11-propanamine;
6-(2-fluorophenyl)-N,N-dimethyl-11H-pyrido[2,3-b][1,4]benzodiazepine-11-propanamine;
6-(2-chlorophenyl)-N,N-dimethyl-11H-pyrido[2,3-b][1,4]benzodiazepine-11-propanamine;
6-(2-bromophenyl)-N,N-dimethyl-11H-pyrido[2,3-b][1,4]benzodiazepine-11-propanamine;
8-chloro-11-methyl-6-(2-nitrophenyl)-11H-pyrido[2,3-b][1,4]benzodiazepine;
8-chloro-6-(2-chlorophenyl)-11-methyl-11H-pyrido[2,3-b][1,4]benzodiazepine;
6-(2-bromophenyl)-8-chloro-11-methyl-11H-pyrido[2,3-b][1,4]benzodiazepine;
6-(3-chlorophenyl)-N,N-dimethyl-11H-pyrido[2,3-b[1,4]benzodiazepine-11-propanamine fumarate [1:1]; or
6-(4-fluorophenyl)-N,N-dimethyl-11H-pyrido[2,3-b][1,4]benzodiazepine-11-propanamine hydrochloride hemihydrate;

7. A process as claimed in Claim 3 for preparing a 6-aryl-11H-pyrido[3,4-b][1,4]benzodiazepine or a 5,6 dihydro derivative thereof of general formula $I_x$.

8. A process as claimed in Claim 3 for preparing a 10-aryl-5H-pyrido[4,3-b][1,4]benzodiazepine or a 10,11 dihydro derivative thereof of general formula $I_y$.

9. A process as claimed in Claim 3 for preparing a 10-aryl-5H-pyrido[3,2-b][1,4]benzodiazepine or a 10,11 dihydro derivative thereof of general formula $I_z$.

10. A process for the preparation of a pharmaceutical composition, the process comprising admixing a compound having the formula

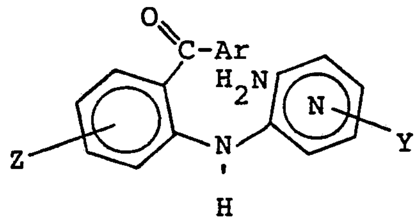

wherein;

Ar represents a 2, 3 or 4-pyridinyl, a 2 or 3-thienyl, a phenyl or a substituted phenyl group, the said substituted phenyl group being substituted with one to three halo, $(C_1—C_8)$alkyl, $(C_1—C_8)$alkoxy, trifluoromethyl or nitro radicals which may be the same or different;

Z represents a hydrogen atom, or a halogen atom, or a $(C_1—C_8)$alkyl, a $(C_1—C_8)$alkoxy, a hydroxy, or a nitro group;

Y represents a hydrogen atom, or one or two $(C_1—C_8)$alkyl, $(C_1—C_8)$alkoxy or hydroxy radicals which may be the same or different; or a pharmaceutically acceptable acid addition salt thereof;

with a pharmaceutically acceptable carrier therefor.

11. A process as claimed in Claim 10 wherein the compound is a pharmaceutically acceptable salt of N-methyl-6-phenyl-11H-pyrido[2,3-b][1,4]benzodiazepine-11-propanamine;

a pharmaceutically acceptable salt of 6-phenyl-11H-pyrido[2,3-b][1,4]benzodiazepine-11-propan amine;

8-chloro-N,N-dimethyl-6-phenyl-11H-pyrido[2,3-b][1,4]benzodiazepine-11-propanamine;

6-(3-chlorophenyl)-N,N-dimethyl-11H-pyrido[2,3-b][1,4]benzodiazepine-11-propanamine fumarate [1:1];

a pharmaceutically acceptable acid addition salt of 6-(4-fluorophenyl)-N,N-dimethyl-11H-pyrido[2,3-b][1,4]benzodiazepine-11-propanamine; or

a pharmaceutically acceptable acid addition salt of 6-(2-chlorophenyl)-N,N=dimethyl-11H-pyrido[2,3-b][1,4]benzodiazepine-11-propanamine.

**Patentansprüche für die Vertragsstaaten: BE FR DE IT LU NL SE CH LI GB**

1. Verbindung mit der Formel

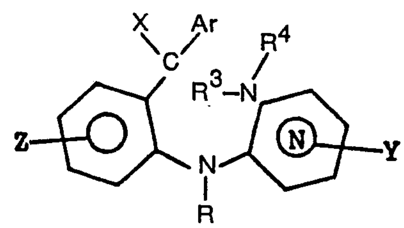

worin

R ein Wasserstoffatom, $(C_1—C_8)$Alkyl, $—Alk^1—NR^1R^2$ oder eine $-Alk^1-N=CH—OC_2H_5$-Gruppe ist;

$R^1$ und $R^2$ beide ein Wasserstoffatom, oder ein $(C_1—C_8)$Alkyl oder eine $—C(O)O—(C_1—C_8)$Alkylgruppe sind, oder $R^1$ und $R^2$ gemeinsam mit dem benachbarten Stickstoffatom ein 1-Phthalimido, 1-Piperidinyl, 1-Pyrrolidinyl, 4-Morpholino, 1-Piperazino oder einen 4-substituierten, heterozyklischen Piperazin-1-yl-Rest bilden;

Ar ein 2-, 3- oder 4-Pyridinyl, 2- oder 3-Thienyl, oder Phenyl oder eine substituierte Phenylgruppe ist, wobei die substituierte Phenylgruppe durch 1 bis 3 Halo, $(C_1—C_8)$Alkyl, $(C_1—C_8)$Alkoxy, Trifluoromethyl oder Nitroradikale substituiert ist, die gleich oder verschieden sein können;

$Alk^1$ eine gerade oder verzweigte Kohlenwasserstoffkette mit 1—8 Kohlenstoffatomen ist;

Z ein Wasserstoffatom der ein Halogenatom, oder ein $(C_1—C_8)$Alkyl, $(C_1—C_8)$Alkoxy, Hydroxy oder eine Nitrogruppe ist;

Y ein Wasserstoffatom oder 1 oder 2 $(C_1—C_8)$Alkyle, $(C_1—C_8)$Alkoxy oder Hydroxyradikale sind, die gleich oder verschieden sein können;

und $R^3$ und $R^4$ beide ein Wasserstoffatom sind,

X=O oder $Q^1$ ist, wobei $Q^1$ eine —C=O-Gruppe oder ein Carbonyläquivalent wie ketal, Thioketal oder Zwillingsdihalogenid ist; und

Säureadditionssalze davon.

2. Verbindung mit der Formel

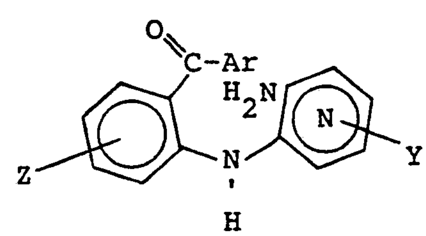

worin

Ar ein 2-, 3- oder 4-Pyridinyl, 2- oder 3-Thienyl, oder Phenyl oder eine substituierte Phenylgruppe ist, wobei die substituierte Phenylgruppe durch 1 bis 3 Halo, ($C_1$—$C_8$)Alkyl, ($C_1$—$C_8$)Alkoxy, Trifluoromethyl oder Nitroradikale substituiert ist, die gleich oder verschieden sein können;

Z ein Wasserstoffatom der ein Halogenatom, oder ein ($C_1$—$C_8$)Alkyl, ($C_1$—$C_8$)Alkoxy, Hydroxy oder eine Nitrogruppe ist;

Y ein Wasserstoffatom oder 1 oder 2 ($C_1$—$C_8$)Alkyle, ($C_1$—$C_8$)Alkoxy oder Hydroxyradikale sind, die gleich oder verschieden sein können; und pharmazeutisch verträgliche Säureadditionalssalze davon.

3. Verbindung nach Anspruch 1, die [2-[(3-Amino-2-pyridinyl)amino]phenyl]phenylmethanon ist.

4. Verbindung nach Anspruch 1, die [2-[(3-Amino-2-pyridinyl)amino]-4-chlorhenyl]phenylmethanon ist.

5. Verbindung nach Anspruch 1, die [2-[(3-Amino-2-pyridinyl)amino]phenyl](4-methylphenyl)-methanon ist.

6. Verbindung nach Anspruch 1, die [2-[(3-Amino-2-pyridinyl)amino]-5-chlorphenyl](2-chlorphenyl)-methanon ist.

7. Verbindung nach Anspruch 1, die [2-[(3-Amino-2-pyridinyl)amino]-3-chlorphenyl]penylmethanon ist.

8. Verbindung nach Anspruch 1, die [2-[(3-Amino-2-pyridinyl)amino]-4-fluorophenyl]phenylmethanon ist.

9. Verbindung nach Anspruch 1, die [2-[(3-Amino-2-pyridinyl)amino]phenyl)[3-chlorphenyl)methanon ist.

10. Verbindung nach Anspruch 1, die [2-[(3-Amino-2-pyridinyl)amino]phenyl(4-fluorophenyl)-methanon ist.

11. Verbindung nach Anspruch 1, die [2-[(3-Amino-2-pyridinyl)amino]phenyl]-(3-trifluormethylphenyl)-methanon ist.

12. Verbindung nach Anspruch 1, die [2-[(3-Amino-2-pyridinyl)amino]phenyl]-(3-fluorphenyl)-methanon ist.

13. Verbindung nach Anspruch 1, die [2-[(3-Amino-2-pyridinyl)amino]phenyl]-(2-fluorophenyl)-methanon ist.

14. Verbindung nach Anspruch 1, die [2-[(3-Amino-2-pyridinyl)amino]phenyl]-(2-chlorphenyl)-methanon ist.

15. Verbindung nach Anspruch 1, die [2-[(3-Amino-2-pyridinyl)amino]phenyl]-(2-bromophenyl)-methanon ist.

16. Verbindung nach Ansprüche 1 bis 15 zur Verwendung bei der Behandlung von Depressionen.

17. Pharmazeutische Zusammensetzung zur Behandlung von Depressionen in einheitlicher Dosierungsform, bestehend aus (a) einer wirksamen Menge einer Verbindung nach einem der Ansprüche 2 bis 15 und (b) einem pharmazeutischen Träger dafür.

18. Pyrido[1,4]benzodiazepine, die 6-Aryl-11H-pyrido[2,3-b][1,4]benzodiazepine oder 5,6-Dihydroderivative davon sind und folgende Formel $I_w$ haben,

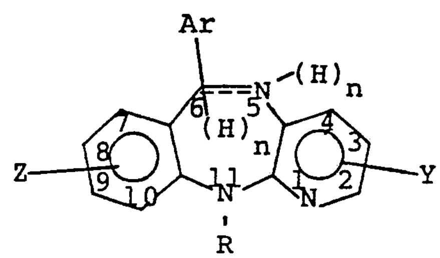

die 6-Aryl-11H-pyrido[3,4-b][1,4]benzodiazepine oder 5,6-Dihydroderivative davon sind und folgende Formel $I_x$ haben,

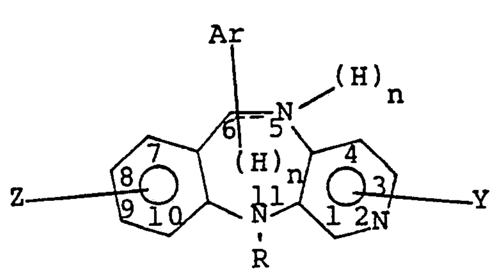

oder die 10-Aryl-5H-pyrido[4,3-b][1,4]benzodiazepine oder 10,11-Dihydroderivate davon sind und folgende Formel $I_y$ haben,

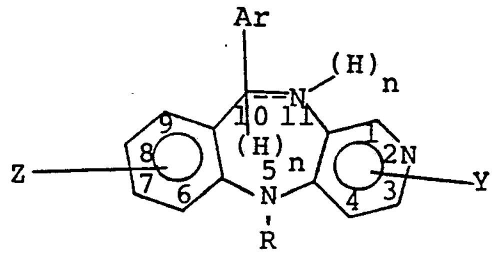

oder die 10-Aryl-5H-pyrido[3,2-b][1,4]benzodiazepine oder 10,11-Dihydroderivative davon sind und folgende Formel $I_z$ haben,

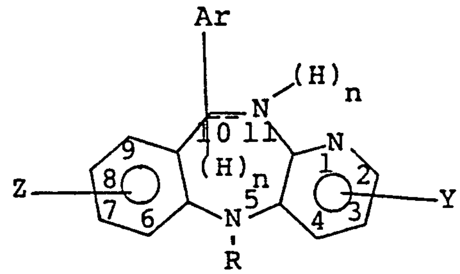

worin

R ein Wasserstoffatom, $(C_1—C_8)$Alkyl, —$Alk^1$—$NR^1R^2$ oder eine -$Alk^1$-N=CH—$OC_2H_5$-Gruppe ist;

$R^1$ und $R^2$ beide ein Wasserstoffatom, oder ein $(C_1—C_8)$Alkyl oder eine —C(O)O—$(C_1—C_8)$Alkylgruppe sind, oder $R^1$ und $R^2$ gemeinsam mit dem benachbarten Stickstoffatom ein 1-Phthalimido, 1-Piperidinyl, 1-Pyrrolidinyl, 4-Morpholino, 1-Piperazino oder einen 4-substituierten, Piperazin-1-yl-Rest bilden;

Ar ein 2-, 3- oder 4-Pyridinyl, 2- oder 3-Thienyl, oder ein Phenyl oder eine substituierte Phenylgruppe ist, wobei die substituierte Phenylgruppe durch 1 bis 3 Halo, $(C_1—C_8)$Alkyl, $(C_1—C_8)$Alkoxy, Trifluoromethyl oder Nitroradikale substituiert ist, die gleich oder verschieden sein können;

$Alk^1$ eine gerade oder verzweigte Kohlenwasserstoffkette mit 1—8 Kohlenstoffatomen ist;

Z ein Wasserstoffatom der ein Halogenatom, oder ein $(C_1—C_8)$Alkyl, $(C_1—C_8)$Alkoxy, Hydroxy oder eine Nitrogruppe ist;

Y ein Wasserstoffatom oder 1 oder 2 $(C_1—C_8)$Alkyle, $(C_1—C_8)$Alkoxy oder Hydroxyradikale sind, die gleich oder verschieden sein können;

n 0 oder 1 ist, und wenn n Null ist, die punktierte Linie eine Doppelbildung ist; und Säureadditionssalze davon, jedoch ausgenommen die Verbindung mit der Formel $I_w$, worin Z und Y beide ein Wasserstoffatom sind, R eine —$(CH_2)_3$—$N(CH_3)_2$-Gruppe ist und Ar eine $C_6H_5$-Gruppe ist.

19. Verbindung nach Anspruch 18, worin R ein $(C_1—C_8)$Alkyl oder eine -$Alk^1$—$NR^1R^2$-Gruppe ist;

$R^1$ und $R^2$ beide ein Wasserstoffatom oder eine $(C_1—C_8)$Alkylgruppe zeigen, oder $R^1$ und $R^2$ gemeinsam mit dem benachbarten Stickstoffatom ein 1-Piperidinyl, 1-Pyrrolidinyl, 4-Morpholinyl oder einen 4-Niedrigalkyl-1-piperzinyl-Rest bilden können;

Ar ein 2-, 3- oder 4-Pyridinyl, ein 2- oder 3-Thienyl, oder ein Phenyl oder eine substituierte Phenylgruppe ist, wobei die substituierte Phenylgruppe durch 1 bis 3 Halo, $(C_1—C_8)$Alkyl, $(C_1—C_8)$Alkoxy, Trifluoromethyl oder Nitroradikale substituiert ist, die gleich oder verschieden sein können;

$Alk^1$ eine gerade oder verzweigte kohlenwasserstoffkette mit 1—8 Kohlenstoffatomen ist;

Z ein Wasserstoffatom der ein Halogenatom, oder ein $(C_1—C_8)$Alkyl, $(C_1—C_8)$Alkoxy, Hydroxy oder eine Nitrogruppe ist;

Y ein Wasserstoffatom oder 1 oder 2 $(C_1—C_8)$Alkyl, $(C_1—C_8)$Alkoxy oder Hydroxyradikale sind, die gleich oder verschieden sein können;

n 0 oder 1 ist, un wenn n Null ist, die punktierte Linie eine Doppelbindung ist; und pharmazeutisch verträgliche Säureadditionssalze davon.

20. Verbindung nach Anspruch 18, die ein 6-Aryl-11H-pyrido[2,3-b][1,4]benzodiazepin oder ein 5,6-Dihydroderivat davon ist und folgende Formel hat.

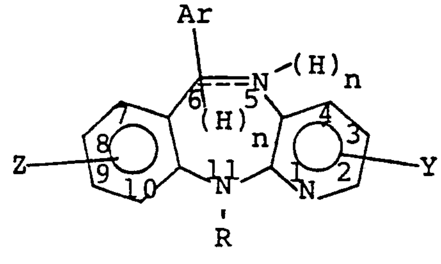

21. Verbindung nach Anspruch 18, die 6-Phenyl-11H-pyrido[2,3-b][1,4]benzodiazepin ist.
22. Verbindung nach Anspruch 18, die 8-Chlor-6-phenyl-11H-pyrido[2,3-b][1,4]benzodiazepin ist.
23. Verbindung nach Anspruch 18, die 9-Chlor-6-phenyl-11H-pyrido[2,3-b][1,4]benzodiazepin ist.
24. Verbindung nach Anspruch 18, die 6-(4-Chlorphenyl)-11H-pyrido[2,3-b][1,4]benzodiazepin ist.
25. Verbindung nach Anspruch 18, die 6-(4-Methylphenyl]-11H-pyrido[2,3-b][1,4]benzodiazepin ist.
26. Verbindung nach Anspruch 18, die 6-(4-Methoxyphenyl)-11H-pyrido[2,3-b][1,4]benzodiazepin ist.
27. Verbindung nach Anspruch 18, die 8-Chlor-N,N-dimethyl-6-phenyl-11H-pyrido[2,3-b][1,4]-benzodiazepin -11-propanamin ist.
28. Verbindung nach Anspruch 18, die 8-Chlor-N,N-dimethyl-6-phenyl-11H-pyrido[2,3-b][1,4]benzodiazepin-11-propanaminoxalat [1:1] ist.
29. Verbindung nach Anspruch 18, die N,N-Dimethyl-6-phenyl-11H-pyrido[2,3-b][1,4]benzodiazepin-11-ethanamin ist.
30. Verbindung nach Anspruch 18, die N,N-Dimethyl-6-phenyl-11H-pyrido[2,3-b][1,4]benzodiazepin-11-ethanaminfumarat [1:1] ist.
31. Verbindung nach Anspruch 18, die 11-[3-(4-Morpholinyl)propyl]-6-phenyl-11H-pyrido[2,3-b][1,4]-benzodiazepin ist.
32. Verbindung nach Anspruch 18, die 11-[3-(4-Morpholinyl)propyl]-6-phenyl-11H-pyrido[2,3-b][1,4]-benzodiazepinfumarat [1:1] ist.
33. Verbindung nach Anspruch 18, die N,N-Diethyl-6-phenyl-11H-pyrido[2,3-b][1,4]benzodiazepin-11-propanamin ist.
34. Verbindung nach Anspruch 18, die N,N-Diethyl-6-phenyl-11H-pyrido[2,3-b][1,4]benzodiazepin-11-propanaminoxalat [1:1] ist.
35. Verbindung nach Anspruch 18, die 9-Chlor-N,N-dimethyl-6-phenyl-11H-pyrido[2,3-b][1,4]benzodiazepin-11-propanamin ist.
36. Verbindung nach Anspruch 18, die 9-Chlor-N,N-dimethyl-6-phenyl-11H-pyrido[2,3-b][1,4]benzodiazepin-11-propanaminfumarat [1:1] ist.
37. Verbindung nach Anspruch 18, die 6-Phenyl-11-[3-(1-piperidinyl)propyl]-11H-pyrido[2,3-b][1,4]-benzodiazepin ist.
38. Verbindung nach Anspruch 18, die 6-Phenyl-11-[3-(1-piperidinyl)propyl]-11H-pyrido[2,3-b][1,4]-benzodiazepinfumarat [1:1] ist.
39. Verbindung nach Anspruch 18, die 6-(4-Chlorphenyl)-N,N-dimethyl-11H-pyrido[2,3-b][1,4]benzodiazepin-11-propanamin ist.
40. Verbindung nach Anspruch 18, die 6-(4-Chlorphenyl)-N,N-dimethyl-11H-pyrido[2,3-b][1,4]benzodiazepin-11-propanaminfurmarat [1:1] ist.
41. Verbindung nach Anspruch 18, die 8-Chlor-N,N-dimethyl-6-phenyl-11H-pyrido[2,3-b][1,4]benzodiazepin-11-ethanamin ist.
42. Verbindung nach Anspruch 18, die 8-Chlor-N,N-dimethyl-6-phenyl-11H-pyrido[2,3-b][1,4]benzodiazepin-11-ethanaminoxalat [1:1] ist.
43. Verbindung nach Anspruch 18, die 8-Chlor-11-methyl-6-phenyl-11H-pyrido[2,3-b][1,4]benzodiazepin ist.
44. Verbindung nach Anspruch 18, die N,N-Dimethyl-6-(4-methylphenyl)-11H-pyrido[2,3-b][1,4]benzodiazepin-11-propanamin ist.
45. Verbindung nach Anspruch 18, die N,N-Dimethyl-6-(4-methylphenyl)-11H-pyrido[2,3-b][1,4]benzodiazepin-11-propanaminfumarat [1:1] ist.
46. Verbindung nach Anspruch 18, die 6-(4-Methoxyphenyl)-N,N-dimethyl-11H-pyrido[2,3-b][1,4]-benzodiazepin-11-propanamin ist.
47. Verbindung nach Anspruch 18, die 6-(4-Methoxyphenyl)-N,N-dimethyl-11H-pyrido[2,3-b][1,4]-benzodiazepin-11-propanaminfumarat [1:1] ist.
48. Verbindung nach Anspruch 18, die 6-(3-Chlorphenyl)-11H-pyrido[2,3-b][1,4]benzodiazepin ist.
49. Verbindung nach Anspruch 18, die 6-(3-Chlorphenyl)-N,N-dimethyl-11H-pyrido[2,3-b][1,4]benzodiazepin-11-propanamin ist.
50. Verbindung nach Anspruch 18, die 6-(4-Fluorophenyl)-11H-pyrido[2,3-b][1,4]benzodiazepin ist.
51. Verbindung nach Anspruch 18, die 6-(4-Fluorophenyl)-N,N-dimethyl-11H-pyrido[2,3-b][1,4]benzodiazepin-11-propanamin ist.
52. Verbindung nach Anspruch 18, die 11-[3-(1,3-Dihydro-1,3-dioxo-2H-isoindol-2-yl)propyl]-6-phenyl-11H-pyrido[2,3-b][1,4]benzodiazepin ist.
53. Verbindung nach Anspruch 18, die 6-Phenyl-11H-pyrido[2,3-b][1,4]benzodiazepin-11-propanamin ist.
54. Verbindung nach Anspruch 18, die 6-Phenyl-11H-pyrido[2,3-b][1,4]benzodiazepin-11-propanamindihydrochloridhalbhydrat ist.
55. Verbindung nach Anspruch 18, die N-[3-[6-phenyl-11H-pyrido[2,3-b][1,4]benzodiazepin-11-yl]-propyl]methanimidsäureethylester ist.
56. Verbindung nach Anspruch 18, die N-Methyl-6-phenyl-11H-pyrido[2,3-b][1,4]benzodiazepin-11-propanamin ist.

57. Verbindung nach Anspruch 18, die N-Methyl-6-phenyl-11H-pyrido[2,3-b][1,4]benzodiazepin-11-propanamindihydrochlorid ist.

58. Verbindung nach Anspruch 18, die N-[3-[6-Phenyl-11H-pyrido[2,3-b][1,4]benzodiazepin-11-yl]-propyl]karbamidsäureethylester ist.

59. Verbindung nach Anspruch 18, die 5,6-Dihydro-N,N-dimethyl-6-phenyl-11H-pyrido[2,3-b][1,4]-benzodiazepin-11-propanamin ist.

60. Verbindung nach Anspruch 18, die 5,6-Dihydro-N,N-dimethyl-6-phenyl-11H-pyrido[2,3-b][1,4]-benzodiazepin-11-propanamindihydrochloridhalbhydrat ist.

61. Verbindung nach Anspruch 18, die 8-Chlor-6-(2-chlorphenyl)-5,6-dihydro-11H-pyrido[2,3-b][1,4]-benzodiazepin ist.

62. Verbindung nach Anspruch 18, die 5,6-Dihydro-N-methyl-6-phenyl-11H-pyrido[2,3-b][1,4]benzodiazepin-11-propanamin ist.

63. Verbindung nach Anspruch 18, die 6-(2-Fluorphenyl)-N,N-dimethyl-11H-pyrido[2,3-b][1,4]benzodiazepin-11-propanamin ist.

64. Verbindung nach Anspruch 18, die 6-(2-Chlorphenyl)-N,N-dimethyl-11H-pyrido[2,3-b][1,4]benzodiazepin-11-propanamin ist.

65. Verbindung nach Anspruch 18, die 6-(2-Bromophenyl)-N,N-dimethyl-11H-pyrido[2,3-b][1,4]benzodiazepin-11-propanamin ist.

66. Verbindung nach Anspruch 18, die 8-Chlor-11-methyl-6-(2-nitrophenyl)-11H-pyrido[2,3-b][1,4]-benzodiazepin ist.

67. Verbindung nach Anspruch 18, die 8-Chlor-6-(2-chlorphenyl)-11-methyl-11H-pyrido[2,3-b][1,4]-benzodiazepin ist.

68. Verbindung nach Anspruch 18, die 6-(2-Bromophenyl)-8-chlor-11-methyl-11H-pyrido[2,3-b][1,4]-benzodiazepin ist.

69. Verbindung nach Anspruch 18, die 6-(3-Chlorphenyl)-N,N-dimethyl-11H-pyrido[2,3-b][1,4]benzodiazepin-11-propanaminfumarat [1:1] ist.

70. Verbindung nach Anspruch 18, die 6-(4-Fluorophenyl)-N,N-dimethyl-11H-pyrido[2,3-b][1,4]benzodiazepin-11-propanaminhydrochloridhalbhydrat ist.

71. Verbindung nach Anspruch 18, die ein 6-Aryl-11H-pyrido[3,4-b][1,4]benzodiazepin oder ein 5,6-Diphydroderivat davon ist und folgende formel hat.

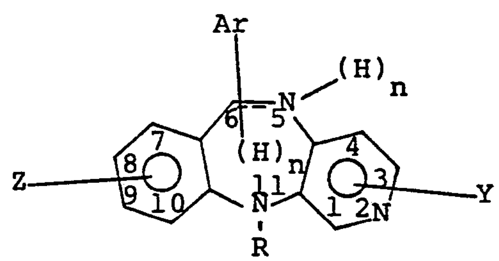

72. Verbindung nach Anspruch 18, die ein 10-Aryl-5H-pyrido[4,3-b][1,4]benzodiazepin oder ein 10,11-Dihydroderivat davon ist und folgende Formel hat.

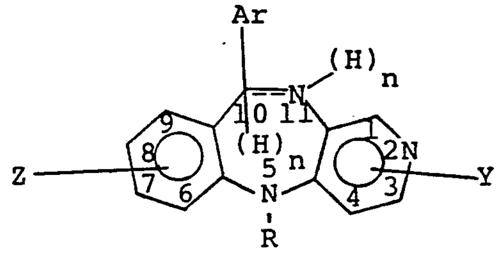

73. Verbindung nach Anspruch 18, die 10-Aryl-5H-pyrido[3,2-b][1,4]benzodiazepin oder ein 10,11-Dihydroderivat davon ist und folgende Formel, hat.

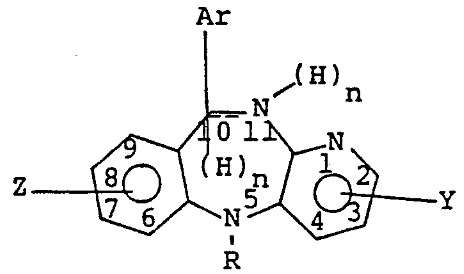

74. Verbindung nach einem Ansprüche 18 bis 73 zur Verwendung bei der Behandlung von Depressionen.

75. Pharmazeutische Zusammensetzung zur Behandlung von Depressionen in einheitlicher Dosierungsform, bestehend aus (a) einer wirksamen Menge einer Verbindung nach einem der Ansprüche 18 bis 74, und (b) einem pharmazeutischen Träger dafür.

76. Pharmazeutische Zusammensetzung nach Anspruch 75, worin die Verbindung ein pharmazeutisch veträgliches Salz von N-Methyl-6-phenyl-11H-pyrido[2,3-b][1,4]benzodiazepin-11-propanamin ist.

77. Pharmazeutische Zusammensetzung nach Anspruch 75, worin die Verbindung ein pharmazeutisch veträgliches Salz von 6-Phenyl-11H-pyrido[2,3-b][1,4]benzodiazepin-11-propanamin ist.

78. Pharmazeutische Zusammensetzung nach Anspruch 75, worin die Verbindung ein 8-Chlor-N,N-dimethyl-6-phenyl-11H-pyrido[2,3-b][1,4]benzodiazepin-11-propanamin ist.

79. Pharmazeutische Zusammensetzung nach Anspruch 75, worin die Verbindung ein 6-(3-Chlorphenyl)-N,N-dimethyl-11H-pyrido[2,3-b][1,4]benzodiazepin-11-propanaminfumarat [1:1] ist.

80. Pharmazeutische Zusammensetzung nach Anspruch 75, worin die Verbindung ein pharmazeutisch verträgliches Additionssalz von 6-(4-Fluorophenyl)-N,N-dimethyl-11H-pyrido[2,3-b][1,4]benzodiazepin-11-propanamin ist.

81. Pharmazeutische Zusammensetzung nach Anspruch 75, worin die Verbindung ein 6-(2-Chlorphenyl)-N,N-dimethyl-11H-pyrido[2,3-b][1,4]benzodiazepin-11-propanamin ist.

82. Verwendung einer Verbindung nach Anspruch 1 bei der Herstellung eines Antidepressivums.

83. Verwendung einer Verbindung nach einem der Ansprüche 1 bis 15 und 18 bis 73 bei der Herstellung eines Antideprssivums.

84. Verbindung nach Anspruch 1 zur Verwendung in der Medizin.

85. Verbindung nach einem der Ansprüche 1 bis 15 und 18 bis 73 zur Verwendung in der Medizin.

**Patentansprüche für di Vertragsstaaten: AT**

1. Verfahren zur Herstellung einer Verbindung mit der Formel

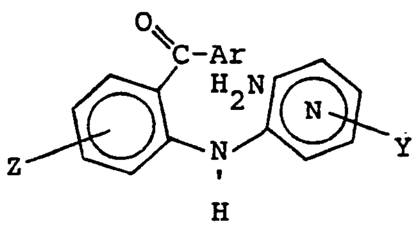

worin

Ar ein 2-, 3- oder 4-Pyridinyl, ein 2- oder 3-Thienyl, ein Phenyl oder eine substituierte Phenylgruppe ist, wobei die substituierte Phenylgruppe durch 1 bis 3 Halo, ($C_1$—$C_8$)Alkyl, ($C_1$—$C_8$)Alkoxy, Trifluoromethyl oder Nitroradikale substituiert ist, die gleich oder verschieden sein können;

Z ein Wasserstoffatom der ein Halogenatom, oder ein ($C_1$—$C_8$)Alkyl, ($C_1$—$C_8$)Alkoxy, Hydroxy oder eine Nitrogruppe ist;

Y ein Wasserstoffatom oder 1 oder 2 ($C_1$—$C_8$)Alkyle, ($C_1$—$C_8$)Alkoxy oder Hydroxyradikale sind, die gleich oder verschieden sein können; oder ein pharmazeutisch veträgliches Additionssalz davon;

das Verfahren aus einer Reaktion besteht zwischen einer Verbindung mit der allgemeinen Formel III,

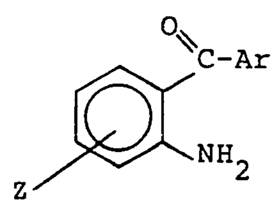

worin Ar und Z wie oben definiert sind, und einer Verbindung mit der allgemeinen Formel IV

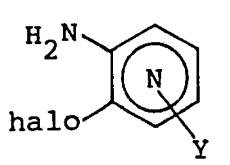

worin Y wie oben definiert ist, und Halo ein Halogenatom ist und wahlweise ein Salz der Verbindung bildet.

2. Verfahren nach Anspruch 1 zur Herstellung von
[2-[(3-Amino-2-pyridinyl)amino]phenyl]phenylmethanon,
[2-[(3-Amino-2-pyridinyl)amino]-4-chlorphenyl]phenylmethanon,
[2-[(3-Amino-2-pyridinyl)amino]phenyl](4-methylphenyl)methanon,
[2-[(3-Amino-2-pyridinyl)amino]-5-chlorphenyl](2-chlorphenyl)methanon,
[2-[(3-Amino-2-pyridinyl)amino]-3-chlorphenyl]phenylmethanon,
[2-[(3-Amino-2-pyridinyl)amino]-4-fluorophenyl]phenylmethanon,
[2-[(3-Amino-2-pyridinyl)amino]phenyl](3-chlorphenyl)methanon,
[2-[(3-Amino-2-pyridinyl)amino]phenyl](4-fluorophenyl)methanon,
[2-[(3-Amino-2-pyridinyl)amino]phenyl](3-trifluormethylphenyl)methanon,
[2-[(3-Amino-2-pyridinyl)amino]phenyl](3-fluorophenylmethanon,
[2-[(3-Amino-2-pyridinyl)amino]phenyl](2-fluorophenylmethanon,
[2-[(3-Amino-2-pyridinyl)amino]phenyl](2-chlorphenyl)methanon oder
[2-[(3-Amino-2-pyridinyl)amino]phenyl](2-bromophenyl)methanon.

3. Verfahren zur Herstellung eines Pyrido[1,4]benzodiazepins, das ein 6-Aryl-11H-pyrido[2,3-b][1,4]benzodiazepin oder ein 5,6-Dihydroderivat davon ist und folgende Formel $I_w$ hat,

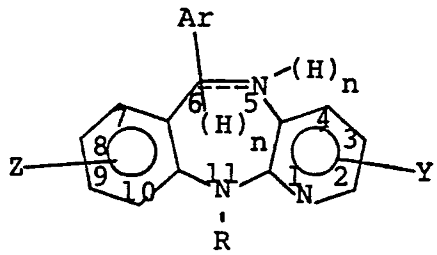

oder das ein 6-Aryl-11H-pyrido[2,3-b][1,4]benzodiazepin oder ein 5,6-Dihydroderivat davon ist und folgende Formel $I_x$ hat,

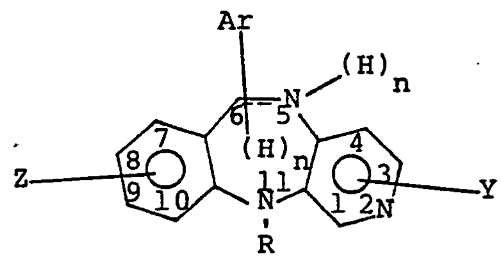

oder das ein 10-Aryl-5H-pyrido[4,3-b][1,4]benzodiazepin oder ein 10,11-Dihydroderivat davon ist und folgende Formel $I_y$ hat,

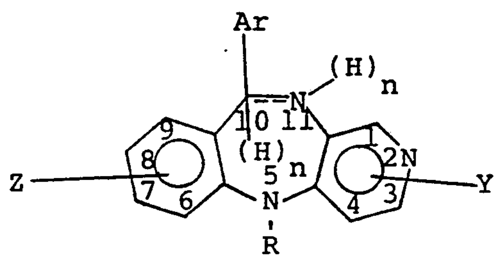

oder das ein 10-Aryl-5H-pyrido[3,2-b][1,4]benzodiazepin oder ein 10,11-Dihydroderivat davon ist und folgende Formel $I_z$ hat,

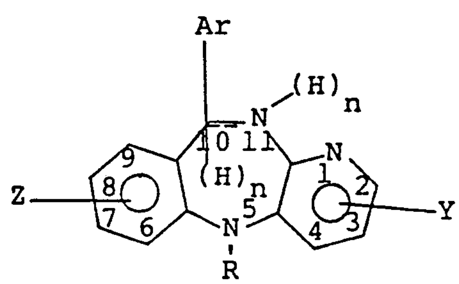

worin

R ein Wasserstoffatom, $(C_1—C_8)$Alkyl, $—Alk^1—NR^1R^2$ oder eine $-Alk^1-N=CH—OC_2H_5$-Gruppe ist;

$R^1$ und $R^2$ beide ein Wasserstoffatom, oder ein $(C_1-C_8)$Alkyl oder eine $-C(O)O-(C_1-C_8)$Alkylgruppe sind, oder $R^1$ und $R^2$ gemeinsam mit dem benachbarten Stickstoffatom ein 1-Phthalimido, 1-Piperidinyl, 1-Pyrrolidinyl, 4-Morpholino, 1-Piperazino oder einen 4-substituierten, Piperazin-1-yl-Rest bilden;

Ar ein 2-, 3- oder 4-Pyridinyl, 2- oder 3-Thienyl, oder Phenyl oder eine substituierte Phenylgruppe ist, wobei die substituierte Phenylgruppe durch 1 bis 3 Halo, $(C_1-C_8)$Alkyl, $(C_1-C_8)$Alkoxy, Trifluoromethyl oder Nitroradikale substituiert ist, die gleich oder verschieden sein können;

$Alk^1$ eine gerade oder verzweigte kohlenwasserstoffkette mit 1—8 Kohlenstoffatomen ist;

Z ein Wasserstoffatom der ein Halogenatom, oder ein $(C_1-C_8)$Alkyl, $(C_1-C_8)$Alkoxy, Hydroxy oder eine Nitrogruppe ist;

Y ein Wasserstoffatom oder 1 oder 2 $(C_1-C_8)$Alkyl, $(C_1-C_8)$Alkoxy oder Hydroxyradikale sind, die gleich oder verschieden sein können;

n 0 oder 1 ist, und wenn n Null ist, die punktierte Linie eine Doppelbildung ist; und Säureadditionssalze davon, jedoch ausgenommen die Verbindung mit der Formel $I_w$, worin Z und Y beide ein Wasserstoffatom sind, R eine $-(CH_2)_3-N(CH_3)_2$-Gruppe ist und Ar eine $C_6H_5$-Gruppe ist.

das Verfahren aus einer Reaktion besteht zwischen einer Verbindung mit der allgemeinen Formel III,

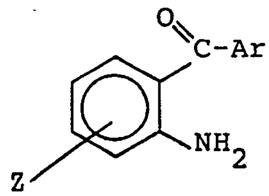

worin Ar und Z wie bei den allgemeinen Formeln $I_w$ bis $I_z$ definiert sind, und einer Verbindung mit der allgemeinen Formel IV

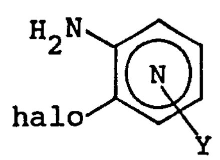

worin Y wie den allgemeinen Formeln $I_w$ bis $I_z$ definiert ist, und Halo ein Halogenatom ist,

zur Bildung einer Verbindung mit den allgemeinen Formeln $I_w$ bis $I_z$ worin R ein Wasserstoffatom ist und n Null ist,

und zur wahlweisen anschließenden Umwandlung der Verbindung mit den allgemeinen Formeln $I_w$ bis $I_z$ zur Bildung einer anderen Verbindung mit den allgemeinen Formeln $I_w$ bis $I_z$,

und zur wahlweisen Bildung eines pharmazeutisch veträglichen Salzes davon.

4. Verfahren nach Anspruch 3, in welchem R ein $(C_1-C_8)$Alkyl oder eine $-Alk^1-NR^1R^2$-Gruppe ist;

$R^1$ und $R^2$ beide ein Wasserstoffatom, oder ein $(C_1-C_8)$Alkylgruppe sind, oder $R^1$ und $R^2$ gemeinsam mit dem benachbarten Stickstoffatom ein 1-Piperidinyl, 1-Pyrrolidinyl, 4-Morpholinyl oder einen 4-Niedrigalkyl-1-piperazinyl-Rest bilden können;

Ar ein 2-, 3- oder 4-Pyridinyl, ein 2- oder 3-Thienyl, oder ein Phenyl oder eine substituierte Phenylgruppe ist, wobei die substituierte Phenylgruppe durch 1 bis 3 Halo, $(C_1-C_8)$Alkyl, $(C_1-C_8)$Alkoxy, Trifluoromethyl oder Nitroradikale substituiert ist, die gleich oder verschieden sein können;

$Alk^1$ eine gerade oder verzweigte Kohlenwasserstoffkette mit 1—8 Kohlenstoffatomen ist;

Z ein Wasserstoffatom der ein Halogenatom, oder ein $(C_1-C_8)$Alkyl, $(C_1-C_8)$Alkoxy, Hydroxy oder eine Nitrogruppe ist;

Y ein Wasserstoffatom oder 1 oder 2 $(C_1-C_8)$Alkyl, $(C_1-C_8)$Alkoxy oder Hydroxyradikale sind, die gleich oder verschieden sein können; und

n 0 oder 1 ist, und wenn n Null ist, die punktierte Linie eine Doppelbindung ist.

5. Verfahren nach Anspruch 3 zur Herstellung von 6-Aryl-11H-pyrido[2,3-b][1,4]benzodiazepin oder eines 5,6-Dihydroderivatives davon mit der allgemeinen Formel $I_w$.

6. Verfharen Anspruch 3 zur Herstellung von
6-Phenyl-11H-pyrido[2,3-b][1,4]benzodiazepin,
8-Chlor-6-phenyl-11H-pyrido[2,3-b][1,4]benzodiazepin,
9-Chlor-6-phenyl-11H-pyrido[2,3-b][1,4]benzodiazepin,
6-(4-Chlorphenyl)-11H-pyrido[2,3-b][1,4]benzodiazepin,
6-(4-Methylphenyl)-11H-pyrido[2,3-b][1,4]benzodiazepin,
6-(4-Methoxyphenyl)-11H-pyrido[2,3-b][1,4]benzodiazepin,
8-Chlor-N,N-dimethyl-6-phenyl-11H-pyrido[2,3-b][1,4]benzodiazepin-11-propanamin,
8-Chlor-N,N-dimethyl-6-phenyl-11H-pyrido[2,3-b][1,4]benzodiazepin-11-propanaminoxalat [1:1],
N,N-Dimethyl-6-phenyl-11H-pyrido[2,3-b][1,4]benzodiazepin-11-ethanamin,
N,N-Dimethyl-6-phenyl-11H-pyrido[2,3-b][1,4]benzodiazepin-11-ethanaminfumarat [1:1],
11-[3-(4-Morpholinyl)propyl]-6-phenyl-11H-pyrido[2,3-b][1,4]benzodiazepin,
11-[3-(4-Morpholinyl)propyl]-6-phenyl-11H-pyrido[2,3-b][1,4]benzodiazepinfumarat [1:1],

N,N-Diethyl-6-phenyl-11H-pyrido[2,3-b][1,4]benzodiazepin-11-propanamin,
N,N-Diethyl-6-phenyl-11H-pyrido[2,3-b][1,4]benzodiazepin-11-propanaminoxalat [1:1],
9-Chlor-N,N-dimethyl-6-phenyl-11H-pyrido[2,3-b][1,4]benzodiazepin-11-propanamin,
9-Chlor-N,N-dimethyl-6-phenyl-11H-pyrido[2,3-b][1,4]benzodiazepin-11-propanaminfumarat [1:1],
6-Phenyl-11-[3-(1-piperidinyl)propyl]-11H-pyrido[2,3-b][1,4]benzodiazepin,
6-Phenyl-11-[3-(1-piperindyl)propyl]-11H-pyrido[2,3-b][1,4]benzodiazepinfumarat [1:1],
6-(4-Chlorphenyl)-N,N-dimethyl-11H-pyrido[2,3-b][1,4]benzodiazepin-11-propanamin,
6-(4-Chlorphenyl)-N,N-dimethyl-11H-pyrido[2,3-b][1,4]benzodiazepin-11-propanaminfumarat [1:1],
8-Chlor-N,N-dimethyl-6-phenyl-11H-pyrido[2,3-b][1,4]benzodiazepin-11-ethanamin,
8-Chlor-N,N-dimethyl-6-phenyl-11H-pyrido[2,3-b][1,4]benzodiazepin-11-ethanaminoxalat [1:1],
8-Chlor-11-methyl-6-phenyl-11H-pyrido[2,3-b][1,4]benzodiazepin,
N,N-Dimethyl-6-(4-methylphenyl)-11H-pyrido[2,3-b][1,4]benzodiazepin-11-propanamin,
N,N-Dimethyl-6-(4-methylphenyl)-11H-pyrido[2,3-b][1,4]benzodiazepin-11-propanaminfurmarat [1:1],
6-(4-Methoxyphenyl)-N,N-dimethyl-11H-pyrido[2,3-b][1,4]benzodiazepin-11-propanamin,
6-(4-Methoxyphenyl)-N,N-dimethyl-11H-pyrido[2,3-b][1,4]benzodiazepin-11-propanamin,
[1,4]benzodiazepin-11-propanamin,
6-(4-Methoxyphenyl)-N,N-dimethyl-11H-pyrido[2,3-b][1,4]benzodiazepin-11-prpanaminfumarat [1:1],
6-(3-Chlorphenyl)-11H-pyrido[2,3-b][1,4]benzodiazepin,
6-(3-Chlorphenyl)-N,N-dimethyl-11H-pyrido[2,3-b][1,4]benzodiazepin-11-propanamin,
6-(4-Fluorophenyl)-11H-pyrido[2,3-b][1,4]benzodiazepin,
6-(4-Fluorophenyl)-N,N-dimethyl-11H-pyrido[2,3-b][1,4]benzodiazepin-11-propanamin,
11-[3-(1,3-Dihydro-1,3-dioxo-2H-isoindol-2-yl)propyl]-6-phenyl-11H-pyrido[2,3-b][1,4]benzodiazepin,
6-Phenyl-11H-pyrido[2,3-b][1,4]benzodiazepin-11-propanamin,
6-Phenyl-11H-pyrido[2,3-b][1,4]benzodiazepin-11-propanamindihydrochloridhalbhydrat,
N-[3-[6-phenyl-11H-pyrido[2,3-b][1,4]benzodiazepin-11-yl]propyl]methanimidsäureethylester,
N-Methyl-6-phenyl-11H-pyrido[2,3-b][1,4]benzodiazepin-11-propanamin,
N-Methyl-6-phenyl-11H-pyrido[2,3-b][1,4]benzodiazepin-11-propanamindihydrochlorid,
N-[3-[6-Phenyl-11H-pyrido[2,3-b][1,4]benzodiazepin-11-yl]propyl]karbamidsäureethylester,
5,6-Dihydro-N,N-dimethyl-6-phenyl-11H-pyrido[2,3-b][1,4]benzodiazepin-11-propanamin,
5,6-Dihydro-N,N-dimethyl-6-phenyl-11H-pyrido[2,3-b][1,4]benzodiazepin-11-propanamindihydrochloridhalbhydrat,
8-Chlor-6-(2-chlorphenyl)-5,6-dihydro-11H-pyrido[2,3-b][1,4]benzodiazepin,
5,6-Dihydro-N-methyl-6-phenyl-11H-pyrido[2,3-b][1,4]benzodiazepin-11-propanamin,
6-(2-Fluorophenyl)-N,N-dimethyl-11H-pyrido[2,3-b][1,4]benzodiazepin-11-propanamin,
6-(2-Chlorphenyl)-N,N-dimethyl-11H-pyrido[2,3-b][1,4]benzodiazepin-11-propanamin,
6-(2-Bromophenyl)-N,N-dimethyl-11H-pyrido[2,3-b][1,4]benzodiazepin-11-propanamin,
8-Chlor-11-methyl-6-(2-nitrophenyl)-11H-pyrido[2,3-b][1,4]benzodiazepin,
8-Chlor-6-(2-chlorphenyl)-11-methyl-11H-pyrido[2,3-b][1,4]benzodiazepin,
6-(2-Bromophenyl)-8-chlor-11-methyl-11H-pyrido[2,3-b][1,4]benzodiazepin,
6-(3-Chlorphenyl)-N,N-dimethyl-11H-pyrido[2,3-b][1,4]benzodiazepin-11-propanaminfumarat [1:1]
oder
6-(4-Fluorophenyl)-N,N-dimethyl-11H-pyrido[2,3-b][1,4]benzodiazepin-11-propanaminhydrochloridhalbhydrat.

7. Verfahren nach Anspruch 3 zur Herstellung von 6-Aryl-11H-pyrido[2,3-b][1,4]benzodiazepin oder eines 5,6-Dihydroderivates davon mit der allgemeinen Formel $I_x$.

8. Verfahren nach Anspruch 3 zur Herstellung von 10-Aryl-5H-pyrido[4,3-b][1,4]benzodiazepin oder eines 10,11-Dihydroderivatives davon mit der allgemeinen Formel $I_y$.

9. Verfharen nach Anspruch 3 zur Herstellung von 10-Aryl-5H-pyrido[3,2-b][1,4]benzodiazepin oder eines 10,11-Dihydroderivates davon mit der allgemeinen Formel $I_z$.

10. Verfahren zur Herstellung einer pharmazeutischen Zusammensetzung, wobei das Verfahren aus einem Zusammenmischen besteht einer Verbindung mit der Formel

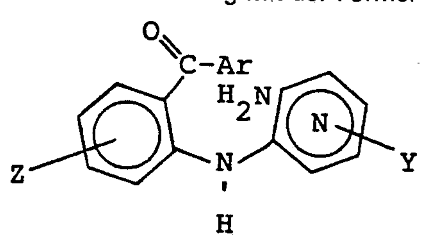

worin

Ar ein 2-, 3- oder 4-Pyridinyl, ein 2- oder 3-Thienyl, oder ein Phenyl oder eine substituierte Phenylgruppe ist, wobei die substituierte Phenylgruppe durch 1 bis 3 Halo, ($C_1$—$C_8$)Alkyl, ($C_1$—$C_8$)Alkoxy, Trifluoromethyl oder Nitroradikale substituiert ist, die gleich oder verschieden sein können;

Z ein Wasserstoffatom oder ein Halogenatom oder ($C_1$—$C_8$)Alkyl, ($C_1$—$C_8$)Alkoxy, Hydroxy oder eine Nitrogruppe ist;

Y ein Wasserstoffatom oder 1 oder 2 ($C_1$—$C_8$)Alkyle, ($C_1$—$C_8$)Alkoxy oder Hydroxyradikale sind, die gleich oder verschieden sein können; oder ein pharmazeutisch veträgliches Säureadditionssalzes davon; mit einem pharmazeutisch verträglichen Träger dafür.

11. Verfahren nach Anspurch 10, worin die Verbindung ein pharamazeutisch veträgliches Salz von N-Methyl-6-phenyl-11H-pyrido[2,3-b][1,4]benzodiazepin-11-propanamin;

ein pharmazeutisch verträgliches Slaz von 6-Phenyl-11H-pyrido[2,3-b][1,4]benzodiazepin-11-propanamin,

8-Chlor-N,N-dimethyl-6-phenyl-11H-pyrido[2,3-b][1,4]benzodiazepin-11-propanamin,

6-(3-Chlorphenyl)-N,N-dimethyl-11H-pyrido[2,3-b][1,4]benzodiazepin-11-propanaminfumarat [1:1];

ein pharmazeutisch verträgliches Säureadditionssalz von 6-(4-Fluorophenyl)-N,N-dimethyl-11H-pyrido[2,3-b][1,4]benzodiazepin-11-propanamin, oder

ein pharmazeutisch verträgliches Säureadditionssalz von 6-(2-Chlorphenyl)-N,N-dimethyl-11H-pyrido[2,3-b][1,4]benzodiazepin-11-propanamin ist.

**Revendications pour le Etats contractants: BE FR BE IT LU NL SE CH LI GB**

1. Composé de formule

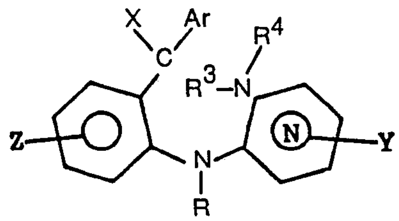

où

R représente un atome d'hydrogène, ou un radical ($C_1$—$C_8$)alcoyle, -$Alk^1$—$NR^1R^2$ ou -$Alk^1$—N=CH—$OC_2H_5$;

$R^1$ et $R^2$ représentent chacun un atome d'hydrogène, ou un radical ($C_1$—$C_8$)alcoyle ou —C(O)O—($C_1$—$C_8$)alcoyle ou bien $R^1$ et $R^2$ pris conjointement avec l'atome d'azote adjacent peuvent former un résidu hétérocyclique 1-phtalimido, 1-pipéridinyle, 1-pyrrolidinyle, 4-morpholino, 1-pipérazino ou pipérazine-1-yle 4-substitué;

Ar représente un radical 2-, 3- ou 4-pyridinyle, 2- ou 3-thiényle, phényle ou phényle substitué, le radical phényle substitué étant substitué par 1 à 3 radicaux halo, ($C_1$—$C_8$)alcoyle, ($C_1$—$C_8$)alcoxy, trifluorométhyle ou nitro qui peuvent être indentiques ou différents;

$Alk^1$ représente un chaîne hydrocarbonée droite ou ramifiée comptant 1 à 8 atomes de carbone;

Z représente un atome d'hydrogène, un atome d'halogène ou un radical ($C_1$—$C_8$)alcoyle, ($C_1$—$C_8$)alcoxy, hydroxyle ou nitro;

Y représente un atome d'hydrogène ou un ou deux radicaux ($C_1$—$C_8$)alcoyle, ($C_1$—$C_8$)alcoxy ou hydroxyle qui peuvent être identiques ou différentes; et

$R^3$ et $R^4$ représente chacun un atome d'hydrogène;

X représente =O ou Q1, où Q1 représente un radical —C=O ou un équivalent de carbonyle tel qu'un radical cétal, thiocétal ou gem-dihalogénure; et

ses sels d'addition d'acides.

2. Composé de formule

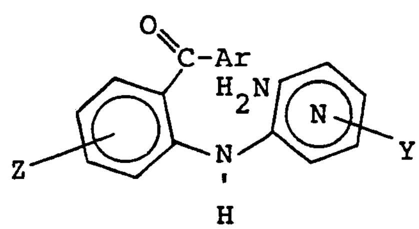

où Ar représente un radical 2-, 3- ou 4-pyridinyle, 2- ou 3-thiényle, phényle ou phényle substitué, le radical phényle substitué étant substitué par 1 à 3 radicaux halo, ($C_1$—$C_8$)alcoyle, ($C_1$—$C_8$)alcoxy, trifluorométhyle ou nitro qui peuvent être indentiques ou différents;

Z représente un atome d'hydrogène, un atome d'halogène ou un radical ($C_1$—$C_8$)alcoyle, ($C_1$—$C_8$)alcoxy, hydroxyle ou nitro;

Y représente un atome d'hydrogène ou un ou deux radicaux ($C_1$—$C_8$)alcoyle, ($C_1$—$C_8$)alcoxy ou hydroxyle qui peuvent être identiques ou différents;

et ses sels d'addition d'acides pharmaceutiquement acceptables.

3. Composé suivant la revendication 1 qui est la [2-[(3-amino-2-pyridinyl)amino]phényl]phénylméthanone.

4. Composé suivant la revendication 1 qui est la [2-[(3-amino-2-pyridinyl)amino]-4-chlorophényl]-phénylméthanone.

5. Composé suivant la revendication 1 qui est la [2-[(3-amino-2-pyridinyl)amino]phényl](4-méthyl-phényl)méthanone.

6. Composé suivant la revendication 1 qui est la [2-[(3-amino-2-pyridinyl)amino]-5-chlorophényl](2-chlorophényl)méthanone.

7. Composé suivant la revendication 1 qui est la [2-[(3-amino-2-pyridinyl)amino]-3-chlorophényl]-phényl)méthanone.

8. Composé suivant la revendication 1 qui est la [2-[(3-amino-2-pyridinyl)amino]-4-fluorophényl]-phénylméthanone.

9. Composé suivant la revendication 1 qui est la [2-[(3-amino-2-pyridinyl)amino]phényl](3-chloro-phényl)méthanone.

10. Composé suivant la revendication 1 qui est la [2-[(3-amino-2-pyridinyl)amino]phényl](4-fluoro-phényl)méthanone.

11. Composé suivant la revendication 1 qui est la [2-[(3-amino-2-pyridinyl)amino]phényl]-(3-trifluoro-méthylphényl)méthanone.

12. Composé suivant la revendication 1 qui est la [2-[(3-amino-2-pyridinyl)amino]phényl]-(3-fluoro-phényl)méthanone.

13. Composé suivant la revendication 1 qui est la [2-[(3-amino-2-pyridinyl)amino]phényl]-(2-fluoro-phényl)méthanone.

14. Composé suivant la revendication 1 qui est la [2-[(3-amino-2-pyridinyl)amino]phényl]-(2-chloro-phényl)méthanone.

15. Composé suivant la revendication 1 qui est la [2-[(3-amino-2-pyridinyl)amino]phényl]-(2-bromo-phényl)méthanone.

16. Composé suivant l'une quelconque des revendications 1 à 15 à utiliser pour le traitement de la dépression.

17. Composition pharmaceutique pour le traitement de la dépression sous forme dosée unitaire comprenant (a) une quantité efficace d'un composé suivant l'une quelconque des revendications 2 à 15 et (b) un excipient pharmaceutique.

18. Pyrido[1,4]benzodiazépines qui sont des 6-aryl-11H-pyrido[2,3-b][1,4]benzodiazépines ou les 5,6-dihydro-dérivés de celles-ci et qui sont de formule $I_w$,

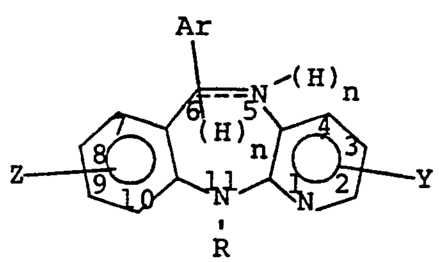

ou qui sont des 6-aryl-11H-pyrido[3,4-b][1,4]benzodiazépines ou les 5,6-dihydro-dérivés de celles-ci et qui sont de formule $I_x$,

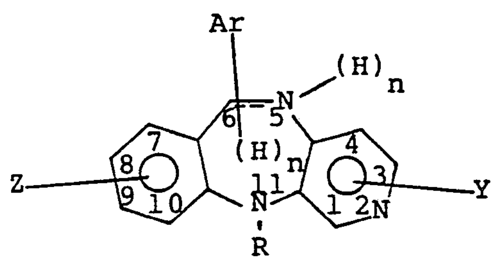

ou qui sont des 10-aryl-5H-pyrido[4,3-b][1,4]benzodiazépines ou les 10,11-dihydro-dérivés de celles-ci et qui sont de formule $I_y$,

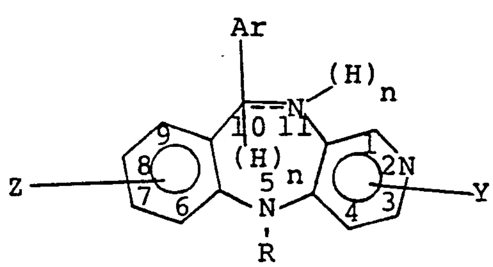

ou qui sont des 10-aryl-5H-pyrido[3,2-b][1,4]benzodiazépines ou les 10,11-dihydro-dérivés de celles-ci et qui sont de formule $I_z$,

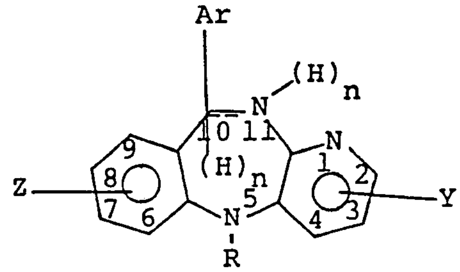

où

R représente un atome d'hydrogène, ou un radical ($C_1$—$C_8$)alcoyle, -$Alk^1$—$NR^1R^2$ ou -$Alk^1$—N=CH—$OC_2H_5$;

$R^1$ et $R^2$ représentent chacun un atome d'hydrogène, ou un radical ($C_1$—$C_8$)alcoyle ou —C(O)O—($C_1$—$C_8$)alcoyle ou bien $R^1$ et $R^2$ pris conjointement avec l'atome d'azote adjacent peuvent former un résidu hétérocyclique 1-phtalimido, 1-pipéridinyle, 1-pyrrolidinyle, 4-morpholino, 1-pipérazino ou pipérazine-1-yle 4-substitué;

Ar représente un radical 2-, 3- ou 4-pyridinyle, 2- ou 3-thiényle, phényle ou phényle substitué, le radical phényle substitué étant substitué par 1 à 3 radicaux halo, ($C_1$—$C_8$)alcoyle, ($C_1$—$C_8$)alcoxy, trifluorométhyle ou nitro qui peuvent être indentiques ou différents;

$Alk^1$ représente un chaîne hydrocarbonée droite ou ramifiée comptant 1 à 8 atomes de carbone;

Z représente un atome d'hydrogène, un atome d'halogène ou un radical ($C_1$—$C_8$)alcoyle, ($C_1$—$C_8$)alcoxy, hydroxyle ou nitro;

Y représente un atome d'hydrogène ou un ou deux radicaux ($C_1$—$C_8$)alcoyle, ($C_1$—$C_8$)alcoxy ou hydroxyle qui peuvent être identiques ou différentes;

n représente 0 ou 1 et lorsque n est zéro, la ligne en pointillés est une double liaison;

et leurs sels d'addition d'acides, mais à l'exclusion du composé de formule $I_w$ où Z et Y représentent tous deux un atome d'hydrogène, R représente un radical —$(CH_2)_3$—$N(CH_3)_2$ et Ar représente un radical $C_6H_5$—.

19. Composé suivant la revendication 18, dans lequel

R représente un radical ($C_1$—$C_8$)alcoyle ou -$Alk^1$—$NR^1R^2$;

$R^1$ et $R^2$ représentent chacun un atome d'hydrogène, ou un radical ($C_1$—$C_8$)alcoyle, ou bien $R^1$ et $R^2$ pris conjointement avec l'atome d'azote adjacent peuvent former un résidu 1-pipérindyle, 1-pyrrolidinyle, 4-morpholinyle ou 4-(alcoyl inférieur)-1-pipérazinyle,

Ar représente un radical 2-, 3- ou 4-pyridinyle, 2- ou 3-thiényle, phényle ou phényle substitué, le radical phényle substitué étant substitué par 1 à 3 radicaux halo, ($C_1$—$C_8$)alcoyle, ($C_1$—$C_8$)alcoxy, trifluorométhyle ou nitro qui peuvent être indentiques ou différents;

$Alk^1$ représente un chaîne hydrocarbonée droite ou ramifiée comptant 1 à 8 atomes de carbone;

Z représente un atome d'hydrogène, un atome d'halogène ou un radical ($C_1$—$C_8$)alcoyle, ($C_1$—$C_8$)alcoxy, hydroxyle ou nitro;

Y représente un atome d'hydrogène ou un ou deux radicaux ($C_1$—$C_8$)alcoyle, ($C_1$—$C_8$)alcoxy ou hydroxyle qui peuvent être identiques ou différents;

n représente 0 ou 1 et lorsque n est zéro la ligne en pointillés est un double liaison;

et ses sels d'addition d'acides pharmaceutiquement acceptables.

20. Composé suivant la revendication 18 qui est une 6-aryl-11H-pyrdio[2,3-b][1,4]benzodiazépine ou un 5,6-dihydro-dérivé de celle-ci et est de formule

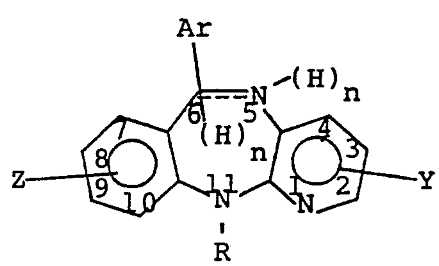

21. Composé suivant la revendication 18 qui est la 6-phényl-11H-pyrido[2,3-b][1,4]benzodiazépine.

22. Composé suivant la revendication 18 qui est la 8-chloro-6-phényle-11H-pyrido[2,3-b][1,4]benzodiazépine.

23. Composé suivant la revendication 18 qui est la 9-chloro-6-phényl-11H-pyrido[2,3-b][1,4]benzodiazépine.

24. Composé suivant la revendication 18 qui est la 6-(4-chlorphényl)-11H-pyrido[2,3-b][1,4]benzodiazépine.

25. Composé suivant la revendication 18 qui est la 6-(méthylphényl)-11H-pyrido[2,3-b][1,4]benzodiazépine.

26. Composé suivant la revendication 18 qui est la 6-(4-méthoxyphényl)-11H-pyrido[2,3-b][1,4]benzodiazépine.

27. Composé suivant la revendication 18 qui est la 8-chloro-N,N-diméthyl-6-phényl-11H-pyrido[2,3-b][1,4]benzodiazépine-11-propanamine.

28. Composé suivant la revendication 18 qui est le [1:1] oxalate de 8-chloro-N,N-diméthyl-6-phényl-11H-pyrido[2,3-b][1,4]benzodiazépine-11-propanamine.

29. Composé suivant la revendication 18 qui est la N,N-diméthyl-6-phényl-11H-pyrido[2,3-b][1,4]benzodiazépine-11-éthanamine.

30. Composé suivant la revendication 18 qui est le [1:1] fumarate de N,N-diméthyl-6-phényl-11H-pyrido[2,3-b][1,4]benzodiazépine-11-éthanamine.

31. Composé suivant la revendication 18 qui est la 11-[3-[4-morpholinyl)propyl]-6-phényl-11H-pyrido[2,3-b][1,4]benzodiazépine.

32. Composé suivant la revendication 18 qui est le [1:1] fumarate de 1-[3-(4-morpholinyl)propyl]-6-phényl-11H-pyrido[2,3-b][1,4]benzodiazèpine.

33. Composé suivant la revendication 18 qui est la N,N-diéthyl-6-phényl-11H-pyrido[2,3-b][1,4]benzodiazépine-11-propanamine.

34. Composé suivant la revendication 18 qui est le [1:1] oxalate de N,N-diméthyl-6-phényl-11H-pyrido[2,3-b][1,4]benzodiazèpine-11-propanamine.

35. Composé suivant la revendication 18 qui est la 9-chloro-N,N-diméthyl-6-phényl-11H-pyrido[2,3-b][1,4]benzodiazépine-11-propanamine.

36. Composé suivant la revendication 18 qui est le [1:1] fumarate de 9-chloro-N,N-diméthyl-6-phényl-11H-pyrido[2,3-b][1,4]benzodiazépine-11-propanamine.

37. Composé suivant la revendication 18 qui est la 6-phényl-11-[3-(1-pipérindyl)propyl]-11H-pyrido[2,3-b][1,4]benzodiazépine.

38. Composé suivant la revendication 18 qui est le [1:1] fumarate de 6-phényle-11-[3-(1-pipéridinyl)propyl]-11H-pyrido[2,3-b][1,4]benzodiazépine.

39. Composé suivant la revendication 18 qui est la 6-(4-chlorophényl)-N,N-diméthyl-11H-pyrido[2,3-b][1,4]benzodiazépine-11-propanamine.

40. Composé suivant la revendication 18 qui est le [1:1] fumarate de 6-(4-chlorophényl)-N,N-diméthyl-11H-pyrido[2,3-b][1,4]benzodiazépine-11-propanamine.

41. Composé suivant la revendication 18 qui est la 8-chloro-N,N-diméthyl-6-phényl-11H-pyrido[2,3-b][1,4]benzodiazépine-11-éthanamine.

42. Composé suivant la revendication 18 qui est le [1:1] oxalate de 8-chloro-N,N-diméthyl-6-phényl-11H-pyrido[2,3-b][1,4]benzodiazépine-11-éthanamine.

43. Composé suivant la revendication 18 qui est la revendication 18 qui est la 8-chloro-11-méthyl-6-phényl-11H-pyrido[2,3-b][1,4]benzodiazépine.

44. Composé suivant la revendication 18 qui est la N,N-diméthyl-6-(4-méthylphényl)-11H-pyrido[2,3-b][1,4]benzodiazépine-11-propanamine.

45. Composé suivant la revendication 18 qui est le [1:1] fumarate de N,N-diméthyl-6-(4-méthylphényl)-11-pyrido[2,3-b][1,4]benzodiazépine-11H-propanamine.

46. Composé suivant la revendication 18 qui est la 6-(4-méthoxyphényl)-N,N-diméthyl-11H-pyrido[2,3-b][1,4]benzodiazépine-11-propanamine.

47. Composé suivant la revendication 18 qui est le [1:1] fumarate de 6-(4-méthoxyphényl)-N,N-diméthyl-11-pyrido[2,3-b][1,4]benzodiazépine-11H-propanamine.

48. Composé suivant la revendication 18 qui est la 6-(3-chlorophényl)-11H-pyrido[2,3-b][1,4]benzodiazépine.

49. Composé suivant la revendication 18 qui est la 6-(3-chlorophényl)-N,N-diméthyl-11H-pyrido[2,3-b][1,4]benzodiazépine-11-propanamine.

50. Composé suivant la revendication 18 qui est la 6-(4-fluorophényl)-11H-pyrido[2,3-b][1,4]benzodiazépine.

51. Composé suivant la revendication 18 qui est la 6-(4-fluorophényl)-N,N-diméthyl-11H-pyrido[2,3-b][1,4]benzodiazépine-11-propanamine.

52. Composé suivant la revendication 18 qui est la 11-[3-(1,3-dihydro-1,3-dioxo-2H-isoindol-2-yl)propyl]-6-phényl-11H-pyrido[2,3-b][1,4]benzodiazépine.

53. Composé suivant la revendication 18 qui est la 6-phényl-11H-pyrido[2,3-b][1,4]benzodiazépine-11-propanamine.

54. Composé suivant la revendication 18 qui est la dichlorhydrate de 6-phényl-11H-pyrido[2,3-b][1,4]benzodiazépine-11-propanamide hémihydraté.

55. Composé suivant la revendication 18 qui est l'ester éthylique d'acide N-[3-[6-phényle-11H-pyrido[2,3-b][1,4]benzodiazépine-11-yl]propyl]méthanimidique.

56. Composé suivant la revendication 18 qui est la N-méthyl-6-phényl-11H-pyrido[2,3-b][1,4]benzodiazépine-11-propanamine.

57. Composé suivant la revendication 18 qui est le dichlorohydrate de N-méthyl-6-phényl-11H-pyrido[2,3-b][1,4]benzodiazépine-11-propanamine.

58. Composé suivant la revendication 18 qui est l'ester éthylique d'acide N-[3-[6-phényle-11H-pyrido[2,3-b][1,4]benzodiazépine-11-yl]propyl]carbamique.

59. Composé suivant la revendication 18 qui est la 5,6-dihydro-N,N-diméthyl-6-phényl-11H-pyrido[2,3-b][1,4]benzodiazépine-11-propanamine.

60. Composé suivant la revendication 18 qui est le dichloroydrate de 5,6-diydro-N,N-diméthyl-6-phényl-11H-pyrido[2,3-b][1,4]benzodiazépine-11-propanamine hémihydrate.

61. Composé suivant la revendication 18 qui est la 8-chloro-6-(2-chlorophényl)-5,6-dihydro-11H-pyrido[2,3-b][1,4]benzodiazépine.

62. Composé suivant la revendication 18 qui est la 5.6-dihydro-N-méthyl-6-phényl-11H-pyrido-[2,3-b][1,4]benzodiazépine-11-propanamine.

63. Composé suivant la revendication 18 qui est la 6-(2-fluorophényl)-N,N-diméthyl-11H-pyrido[2,3-b][1,4]benzodiazépine-11-propanamine.

64. Composé suivant la revendication 18 qui est la 6-(2-chlorophényl)-N,N-diméthyl-11H-pyrido[2,3-b][1,4]benzodiazépine-11-propanamine.

65. Composé suivant la revendication 18 qui est la 6-(2-bromophényl)-N,N-diméthyl-11H-pyrido[2,3-b][1,4]benzodiazépine-11-propanamine.

66. Composé suivant la revendication 18 qui est la 8-chloro-11-méthyl-6-(2-nitrophényl)-11H-pyrido[2,3-b][1,4]benzodiazépine.

67. Composé suivant la revendication 18 qui est la 8-chloro-6-(2-chlorophényl)-11-méthyl-11H-pyrido[2,3-b][1,4]benzodiazépine.

68. Composé suivant la revendication 18 qui est la 6-(2-bromophényl)-8-chloro-11-méthyl-11H-pyrido[2,3-b][1,4]benzodiazépine.

69. Composé suivant la revendication 18 qui est le [1:1] fumarate de 6-(3-chlorophényl)-N,N-diméthyl-11H-pyrido[2,3-b][1,4]benzodiazépine-11-propanamine.

70. Composé suivant la revendication 18 qui est le dichlorohydrate de 6-(4-fluorophényl)-N,N-diméthyl-11H-pyrido[2,3-b][1,4]benzodiazépine-11-propanamine hémihydraté.

71. Composé suivant la revendication 18 qui est une 6-aryl-11H-pyrido[3,4-b][1,4]benzodiazépine ou un 5,6-dihydro-dérivé de celle-ci et est de formule

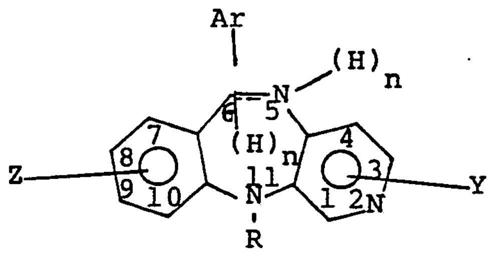

72. Composé suivant la revendication 18 qui est une 10-aryl-5H-pyrido[4,3-b][1,4]benzodiazépine ou un 10,11-dihydro-dérivé de celle-ci et est de formule

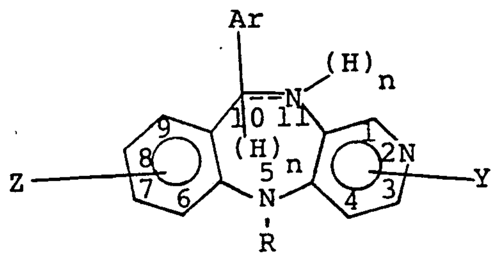

73. Composé suivant la revendication 18 qui est la 10-aryl-5H-pyrido[3,2-b][1,4]benzodiazépine ou un 10,11-dihydro-dérivé de celle-ci et est de formule

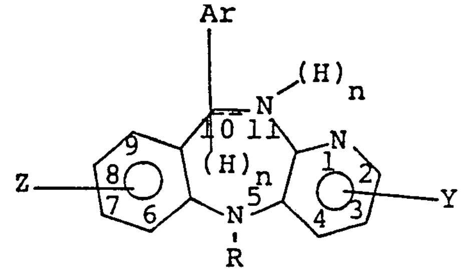

74. Composé suivant l'une quelconque des revendications 18 à 73 à utiliser pour le traitement de la dépression.

75. Composition pharmaceutique pour le traitment de la dépression sous forme dosée unitaire comprenant (a) une quantité efficace d'un composé suivant l'une quelconque des revendications 18 à 74 et (b) un excipient pharmacetique.

76. Composition pharmaceutique suivant la revendication 75, dans laquelle le composé un sel pharmaceutiquement acceptable de la N-méthyl-6-phényl-11H-pyrido[2,3-b](1,4]benzodiazépine-11-propanamine.

77. Composition pharmaceutique suivant la revendication 75, dans laquelle le composé un sel pharmaceutiquement acceptable de la 6-phényle-11H-pyrido[2,3-b](1,4]benzodiazépine-11-propanamine.

78. Composition pharmaceutique suivant la revendication 75, dans laquelle le composé est la 8-chloro-N,N-diméthyl-6-phényl-11H-pyrido[2,3-b](1,4]benzodiazépine-11-propanamine.

79. Composition pharmaceutique suivant la revendication 75, dans laquelle le composé est le [1:1] fumarate de 6-(3-chlorophényl)-N,N-diméthyl-11H-pyrido[2,3-b](1,4]benzodiazépine-11-propanamine.

80. Composition pharmaceutique suivant la revendication 75, dans laquelle le composé est un sel d'addition d'acide pharmaceutiquement acceptable de la 6-(4-fuorophényl)-N,N-diméthyl-11H-pyrido[2,3-b](1,4]benzodiazépine-11-propanamine.

81. Composition pharmaceutique suivant la revendication 75, dans laquelle le composé est la 6-(2-chlorophényle)-N,N-diméthyl-11H-pyrido[2,3-b](1,4]benzodiazépine-11-propanamine.

82. Utilisation d'un composé suivant la revendication 1 dans la préparation d'un agent antidépresseur.

83. Utilisation d'un composé suivant l'une quelconque des revendications 1 à 15 et 18 à 73 dans la préparation d'un agent antidépresseur.

84. Composé suivant la revendication 1 à utiliser en médecine.

85. Composé suivant l'une quelconque des revendications 1 à 15 et 18 à 73 à utiliser en médecine.

**Revendications pour l'Etat contractant: AT**

1. Procédé de préparation d'un composé de formule

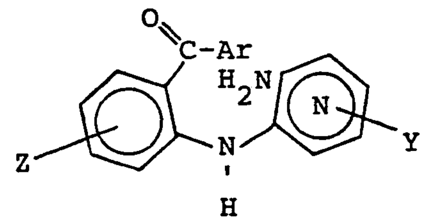

où

Ar représente un radical 2-, 3- ou 4-pyridinyle, 2- ou 3-thiényle, phényle ou phényle substitué, le radical phényle substitué étant substitué par 1 à 3 radicaux halo, ($C_1$—$C_8$)alcoyle, ($C_1$—$C_8$)alcoxy, trifluorométhyle ou nitro qui peuvent être indentiques ou différents;

Z représente un atome d'hydrogène, un atome d'halogène ou un radical ($C_1$—$C_8$)alcoyle, ($C_1$—$C_8$)alcoxy, hydroxyle ou nitro;

Y représente un atome d'hydrogène ou un ou deux radicaux ($C_1$—$C_8$)alcoyle, ($C_1$—$C_8$)alcoxy ou hydroxyle qui peuvent être identiques ou différentes;

ou d'un sel d'addition d'acide pharmaceutiquement acceptable de celui-ci;

lequel procédé comprend la réaction d'un composé de formule générale III

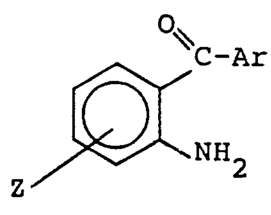

où Ar et Z sont tels que définis ci-dessus, avec un composé de formule générale IV

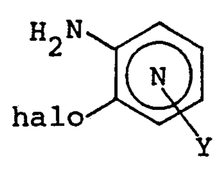

où Y est tel que défini ci-dessus et halo représente un atome d'halogène, et la formation éventuelle d'un sel du composé.

2. Procédé suivant la revendication 1, de préparation de
la [2-[(3-amino-2-pyridinyl)amino]phényl]phénylméthanone.
la [2-[(3-amino-2-pyridinyl)amino]-4-chlorophényl]phénylméthanone.
la [2-[(3-amino-2-pyridinyl)amino]phényl](4-méthylphényl)méthanone.
la [2-[(3-amino-2-pyridinyl)amino]-5-chlorophényl](2-chlorophényl)méthanone.
la [2-[(3-amino-2-pyridinyl)amino]-3-chlorophényl]phényl)méthanone.
la [2-[(3-amino-2-pyridinyl)amino]-4-fluorophényl]-phénylméthanone.
la [2-[(3-amino-2-pyridinyl)amino]phényl](3-chlorophényl)méthanone.
la [2-[(3-amino-2-pyridinyl)amino]phényl](4-fluorophényl)méthanone.
la [2-[(3-amino-2-pyridinyl)amino]phényl]-(3-trifluorométhylphényl)méthanone.
la [2-[(3-amino-2-pyridinyl)amino]phényl]-(3-fluorophényl)méthanone.
la [2-[(3-amino-2-pyridinyl)amino]phényl]-(2-fluorophényl)méthanone.
la [2-[(3-amino-2-pyridinyl)amino]phényl]-(2-chlorophényl)méthanone, ou
la [2-[(3-amino-2-pyridinyl)amino]phényl]-(2-bromophényl)méthanone.

3. Procédé de préparation d'une pyrido[1,4]benzodiazépines qui est une 6-aryl-11H-pyrido[2,3-b][1,4]benzodiazépine ou un 5,6-dihydro-dérivé de celle-ci et qui est de formule $I_w$,

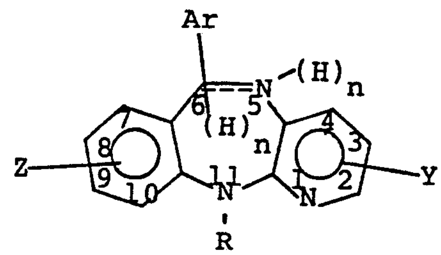

ou qui est une 6-aryl-11H-pyrido[3,4-b][1,4]benzodiazépine ou un 5,6-dihydro-dérivé de celle-ci et qui est de formule $I_x$,

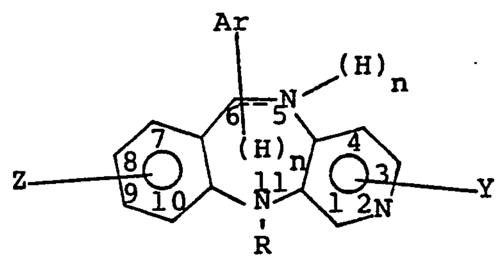

ou qui est une 10-aryl-5H-pyrido[4,3-b][1,4]benzodiazépine ou un 10,11-dihydro-dérivés de celle-ci et qui est de formule $I_y$,

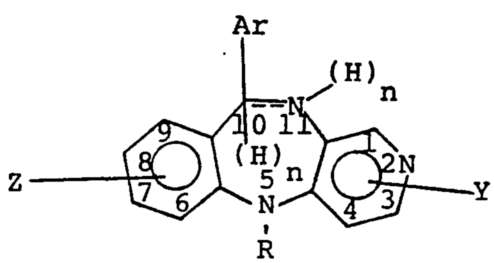

ou qui est une 10-aryl-5H-pyrido[3,2-b][1,4]benzodiazépine ou les 10,11-dihydro-dérivé de celle-ci et qui est de formule $I_z$,

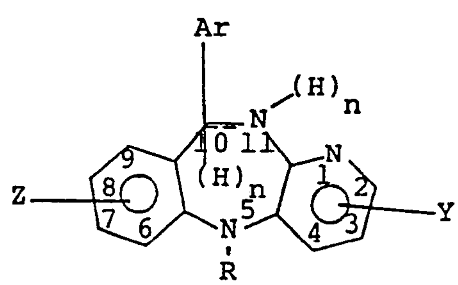

où

R représente un atome d'hydrogène, ou un radical $(C_1—C_8)$alcoyle, -$Alk^1—NR^1R^2$ ou -$Alk^1—N{=}CH—OC_2H_5$;

$R^1$ et $R^2$ représentent chacun un atome d'hydrogène, ou un radical $(C_1—C_8)$alcoyle ou —C(O)O—$(C_1—C_8)$alcoyle ou bien $R^1$ et $R^2$ pris conjointement avec l'atome d'azote adjacent peuvent former un résidu hétérocyclique 1-phtalimido, 1-pipéridinyle, 1-pyrrolidinyle, 4-morpholino, 1-pipérazino ou pipérazine-1-yle 4-substitué;

Ar représente un radical 2-, 3- ou 4-pyridinyle, 2- ou 3-thiényle, phényle ou phényle substituté, le radical phényle substitué étant substitué par 1 à 3 radicaux halo, $(C_1—C_8)$alcoyle, $(C_1—C_8)$alcoxy, trifluorométhyle ou nitro qui peuvent être indentiques ou différents;

$Alk^1$ représente un chaîne hydrocarbonée droite ou ramifiée comptant 1 à 8 atomes de carbone;

Z représente un atome d'hydrogène, un atome d'halogène ou un radical $(C_1—C_8)$alcoyle, $(C_1—C_8)$alcoxy, hydroxyle ou nitro;

Y représente un atome d'hydrogène ou un ou deux radicaux $(C_1—C_8)$alcoyle, $(C_1—C_8)$alcoxy ou hydroxyle qui peuvent être identiques ou différentes;

n représente 0 ou 1 et lorsque n est zéro, la ligne en pointillés est une double liasion;

et de leurs sels d'addition d'acides, mais à l'exculsion du composé de formule $I_w$ où Z et Y représentent tous deux un atome d'hydrogène, R eprésente un radical $—(CH_2(_3—N(CH_3)_2$ et Ar représente un radical $C_6H_5—$,

lequel procédé comprend la réaction d'un composé de formule générale III

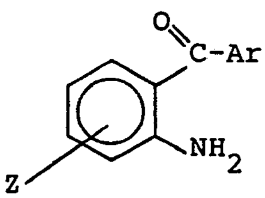

où Ar et Z sont tels que définis à propos des formules générales $I_w$ à $I_z$, avec un composé de formule générale IV

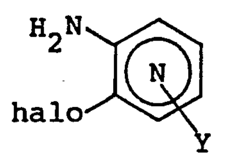

où Y est tel que défini à propos des formules générales $I_w$ à $I_z$ et halo représente un atome d'halogène, pour former un composé de formule générale $I_w$ à $I_z$ où R représente un atome d'hydrogène et n est zéro, et eventuellement ensuite, la conversion du composé de formule générale $I_w$ à $I_z$ ainsi obtenu en un autre composé de formule générale $I_w$ à $I_z$, et éventuellment la formation d'un sel pharmaceutiquement acceptable de celui-ci.

4. Procédé suivant la revendication 3, dans lequel

R représente un radical $(C_1—C_8)$alcoyle ou -$Alk^1—NR^1R^2$;

$R^1$ et $R^2$ représentent chacun un atome d'hydrogène, ou un radical $(C_1—C_8)$alcoyle, ou bien $R^1$ et $R^2$ pris conjointement avec l'atome d'azote adjacent peuvent former un résidu 1-pipéridinyle, 1-pyrrolidinyle, 4-morpholinyle ou 4-(alcoyl inférieur)-1-pipérazineyle;

Ar représente un radical 2-, 3- ou 4-pyridinyle, 2- ou 3-thiényle, phényle ou phényle substituté, le radical phényle substitué étant substitué par 1 à 3 radicaux halo, $(C_1—C_8)$alcoyle, $(C_1—C_8)$alcoxy, trifluorométhyle ou nitro qui peuvent être indentiques ou différents;

$Alk^1$ représente un chaîne hydrocarbonée droite ou ramifiée comptant 1 à 8 atomes de carbone;

Z représente un atome d'hydrogène, un atome d'halogène ou un radical $(C_1—C_8)$alcoyle, $(C_1—C_8)$alcoxy, hydroxyle ou nitro;

Y représente un atome d'hydrogène ou un ou deux radicaux $(C_1—C_8)$alcoyle, $(C_1—C_8)$alcoxy ou hydroxyle qui peuvent être identiques ou différents;

n représente 0 ou 1 et lorsque n est zéro la ligne en pointillés est une double liaison.

5. Procédé suivant la revendication 3, de préparation d'une 6-aryl-11H-pyrido[2,3-b][1,4]benzodiazépine ou d'un 5,6-dihydro-dérivé de celle-ci que est de formule $I_w$.

6. Procédé suivant la revendication 3, pour préparer

la 6-phényl-11H-pyrdio[2,3-b][1,4]benzodiazépine,

la 8-chloro-6-phényle-11H-pyrido[2,3-b][1,4]benzodiazépine,

la 9-chloro-6-phényl-11H-pyrido[2,3-b][1,4]benzodiazépine,

la 6-(4-chlorphényl)-11H-pyrido[2,3-b][1,4]benzodiazépine,

la 6-(méthylphényl)-11H-pyrido[2,3-b][1,4]benzodiazépine,

la 6-(4-méthoxyphényl)-11H-pyrido[2,3-b][1,4]benzodiazépine,

la 8-chloro-N,N-diméthyl-6-phényl-11H-pyrido[2,3-b][1,4]benzodiazépine-11-propanamine,

le [1:1] oxalate de 8-chloro-N,N-diméthyl-6-phényl-11H-pyrido[2,3-b][1,4]benzodiazépine-11-propanamine,
la N,N-diméthyl-6-phényl-11H-pyrido[2,3-b][1,4]benzodiazépine-11-éthanamine,
le [1:1] fumarate de N,N-diméthyl-6-phényl-11H-pyrido[2,3-b][1,4]benzodiazépine-11-éthanamine,
la 11-[3-[4-morpholinyl)propyl]-6-phényl-11H-pyrido[2,3-b][1,4]benzodiazépine,
le [1:1] fumarate de 1-[3-(4-morpholinyl)propyl]-6-phényl-11H-pyrido[2,3-b][1,4]benzodiazèpine,
la N,N-diéthyl-6-phényl-11H-pyrido[2,3-b][1,4]benzodiazépine-11-propanamine,
le [1:1] oxalate de N,N-diéthyl-6-phényl-11H-pyrido[2,3-b][1,4]benzodiazèpine-11-propanamine,
la 9-chloro-N,N-diméthyl-6-phényl-11H-pyrido[2,3-b][1,4]benzodiazépine-11-propanamine,
le [1:1] fumarate de 9-chloro-N,N-diméthyl-6-phényl-11H-pyrido[2,3-b][1,4]benzodiazépine-11-propanamine,
la 6-phényl-11-[3-(1-pipérindyl)propyl]-11H-pyrido[2,3-b][1,4]benzodiazépine,
le [1:1] fumarate de 6-phényle-11-[3-(1-pipéridinyl)propyl]-11H-pyrido[2,3-b][1,4]benzodiazépine,
la 6-(4-chlorophényl)-N,N-diméthyl-11H-pyrido[2,3-b][1,4]benzodiazépine-11-propanamine,
le [1:1] fumarate de 6-(4-chlorophényl)-N,N-diméthyl-11H-pyrido[2,3-b][1,4]benzodiazépine-11-propanamine,
la 8-chloro-N,N-diméthyl-6-phényl-11H-pyrido[2,3-b][1,4]benzodiazépine-11-éthanamine,
le [1:1] oxalate de 8-chloro-N,N-diméthyl-6-phényl-11H-pyrido[2,3-b][1,4]benzodiazépine-11-éthanamine,
la 8-chloro-11-méthyl-6-phényl-11H-pyrido[2,3-b][1,4]benzodiazépine,
la N,N-diméthyl-6-(4-méthylphényl)-11H-pyrido[2,3-b][1,4]benzodiazépine-11-propanamine,
le [1:1] fumarate de N,N-diméthyl-6-(4-méthylphényl)-11H-pyrido[2,3-b][1,4]benzodiazépine-11-propanamine,
la 6-(4-méthoxyphényl)-N,N-diméthyl-11H-pyrido[2,3-b][1,4]benzodiazépine-11-propanamine,
le [1:1] fumarate de 6-(4-méthoxyphényl)-N,N-diméthyl-11H-pyrido[2,3-b][1,4]benzodiazépine-11-propanamine,
la 6-(3-chlorophényl)-11H-pyrido[2,3-b][1,4]benzodiazépine,
la 6-(3-chlorophényl)-N,N-diméthyl-11H-pyrido[2,3-b][1,4]benzodiazépine-11-propanamine,
la 6-(4-fluorophényl)-11H-pyrido[2,3-b][1,4]benzodiazépine,
la 6-(4-fluorophényl)-N,N-diméthyl-11H-pyrido[2,3-b][1,4]benzodiazépine-11-propanamine,
la 11-[3-(1,3-dihydro-1,3-dioxo-2H-isoindol-2-yl)propyl]-6-phényl-11H-pyrido[2,3-b][1,4]benzodiazépine,
la 6-phényl-11H-pyrido[2,3-b][1,4]benzodiazépine-11-porpanamine,
la dichlorhydrate de 6-phényl-11H-pyrido[2,3-b][1,4]benzodiazépine-11-propanamide hémihydraté,
l'ester éthylique d'acide N-[3-[6-phényle-11H-pyrido[2,3-b][1,4]benzodiazépine-11-yl]propyl]-méthanimidique,
la N-méthyl-6-phényl-11H-pyrido[2,3-b][1,4]benzodiazépine-11-propanamine,
le dichlorohydrate de N-méthyl-6-phényl-11H-pyrido[2,3-b][1,4]benzodiazépine-11-propanamine,
l'ester éthylique d'acide N-[3-[6-phényle-11H-pyrido[2,3-b][1,4]benzodiazépine-11-yl]propyl]-carbamique,
la 5,6-dihydro-N,N-diméthyl-6-phényl-11H-pyrido[2,3-b][1,4]benzodiazépine-11-propanamine,
le dichlorohydrate de 5,6-diydro-N,N-diméthyl-6-phényl-11H-pyrido[2,3-b][1,4]benzodiazépine-11-propanamine hémihydrate,
la 8-chloro-6-(2-chlorophényl)-5,6-dihydro-11H-pyrido[2,3-b][1,4]benzodiazépine,
la 5,6-dihydro-N-méthyl-6-phényl-11H-pyrido-[2,3-b][1,4]-benzodiazépine-11-propanamine,
la 6-(2-fluorophényl)-N,N-diméthyl-11H-pyrido[2,3-b][1,4]benzodiazépine-11-propanamine,
la 6-(2-chlorophényl)-N,N-diméthyl-11H-pyrido[2,3-b][1,4]benzodiazépine-11-propanamine,
la 6-(2-bromophényl)-N,N-diméthyl-11H-pyrido[2,3-b][1,4]benzodiazépine-11-propanamine,
la 8-chloro-11-méthyl-6-(2-nitrophényl)-11H-pyrido[2,3-b][1,4]benzodiazépine,
la 8-chloro-6-(2-chlorophényl)-11-méthyl-11H-pyrido[2,3-b][1,4]benzodiazépine,
la 6-(2-bromophényl)-8-chloro-11-méthyl-11H-pyrido[2,3-b][1,4]benzodiazépine,
le [1:1] fumarate de 6-(3-chlorophényl)-N,N-diméthyl-11H-pyrido[2,3-b][1,4]benzodiazépine-11-propanamine, ou
le dichlorohydrate de 6-(4-fluorophényl)-N,N-diméthyl-11H-pyrido[2,3-b][1,4]benzodiazépine-11-propanamine hémihydraté.

7. Procédé suivant la revendication 3 de préparation d'une 6-aryl-11H-pyrido[3,4-b][1,4]benzodiazépine ou d'un 5,6-dihydro-dérivé de celle-ci de formule générale $I_x$.

8. Procédé suivant la revendication 3 de préparation d'une 10-aryl-5H-pyrido[4,3-b][1,4]benzodiazépine ou un 10,11-dihydro-dérivé de celle-ci de formule générale $I_y$

9. Procédé suivant la revendication 3 de préparation d'une 10-aryl-5H-pyrido[3,2-b][1,4]benzodiazépine ou un 10,11-dihydro-dérivé de celle-ci de formule générale $I_z$.

10. Procédé de préparation d'une composition pharmaceutique, lequel procédé comprend le mélange d'un composé de formule

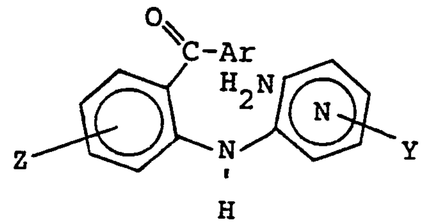

où

Ar représente un radical 2-, 3- ou 4-pyridinyle, 2- ou 3-thiényle, phényle ou phényle substitué, le radical phényle substitué étant substitué par 1 à 3 radicaux halo, ($C_1$—$C_8$)alcoyle, ($C_1$—$C_8$)alcoxy, trifluorométhyle ou nitro qui peuvent être indentiques ou différents;

Z représente un atome d'hydrogène, un atome d'halogène ou un radical ($C_1$—$C_8$)alcoyle, ($C_1$—$C_8$)alcoxy, hydroxyle ou nitro;

Y représente un atome d'hydrogène ou un ou deux radicaux ($C_1$—$C_8$)alcoyle, ($C_1$—$C_8$)alcoxy ou hydroxyle qui peuvent être identiques ou différentes;

ou d'un sel d'addition d'acide pharmaceutiquement acceptables de celui-ci;

avec un excipient pharmaceutiquement acceptable.

11. Procédé suivant la revendication 10, dans lequel le composé est

un sel pharmaceutiquement acceptable de la N-méthyl-6-phényl-11H-pyrido[2,3-b][1,4]benzodiazépine-11-propanamine,

un sel pharmaceutiquement acceptable de la 6-phényl-11H-pyrido[2,3-b][1,4]benzodiazépine-11-propanamine,

la 8-chloro-N,N-diméthyl-6-phényl-11H-pyrido[2,3-b][1,4]benzodiazépine-11-propanamine,

le [1:1] fumarate de 6-(3-chlorophényl-N,N-diméthyl-11H-pyrido[2,3-b][1,4]benzodiazépine-11-propanamine,

un sel d'addition d'acide pharmaceutiquement acceptable de la 6-(4-fluorophényl)-N,N-diméthyl-11H-pyrido[2,3-b][1,4]benzodiazépine-11-propanamine, ou

un sel d'addition d'acide pharmaceutiquement acceptable de la 6-(2-chlorophényl)-N,N-diméthyl-11H-pyrido[2,3-b][1,4]benzodiazépine-11-propanamine.